# EUROPEAN PATENT APPLICATION

(11) **EP 4 621 068 A1**
(43) Date of publication of application: **24.09.2025**
(21) Application number: 23891957.5
(22) Date of filing: 14.11.2023
(51) Int. Cl.: C12Q 1/6818

(54) **DETECTION OF TARGET NUCLEIC ACID BY QUENCHING-PTO CLEAVAGE AND EXTENSION (Q-PTOCE) ASSAY**

(30) Priority: 16.11.2022 KR 20220153926
(71) Applicant: Seegene, Inc., Seoul 05548 (KR)
(72) Inventor: YANG, Boo Hyun, Seoul 05548 (KR); KIM, Jeong Woo, Seoul 05548 (KR)
(74) Representative: Dehns
(86) International application number: PCT/KR2023/018212
(87) International publication number: WO 2024/106890

(57) **Abstract**

The present disclosure relates to a method for detecting a target nucleic acid in a sample by Quenching-PTO Cleavage and Extension (Q-PTOCE) assay. The present disclosure has an advantage in that the signal detection temperature can be selected within a higher temperature range by utilizing not only the cleavage of the Q-PTO and hybridization between the reporter-carrying fragment and the CTO, but also the extension of the reporter-carrying fragment after hybridization

## Description

### Technical Field

The present disclosure relates to a method for detecting a target nucleic acid in a sample by Quenching-PTO Cleavage and Extension (Q-PTOCE) assay.

### Background Technology

For the detection of target nucleic acids, real-time detection methods that enable the detection of target nucleic acids while monitoring target amplification in real time are widely used. Real-time detection methods generally utilize labeled probes or primers that hybridize specifically with target nucleic acids. Examples of methods utilizing hybridization between labeled probes and target nucleic acids include molecular beacon method using dual-labeled probes with a hairpin structure (Tyagi et al., Nature Biotechnology, v.14 MARCH 1996), HyBeacon method (French DJ et al., Mol. Cell Probes, 15(6):363-374(2001)), hybridization probe method using two probes, each labeled as donor and acceptor (Bernad et al., 147-148 Clin Chem 2000; 46), and Lux method using single-labeled oligonucleotides (U.S. Patent No. 7,537,886). TaqMan method (U.S. Pat. Nos. 5,210,015 and 5,538,848) using cleavage of dual-labeled probes by 5'-nuclease activity of DNA polymerase is also widely used in the art. Examples of methods using labeled primers include Sunrise primer method (Nazarenko et al., 2516-2521 Nucleic Acids Research, 1997, v.25 no.12, and U.S. Pat. No. 6,117,635), Scorpion primer method (Whitcombe et al., 804-807, Nature Biotechnology v.17 AUGUST 1999 and U.S. Pat. No. 6,326,145), and TSG primer method (WO 2011/078441).

As alternative approaches, real-time detection methods using duplexes formed depending on the presence of target nucleic acids have been proposed: Invader assay (U.S. Pat. Nos. 5,691,142, 6,358,691 and 6,194,149), PTOCE (PTO cleavage and extension) method (WO 2012/096523), PCE-SH (PTO Cleavage and Extension-Dependent Signaling Oligonucleotide Hybridization) method (WO 2013/115442), and PCE-NH (PTO Cleavage and Extension-Dependent Non-Hybridization) method (PCT/KR2013/012312).

Since the above-described conventional real-time detection techniques can detect only one target nucleic acid per label, the number of target nucleic acids that can be simultaneously detected in a single reaction is limited by the number of labels that can be used (e.g., 5 or less).

Melting analysis may be used to detect multiple target nucleic acids using a single label. However, melting analysis has significant drawbacks as it takes longer than real-time techniques and becomes increasingly difficult to design probes with different Tm values as the number of target nucleic acids increases.

As described above, conventional real-time detection techniques or melting analysis are limited when detecting multiple target nucleic acids.

Therefore, there is a need for a real-time detection method that can detect multiple target nucleic acids simultaneously, even with a limited number of labels in a single reaction.

Throughout this application, various patents and publications are referenced and citations are provided in parentheses. The disclosures of these patents and publications in their entirety are hereby incorporated by reference into this application in order to more fully describe this invention and the state of the art to which this invention pertains.

### Disclosure

### Technical Problem

The present inventors have endeavored to develop a novel method for detecting target nucleic acids in real time with improved convenience and high efficiency. As a result, the present inventors have established a novel protocol for detecting a target nucleic acid, in which target nucleic acid detection is achieved not only through probe hybridization but also through enzymatic reactions of cleavage and extension, by utilizing two quencher molecules and a reporter molecule. The protocol of the present disclosure can be particularly applied to detect a target nucleic acid having the highest detection temperature in the method for detecting multiple target nucleic acids, wherein signals are provided only for a single target nucleic acid at a single temperature in a single reaction vessel.

Therefore, it is an object of the present disclosure to provide a method for detecting a target nucleic acid in a sample by Quenching-PTO Cleavage and Extension (Q-PTOCE) assay.

It is another object of the present disclosure to provide a composition for detecting a target nucleic acid, comprising a primer, a Q-PTO, and a CTO.

It is another object of the present disclosure to provide a method for detecting n target nucleic acids in a sample using the above-described composition for detecting a target nucleic acid.

Other objects and advantages of the present invention will become apparent from the detailed description to follow taken in conjunction with the appended claims and drawings.

### Solution to Problem

According to an aspect of the present disclosure, there is provided a method for detecting a target nucleic acid in a sample by Quenching-PTO Cleavage and Extension (Q-PTOCE) assay, comprising:
(a) hybridizing a primer and a Quenching-Probing and Tagging Oligonucleotide (Q-PTO) with the target nucleic acid;
   wherein the primer comprises a hybridizing nucleotide sequence to a first region of the target nucleic acid,
   wherein the Q-PTO comprises in a 5' to 3' direction: (i) a 5'-tagging portion comprising a non-hybridizing nucleotide sequence to the target nucleic acid, and (ii) a hybridizing nucleotide sequence to a second region of the target nucleic acid,
   wherein the 5'-tagging portion of the Q-PTO is not hybridized with the target nucleic acid and the 3'-targeting portion of the Q-PTO is hybridized with the target nucleic acid,
   wherein the Q-PTO has a first quencher molecule in the 3'-targeting portion and a reporter molecule located at a position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in a reporter-carrying fragment,
   wherein the primer is located upstream of the Q-PTO;
(b) contacting the resultant of the step (a) to a DNA polymerase having 5' nuclease activity under conditions for cleavage of the Q-PTO to provide the reporter-carrying fragment;
   wherein the primer is extended to induce cleavage of the Q-PTO by the DNA polymerase having 5' nuclease activity such that the cleavage releases the reporter-carrying fragment;
(c) hybridizing the reporter-carrying fragment with a Capturing and Templating Oligonucleotide (CTO);
   wherein the CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment,
   wherein the capturing portion of the CTO is hybridized with the reporter-carrying fragment,
   wherein the CTO has a second quencher molecule located at a position such that, upon hybridization of the capturing portion of the CTO with the reporter-carrying fragment, the second quencher molecule is in close proximity to the reporter molecule, thereby causing the second quencher molecule to quench a signal from the reporter molecule;
(d) performing an extension reaction using the resultant of the step (c) and the DNA polymerase having 5' nuclease activity;
   wherein the reporter-carrying fragment hybridized with the capturing portion of the CTO is extended to generate an extended strand comprising an extended sequence complementary to the templating portion of the CTO, thereby forming an extended duplex between the extended strand and the CTO; and
(e) detecting the presence of the extended strand;
   whereby the presence of the extended strand indicates the presence of the target nucleic acid.

According to an embodiment of the present disclosure, the first quencher molecule of the Q-PTO is located immediately adjacent to or 1 to 30 nucleotides apart from the reporter molecule of the Q-PTO.

According to an embodiment of the present disclosure, the first quencher molecule of the Q-PTO and the second quencher molecule of the CTO are the same type of quencher molecule.

According to an embodiment of the present disclosure, the **Tm** value of the extended duplex is adjustable by (i) a sequence and/or length of the reporter-carrying fragment, (ii) a sequence and/or length of the CTO or (iii) the sequence and/or length of the reporter-carrying fragment and the sequence and/or length of the CTO.

According to an embodiment of the present disclosure, the presence of the extended strand in the step (e) is detected by measuring a signal provided from the extended duplex.

According to an embodiment of the present disclosure, the signal provided from the extended duplex is a signal generated from dissociation of the extended duplex.

According to an embodiment of the present disclosure, the presence of the extended strand in the step (e) is detected by measuring a signal at a temperature at which the extended duplex dissociates.

According to an embodiment of the present disclosure, the Q-PTO and/or the CTO are blocked at its 3'-end to prohibit its extension.

According to an embodiment of the present disclosure, the method further comprises repeating all or a portion of the steps (a)-(e) with denaturation.

According to an embodiment of the present disclosure, the target nucleic acid comprises a nucleotide variation.

According to an embodiment of the present disclosure, the method is performed in the presence of an additional primer that pairs with the primer to amplify the target nucleic acid.

According to another aspect of the present disclosure, there is provided a composition for detecting a target nucleic acid in a sample, comprising:
(i) a primer;
   wherein the primer comprises a hybridizing nucleotide sequence to a first region of the target nucleic acid,
(ii) a Quenching-Probing and Tagging Oligonucleotide (Q-PTO);
   wherein the Q-PTO comprises in a 5' to 3' direction: (i) a 5'-tagging portion comprising a non-hybridizing nucleotide sequence to the target nucleic acid, and (ii) a hybridizing nucleotide sequence to a second region of the target nucleic acid,
   wherein the 5'-tagging portion of the Q-PTO is not hybridized with the target nucleic acid and the 3'-targeting portion of the Q-PTO is hybridized with the target nucleic acid,
   wherein the primer is extended to induce cleavage of the Q-PTO by a DNA polymerase having 5' nuclease activity such that the cleavage releases the reporter-carrying fragment;
   wherein the Q-PTO has a first quencher molecule in the 3'-targeting portion and a reporter molecule located at position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in the reporter-carrying fragment,
(iii) a Capturing and Templating Oligonucleotide (CTO);
   wherein the CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment,
   wherein the capturing portion of the CTO is hybridized with the reporter-carrying fragment,
   wherein the CTO has a second quencher molecule located at positions such that upon hybridization of the capturing portion of the CTO with the reporter-carrying fragment, the second quencher molecule is in close proximity to the reporter molecule, thereby causing the second quencher molecule to quench a signal from the reporter molecule,
   wherein the reporter-carrying fragment hybridized with the capturing portion of the CTO is extended to generate an extended strand comprising an extended sequence complementary to the templating portion of the CTO, thereby forming an extended duplex between the extended strand and the CTO.

According to an embodiment of the present disclosure, the first quencher molecule of the Q-PTO is located immediately adjacent to or 1 to 30 nucleotides apart from the reporter molecule of in the Q-PTO.

According to an embodiment of the present disclosure, the first quencher molecule of the Q-PTO and the second quencher molecule of the CTO are the same type of quencher molecule.

According to an embodiment of the present disclosure, the **Tm** value of the extended duplex is adjustable by (i) a sequence and/or length of the fragment, (ii) a sequence and/or length of the CTO or (iii) the sequence and/or length of the fragment and the sequence and/or length of the CTO.

According to an embodiment of the present disclosure, the Q-PTO, the CTO or the Q-PTO and the CTO are blocked at its 3'-end to prohibit its extension.

According to an embodiment of the present disclosure, the composition further comprises a DNA polymerase having 5' nuclease activity.

According to an embodiment of the present disclosure, the composition further comprises an additional primer that pairs with the primer to amplify the target nucleic acid.

According to an embodiment of the present disclosure, the composition provides a signal dependent on the presence of the target nucleic acid.

According to an embodiment of the present disclosure, the signal dependent on the presence of the target nucleic acid is a signal provided from the extended duplex.

According to an embodiment of the present disclosure, the composition has a signal-changing temperature range in which the signal changes depending on the presence of the target nucleic acid, and a signal-constant temperature range in which the signal is constant even in the presence of the target nucleic acid.

According to an embodiment of the present disclosure, the signal-changing temperature range is higher than the signal-constant temperature range.

According to an embodiment of the present disclosure, the extended duplex forms a double strand at temperatures within the signal-constant temperature range in the **presence** of the target nucleic acid.

According to an embodiment of the present disclosure, the extended duplex dissociates at temperatures within the signal-changing temperature range in the presence of the target nucleic acid.

According to an embodiment of the present disclosure, the signal-changing temperature range depends on the **Tm** value of the extended duplex.

According to another aspect of the present disclosure, there is provided a method for detecting n target nucleic acids in a sample, comprising:
(a) detecting signals at *n* detection temperatures, while incubating with *n* compositions for detecting the n target nucleic acids, a sample suspected of containing at least one of the *n* target nucleic acids in a reaction vessel;
   wherein n is an integer of 2 or more,
   wherein the incubating comprises a plurality of reaction cycles and the detection of signals is carried out at at least one of the plurality of reaction cycles,
   wherein each of the n compositions for detecting the *n* target nucleic acids provides a signal change at a corresponding detection temperature among the n detection temperatures in the presence of a corresponding target nucleic acid, the signal change indicating the presence of a corresponding target nucleic acid,
   wherein a composition for detecting an *i*^{th} target nucleic acid among the *n* compositions for detecting the *n* target nucleic acids provides a signal change at an *i*^{th} detection temperature among the n detection temperatures and provides a constant signal at the other detection temperatures in the presence of the *i*^{th} target nucleic acid,
   wherein *i* represents an integer from 1 to *n,* and the *i*^{th} detection temperature is lower than a (*i*+1)^{th} detection temperature,
   wherein in the temperature range covering all of the n detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid,
   **wherein** the composition for detecting the *i*^{th} target nucleic acid is any one of:
      (i) an Under-Signal-Change (UnderSC) composition having a characteristic that the signal-changing temperature range is lower than the signal-constant temperature range,
      (ii) an Inter-Signal-Change (InterSC) composition having a characteristic that the signal-changing temperature range is higher than one of two signal-constant temperature ranges, and lower than the other of the two signal-constant temperature ranges, and
      (iii) an Over-Signal-Change (OverSC) composition having a characteristic that the signal-changing temperature range is higher than the signal-constant temperature range, and
   wherein the composition for detecting the *n*^{th} target nucleic acid among the *n* compositions for detecting the *n* target nucleic acids is a composition comprising a primer, a Q-PTO and a CTO, and;
(b) determining the presence of the *n* target nucleic acids from the signals detected in step (a), wherein the presence of the *i*^{th} target nucleic acid is determined by the signal change detected at the *i*^{th} detection temperature.

According to an embodiment of the present disclosure, the *i*^{th} detection temperature is selected within the signal-changing temperature range of the composition for detecting the *i*^{th} target nucleic acid, wherein the *i*^{th} detection temperature is not comprised in the signal-changing temperature ranges of the compositions for detecting the other target nucleic acids.

According to an embodiment of the present disclosure, the signal-changing temperature range of the composition for detecting the *i*^{th} target nucleic acid overlaps partially with the signal-changing temperature range of a composition for detecting a target nucleic acid having an adjacent detection temperature, and does not overlap with the signal-changing temperature range of a composition for detecting a target nucleic acid having a detection temperature that is not adjacent thereto.

According to an embodiment of the present disclosure, when *n is* 2, the composition for detecting the first target nucleic acid is an UnderSC or InterSC composition.

According to an embodiment of the present disclosure, when *n is* 3 or more, the **composition** for detecting the first target nucleic acid is an UnderSC or InterSC composition, and each of compositions for detecting target nucleic acids other than the first target nucleic acid and the *n*^{th} target nucleic acid is an InterSC composition.

According to an embodiment of the present disclosure, the composition for detecting the *i*^{th} target nucleic acid comprises a label that provides a signal dependent on the presence of the *i*^{th} target nucleic acid.

According to an embodiment of the present disclosure, the label is linked to an oligonucleotide or is incorporated into an oligonucleotide during the incubating.

According to an embodiment of the present disclosure, the composition for detecting the *i*^{th} target nucleic acid provides a duplex providing a signal change.

According to an embodiment of the present disclosure, the duplex providing the signal change has initially been included in the composition for detecting the *i*^{th} target nucleic acid.

According to an embodiment of the present disclosure, the duplex providing the signal change is generated by hybridization between a label-linked oligonucleotide and an oligonucleotide hybridizable with the label-linked oligonucleotide.

According to an embodiment of the present disclosure, the duplex providing the signal change is generated in incubating.

According to an embodiment of the present disclosure, the duplex providing the signal change is generated by hybridization between a label-linked oligonucleotide and the corresponding target nucleic acid.

According to an embodiment of the present disclosure, the duplex providing the signal change is generated by a cleavage reaction dependent on the presence of the corresponding target nucleic acid.

According to an embodiment of the present disclosure, the duplex providing the signal change comprises a label.

According to an embodiment of the present disclosure, the composition for detecting the *i*^{th} target nucleic acid provides a duplex providing a signal change, and the signal-changing temperature range of the composition for detecting the *i*^{th} target nucleic acid varies depending on the length and/or sequence of the duplex.

According to an embodiment of the present disclosure, the detection of signals is carried out at at least two of the plurality of reaction cycles.

According to an embodiment of the present disclosure, the signal change is measured using the signals detected at the at least two of the plurality of reaction cycles.

According to an embodiment of the present disclosure, the signal change at the *i*^{th} detection temperature is measured using a signal detected at the at least one of the plurality of reaction cycles and a reference signal value.

According to an embodiment of the present disclosure, the reference signal value is obtained from a reaction in the absence of the *i*^{th} target nucleic acid.

According to an embodiment of the present disclosure, the detection of a signal at each of the n detection temperatures is carried out using a single type of detector.

According to an embodiment of the present disclosure, the signals detected at the n detection temperatures are not differentiated from each other by the single type of detector.

According to an embodiment of the present disclosure, the incubating comprises a nucleic acid amplification reaction.

### Advantageous Effects

The features and advantages of the present disclosure are summarized as follows:
(a) According to the present disclosure, the Q-PTO hybridized with the target nucleic acid is cleaved to release a reporter-carrying fragment, and the reporter-carrying fragment hybridizes with the CTO to provide an extended strand generated in a target-dependent manner.
(b) In the present disclosure, the Q-PTO comprises a reporter molecule and a first quencher molecule, and the CTO comprises a second quencher molecule, wherein (i) prior to hybridization of the Q-PTO with the target nucleic acid and its cleavage, the reporter molecule of the Q-PTO is quenched by the first quencher molecule, and (ii) upon cleavage of the Q-PTO, the reporter molecule is included in the reporter-carrying fragment generated by the cleavage, and the reporter-carrying fragment generates an extended duplex through hybridization with the CTO and an extension reaction, while the reporter molecule remains quenched by the second quencher molecule. That is, the method according to the present disclosure is characterized in that the signal indicating the presence of the target nucleic acid is provided from the dissociation of the extended duplex, and that the temperature at which the signal indicating the presence of the target nucleic acid is provided can be adjusted by controlling the temperature at which the extended duplex dissociates.
(c) Particularly, the present disclosure has an advantage in that a signal detection temperature can be selected in a higher temperature range by utilizing not only the cleavage of the Q-PTO and the hybridization between the reporter-carrying fragment and the CTO, but also the extension of the reporter-carrying fragment after hybridization. Specifically, when a signal is provided solely from the dissociation of a hybrid (duplex) between the reporter-carrying fragment and the CTO, the temperature range available for selecting a signal detection temperature is dependent on the Tm of the hybrid, thereby limiting the selection of a signal detection temperature in a higher range. However, the method according to the present disclosure allows for adjusting the signal detection temperature (i.e., the temperature at which the duplex dissociates) by the length and/or sequence of the reporter-carrying fragment as well as the extended sequence, thereby enabling signal detection at a higher temperature.
(d) In a method for detecting multiple target nucleic acids, wherein signals are provided only for a single target nucleic acid at a single temperature in a single reaction vessel, the Q-PTOCE assay according to the present disclosure can be used as a method and/or composition for detecting the target nucleic acid having the highest detection temperature.

### Description of Drawings

FIG. 1 schematically illustrates the Q-PTOCE assay of the present disclosure.

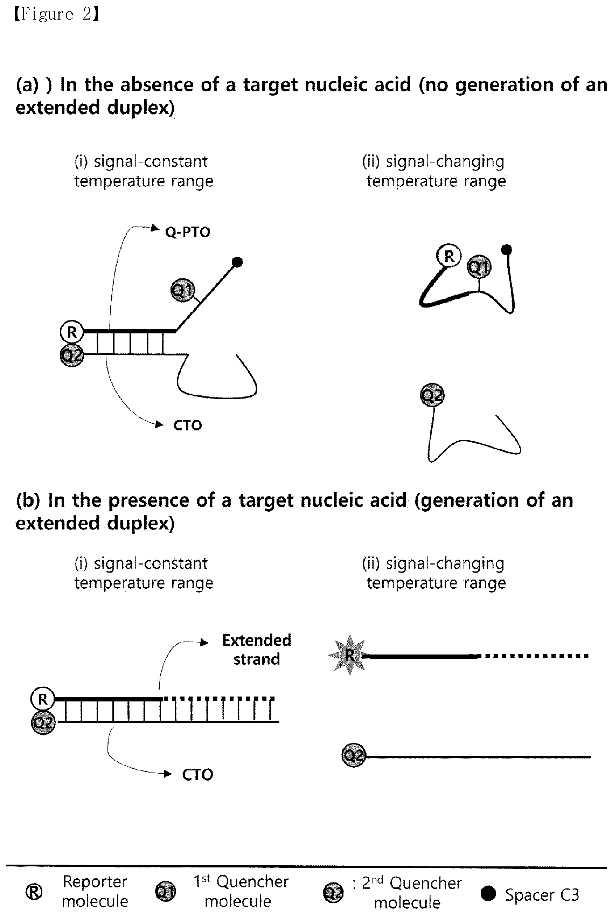

FIG. 2 shows the conformations of oligonucleotides depending on the presence of the target nucleic acid and temperature. In particular, in FIG. 2, the Q-PTO has a reporter molecule and a first quencher molecule positioned in close three-dimensional proximity to each other due to its conformational structure (e.g., random coil). Specifically, FIG. 2(a) shows the conformations of the Q-PTO and the CTO in (i) a signal-constant temperature range and (ii) a signal-changing temperature range, when the target nucleic acid is absent or before the Q-PTOCE composition reacts with the target nucleic acid. FIG. 2(b) shows the conformations of the extended duplex in (i) a signal-constant temperature range and (ii) a signal-changing temperature range after the Q-PTOCE composition has reacted with the target nucleic acid.

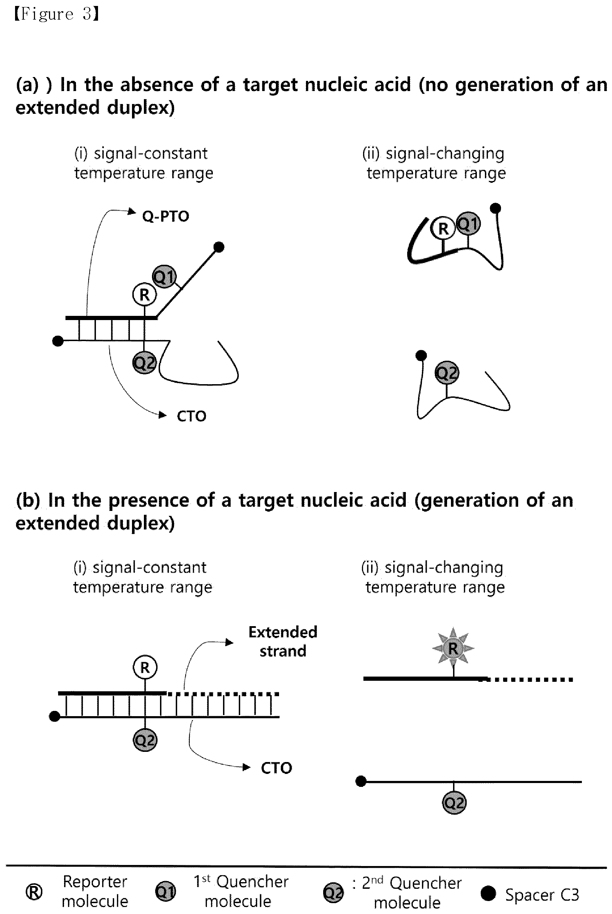

FIG. 3 shows the conformations of oligonucleotides depending on the presence of the target nucleic acid and temperature. In particular, in FIG. 3, the Q-PTO has a reporter molecule and a first quencher molecule positioned sufficiently close to each other to allow interaction. More particularly, FIG. 3(a) shows the conformations of the Q-PTO and the CTO in (i) a signal-constant temperature range and (ii) a signal-changing temperature range, when the target nucleic acid is absent or before the Q-PTOCE composition reacts with the target nucleic acid. FIG. 3(b) shows the conformations of the extended duplex in (i) a signal-constant temperature range and (ii) a signal-changing temperature range after the Q-PTOCE composition has reacted with the target nucleic acid.
FIG. 4 shows the amount ratio and melt curve of the reaction product obtained from the reaction of the Q-PTOCE composition with the target nucleic acid. FIG. 4(a) shows (i) the amount ratio (or abundance ratio) of the Q-PTO and the CTO and (ii) the amount ratio (or abundance ratio) of the extended duplex, as well as their melt curves, in the early, intermediate, and late cycles when all or a portion of steps (a)-(d) of the Q-PTOCE assay are repeated (e.g., target nucleic acid amplification). FIG. 4(b) presents merged melt curves in each cycle in FIG. 4(a).
FIG. 5 shows the amplification curve results at different temperatures in real-time PCR of Example 1.
FIG. 6 schematically shows the signal generation mechanism of two compositions for detecting target nucleic acids used in Example 2, depending on the presence of the target nucleic acids.
FIG. 7 shows the amplification curves at different temperatures in multiplex real-time PCR of Example 2.

### Best Mode

The present inventors have endeavored to develop a novel method for detecting target nucleic acids in real time with improved convenience and high efficiency. As a result, the present inventors have established a novel protocol for detecting a target nucleic acid, in which target nucleic acid detection is achieved not only through probe hybridization but also through enzymatic reactions of cleavage and extension, by utilizing two quencher molecules and a reporter molecule.

### I. Method for Detecting a Target Nucleic Acid by Q-PTOCE Assay

In one aspect, the present disclosure provides a method for detecting a target nucleic acid in a sample by Quenching-PTO Cleavage and Extension (Q-PTOCE) assay, comprising:
(a) hybridizing a primer and a Quenching-Probing and Tagging Oligonucleotide (Q-PTO) with the target nucleic acid;
   wherein the primer comprises a hybridizing nucleotide sequence to a first region of the target nucleic acid,
   wherein the Q-PTO comprises in a 5' to 3' direction: (i) a 5'-tagging portion comprising a non-hybridizing nucleotide sequence to the target nucleic acid, and (ii) a hybridizing nucleotide sequence to a second region of the target nucleic acid,
   wherein the 5'-tagging portion of the Q-PTO is not hybridized with the target nucleic acid and the 3'-targeting portion of the Q-PTO is hybridized with the target nucleic acid,
   wherein the Q-PTO has a first quencher molecule in the 3'-targeting portion and a reporter molecule located at a position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in a reporter-carrying fragment,
   wherein the primer is located upstream of the Q-PTO;
(b) contacting the resultant of the step (a) to a DNA polymerase having 5' nuclease activity under conditions for cleavage of the Q-PTO to provide the reporter-carrying fragment;
   wherein the primer is extended to induce cleavage of the Q-PTO by the DNA polymerase having 5' nuclease activity such that the cleavage releases the reporter-carrying fragment;
(c) hybridizing the reporter-carrying fragment with a Capturing and Templating Oligonucleotide (CTO);
   wherein the CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment,
   wherein the capturing portion of the CTO is hybridized with the reporter-carrying fragment,
   wherein the CTO has a second quencher molecule located at a position such that upon hybridization of the capturing portion of the CTO with the reporter-carrying fragment, the second quencher molecule is in close proximity to the reporter molecule, thereby causing the second quencher molecule to quench a signal from the reporter molecule;
(d) performing an extension reaction using the resultant of the step (c) and the DNA polymerase having 5' nuclease activity;
   wherein the reporter-carrying fragment hybridized with the capturing portion of the CTO is extended to generate an extended strand comprising an extended sequence complementary to the templating portion of the CTO, thereby forming an extended duplex between the extended strand and the CTO; and
(e) detecting the presence of the extended strand;
   whereby the presence of the extended strand indicates the presence of the target nucleic acid.

Terms such as "first," "second," "A," "B," "(a)," "(b)," "(i)," and "(ii)" may be used to describe the components of the present disclosure. These terms are merely used to distinguish one component from another and do not limit the nature, order, sequence, or other characteristics of the components.

The method of the present disclosure utilizes consecutive events resulting from probe hybridization, namely, cleavage and extension of the Q-PTO, which is referred to as "Quenching-PTO Cleavage and Extension (Q-PTOCE)" assay.

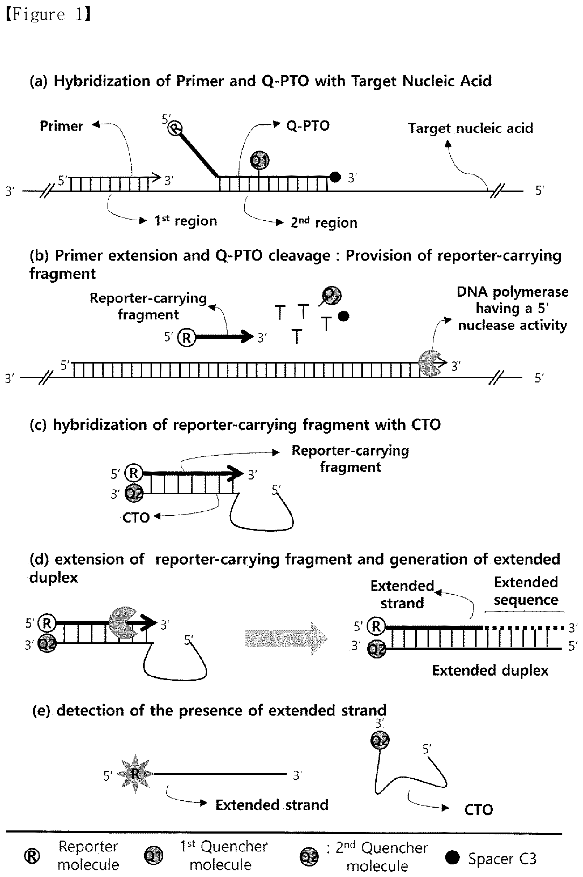

The Q-PTOCE assay of the present disclosure is schematically illustrated in FIG. 1. A detailed description of the Q-PTOCE assay is as follows:

### Step (a): Hybridization of the Primer and Q-PTO with the Target Nucleic Acid

According to the method of the present disclosure, the target nucleic acid is hybridized with a primer and a Quenching-Probing and Tagging Oligonucleotide (Q-PTO) (see FIG. 1(a)).

The term "target nucleic acid", "target nucleic acid sequence", or "target sequence" as used herein refers to a nucleic acid sequence to be detected or quantified. The target nucleic acid includes a single-strand as well as a double-strand. The target nucleic acid includes, not only a sequence initially present in a nucleic acid sample, but also a sequence newly generated in a reaction.

The target nucleic acid includes any DNA (gDNA and cDNA) RNA molecules, and their hybrids (chimeric nucleic acids). The target nucleic acid may be in a double-stranded or single-stranded form.

Target nucleic acids include any naturally occurring prokaryotic, eukaryotic (for example, protozoans and parasites, fungi, yeast, higher plants, lower animals, and higher animals, including mammals and humans) or viral (for example, Herpes virus, HIV, influenza virus, Epstein-Barr virus, hepatitis virus, polio virus, etc.) or viroid nucleic acid. In addition, the nucleic acid molecule may be any nucleic acid molecule which is produced or can be produced by recombination, or any nucleic acid molecule which is or can be chemically synthesized. As such, the nucleic acid sequence may or may not be found in nature. The target nucleic acid sequence may be of a known or unknown sequence.

As used herein, the term "sample" refers to any cell, tissue, or fluid from a biological source, or any other medium that can advantageously be evaluated according to the present invention, and includes virus, bacteria, tissues, cells, blood, serum, plasma, lymph, milk, urine, feces, intraocular fluid, saliva, semen, brain extract, spinal fluid, appendix, spleen and tonsil tissue extracts, amniotic fluid, ascites, and non-biological samples (e.g., food and water). In addition, the sample includes naturally occurring nucleic acid molecules isolated from a biological source, and synthesized nucleic acid molecules. Further, the sample may be a lysate, an extract or an isolated target nucleic acid itself of a specific specimen.

As used herein, the term "primer" refers to an oligonucleotide that is capable of acting as a point of initiation of synthesis when placed under conditions in which synthesis of primer extension product which is complementary to a target nucleic acid strand (template) is induced, *i.e.,* in the presence of nucleotides and an agent for polymerization, such as DNA polymerase, and at a suitable temperature and pH.

The term "probe" as used herein refers to a single-stranded nucleic acid molecule comprising one or more portions substantially complementary to a target nucleic acid sequence.

Particularly, the primer and probe are single-stranded deoxyribonucleotide molecules. The primer or probe used in the present disclosure may comprise naturally occurring dNMPs (i.e., dAMP, dGMP, dCMP, and dTMP), modified nucleotides, or non-natural nucleotides. Additionally, the primer or probe may comprise ribonucleotides.

The primer should be sufficiently long to prime the synthesis of an extension product in the presence of a polymerization agent. The exact length of the primer will vary depending on many factors, including temperature, application, and the source of the primer. As used herein, the terms "annealing" or "priming" refer to the apposition of an oligodeoxynucleotide or nucleic acid to a template nucleic acid, wherein such apposition allows a polymerase to polymerize nucleotides to form a nucleic acid molecule complementary to the template nucleic acid or a portion thereof.

The term "hybridize", "hybridizing" or "hybridization" as used herein refers to the formation of double strands by noncovalent association between two complementary single-stranded polynucleotides under certain hybridization conditions or stringent conditions.

In particular, the expression herein that one oligonucleotide "comprises a hybridizing nucleotide sequence" to another oligonucleotide means that all or a portion of one oligonucleotide has a complementary nucleotide sequence necessary for hybridization with all or a portion of another oligonucleotide.

The term "complementary" is used herein to mean that primers or probes are sufficiently complementary to hybridize selectively to a target nucleic acid under the designated annealing conditions or stringent conditions, encompassing the terms "substantially complementary" and "perfectly complementary", particularly perfectly complementary.

In contrast, the term "non-complementary" is used herein to mean that primers or probes are sufficiently non-complementary not to hybridize selectively to a target nucleic acid under the designated annealing conditions or stringent conditions, encompassing the terms "substantially non-complementary" and "perfectly non-complementary", particularly perfectly non-complementary.

Further, when referring to hybridization of a portion of one oligonucleotide to another oligonucleotide, the portion of one oligonucleotide can be regarded as an individual oligonucleotide.

The hybridization may occur between two nucleic acid strands perfectly matched or substantially matched with some mismatches (e.g., 1-4 mismatches). The complementarity for hybridization may depend on hybridization conditions, particularly temperature.

The hybridization between two oligonucleotides (e.g., hybridization of the primer and the target nucleic acid or hybridization of the Q-PTO and the target nucleic acid) may be carried out under suitable hybridization conditions routinely determined through optimization procedures. Conditions such as temperature, concentration of components, hybridization and washing times, buffer components, and their pH and ionic strength may be varied depending on various factors, including the length and GC content of the oligonucleotide and the target nucleotide sequence. For instance, when a relatively short oligonucleotide is used, it is preferable that low-stringency conditions are adopted. The detailed conditions for hybridization can be found in Joseph Sambrook, et al., Molecular Cloning, A Laboratory Manual, Cold Spring Harbor Laboratory Press, Cold Spring Harbor, N.Y. (2001); and M. L. M. Anderson, Nucleic Acid Hybridization, Springer-Verlag New York Inc. N.Y. (1999).

There is no intended distinction between the terms "annealing" and "hybridizing", and these terms will be used interchangeably.

The term "Quenching-Probing and Tagging Oligonucleotide (Q-PTO)" as used herein refers to an oligonucleotide comprising, in a 5' to 3' direction: (i) a 5'-tagging portion comprising a non-hybridizing nucleotide sequence to the target nucleic acid, and (ii) a hybridizing nucleotide sequence to a second region of the target nucleic acid; wherein the 5'-tagging portion of the Q-PTO is not hybridized with the target nucleic acid, and the 3'-targeting portion of the Q-PTO is hybridized with the target nucleic acid. That is, the Q-PTO of the present disclosure comprises the following two portions: (i) a 5'-tagging portion, which comprises a non-hybridizing nucleotide sequence to the target nucleic acid and is released from the Q-PTO (specifically, the 3'-targeting portion of the Q-PTO) upon hybridization of the Q-PTO with the target nucleic acid followed by nucleic acid cleavage; and (ii) a 3'-targeting portion that functions as a probe.

In one embodiment, the hybridization in step (a) is carried out under stringent conditions in which the 3'-targeting portion of the Q-PTO is hybridized with the second region of the target nucleic acid, while the 5'-tagging portion of the Q-PTO is not hybridized with the target nucleic acid.

In the present disclosure, the Q-PTO comprises a first quencher molecule and a reporter molecule. Specifically, the Q-PTO has a first quencher molecule in the 3'-targeting portion and a reporter molecule located at a position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in a reporter-carrying fragment.

The reporter molecule and the quencher molecule used in the present disclosure are interactive labels.

As a representative example of the interactive label system, the FRET (fluorescence resonance energy transfer) label system includes a fluorescent reporter molecule (donor molecule) and a quencher molecule (acceptor molecule). In FRET, the energy donor is fluorescent, but the energy acceptor may be fluorescent or non-fluorescent. In another form of interactive label systems, the energy donor is non-fluorescent, e.g., a chromophore, and the energy acceptor is fluorescent. In yet another form of interactive label systems, the energy donor is luminescent, e.g., bioluminescent, chemiluminescent, or electrochemiluminescent, and the acceptor is fluorescent. The interactive label system includes a label pair based on "contact-mediated quenching" (Salvatore et al., Nucleic Acids Research, 2002 (30) no.21 e122 and Johansson et al., J. AM. CHEM. SOC 2002 (124) pp 6950-6956). The interactive label system includes any label system in which signal change is induced by interaction between at least two molecules (e.g., dyes).

The reporter molecule and the quencher molecule useful as interactive labels may include any molecule known in the art. Examples of those are: Cy2^{™} (506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), Magnesium Green^{™}(531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™} (576), Pyronin Y (580), Rhodamine B (580), TAMRA (582), Rhodamine Red^{™} (590), Cy3.5^{™} (596), ROX (608), Calcium Crimson^{™} (615), Alexa^{™} 594 (615), Texas Red (615), Nile Red (628), YO-PRO^{™}-3 (631), YOYO^{™}-3 (631), Rphycocyanin (642), C-Phycocyanin (648), TO-PRO^{™}-3 (660), TOTO3 (660), DiD DiIC(5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705) and Quasar 705 (610). The numeric in parenthesis is a maximum emission wavelength in nanometer. In a specific embodiment, the reporter molecule and the quencher molecule include JOE, FAM, TAMRA, ROX and fluorescein-based label.

Suitable reporter-quencher pairs are disclosed in various publications as follows: Pesce et al., editors, Fluorescence Spectroscopy (Marcel Dekker, New York, 1971); White et al., Fluorescence Analysis: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996); U.S. Pat. Nos. 3,996,345 and 4,351,760.

In one embodiment, a non-fluorescent black quencher molecule capable of quenching fluorescence of a wide wavelength or a specific wavelength may be used. Examples of non-fluorescent black quencher molecules include BHQ and DABCYL.

In the FRET label applied to the present disclosure, the reporter comprises a donor of FRET and the quencher comprises the remaining partner (acceptor) of FRET. For example, a fluorescein dye is used as a reporter and a rhodamine dye is used as a quencher.

The Q-PTO does not require a specific length. For example, the length of the Q-PTO may be 15-150 nucleotides, 15-100 nucleotides, 15-80 nucleotides, 15-60 nucleotides, 15-40 nucleotides, 20-150 nucleotides, 20-100 nucleotides, 20-80 nucleotides, 20-60 nucleotides, 20-50 nucleotides, 30-150 nucleotides, 30-100 nucleotides, 30-80 nucleotides, 30-60 nucleotides, 30-50 nucleotides, 35-100 nucleotides, 35-80 nucleotides, 35-60 nucleotides, or 35-50 nucleotides.

The 3'-targeting portion of the Q-PTO may have any length as long as it specifically hybridizes with the target nucleic acid. For example, the length of the 3'-targeting portion of the Q-PTO may be 10-100 nucleotides, 10-80 nucleotides, 10-50 nucleotides, 10-40 nucleotides, 10-30 nucleotides, 15-100 nucleotides, 15-80 nucleotides, 15-50 nucleotides, 15-40 nucleotides, 15-30 nucleotides, 20-100 nucleotides, 20-80 nucleotides, 20-50 nucleotides, 20-40 nucleotides, or 20-30 nucleotides.

The 5'-tagging portion of the Q-PTO may have any length as long as it can specifically hybridize to the templating portion of the CTO and be extended. For example, the length of the 5'-tagging portion of the Q-PTO may be 5-50 nucleotides, 5-40 nucleotides, 5-30 nucleotides, 5-20 nucleotides, 10-50 nucleotides, 10-40 nucleotides, 10-30 nucleotides, 10-20 nucleotides, 15-50 nucleotides, 15-40 nucleotides, 15-30 nucleotides, or 15-20 nucleotides.

The 3'-end of the Q-PTO may have a 3'-OH terminal. In particular, the 3'-end of the Q-PTO may be "blocked" to prevent extension. Blocking can be achieved by conventional methods. For example, blocking can be performed by adding a chemical moiety such as biotin, label, phosphate group, alkyl group, non-nucleotide linker, phosphorothioate, or alkane-diol residue to the 3'-hydroxyl group of the last nucleotide. Alternatively, blocking can be performed by removing the 3'-hydroxyl group of the last nucleotide or by using a nucleotide with no 3'-hydroxyl group such as dideoxynucleotide.

Alternatively, the Q-PTO may be designed to have a hairpin structure.

The non-hybridization between the 5'-tagging portion of the Q-PTO and the target nucleic acid refers to non-formation of a stable double-strand between them under certain hybridization conditions. In one embodiment, the 5'-tagging portion of the Q-PTO not involved in the hybridization with the target nucleic acid forms a single-strand.

The primer used in the present disclosure may be refers to an upstream primer or a forward primer located upstream of the Q-PTO. When the target nucleic acid is double stranded, the primer and the Q-PTO are hybridized with one strand of the double-stranded target nucleic acid, and the Q-PTO is positioned downstream of the primer. The primer is hybridized with a specific portion (i.e., a first region of the target nucleic acid) in the 3'-direction relative to the portion (i.e., a second region of the target nucleic acid) of the target nucleic acid strand to which the Q-PTO is hybridized.

In one embodiment, when the target nucleic acid is double-stranded, one strand of the double-stranded target nucleic acid comprises a first region and a second region of the target nucleic acid. Specifically, the target nucleic acid comprises in a 3' to 5' direction: (i) a first region to be hybridized with the primer, and (ii) a second region to be hybridized with the Q-PTO (see FIG. 1(a)).

In one embodiment, the method is performed in the presence of an additional primer that pairs with and amplifies the target nucleic acid. The additional primer may be referred to as a downstream primer or a reverse primer. The additional primer further generates the target nucleic acid that is hybridized with the Q-PTO, thereby enhancing the sensitivity of target detection.

In one embodiment, when the primer and the additional primer are used, a DNA polymerase with 5' nuclease activity, which is used in step (b) described below, is used for the extension of the two primers, or additionally, a template-dependent nucleic acid polymerase is used

In one embodiment, the primer and/or the additional primer has a dual priming oligonucleotide (DPO) structure. Oligonucleotides with a DPO structure exhibit significantly improved target specificity compared to conventional primers and probes (see WO 2006/095981; Chun et al., Dual priming oligonucleotide system for the multiplex detection of respiratory viruses and SNP genotyping of the CYP2C19 gene, Nucleic Acid Research, 35:6e40 (2007)).

### Step (b): Provision of the reporter-carrying fragment

Afterwards, the resultant of step (a) is contacted to a DNA polymerase having 5' nuclease activity under conditions for cleavage of the Q-PTO. The primer is extended to induce cleavage of the Q-PTO by the DNA polymerase having 5' nuclease activity such that the cleavage releases a reporter-carrying fragment (see FIG. 1(b)).

The phrase 'conditions for cleavage of the Q-PTO' as used herein refers to conditions sufficient to cleave the Q-PTO hybridized with the target nucleic acid by a DNA polymerase having 5' nuclease activity, including temperature, pH, ionic strength, buffer, length and sequence of oligonucleotides, and enzymes. For example, when Taq DNA polymerase is used as the DNA polymerase having 5' nuclease activity, the conditions for cleavage of the Q-PTO include Tris-HCl buffer, KCI, MgCl₂, and temperature.

When the Q-PTO is hybridized with the target nucleic acid, its 3'-targeting portion is involved in the hybridization, while its 5'-tagging portion does not hybridize with the target nucleic acid and forms a single strand (see FIG. 1(a)). As such, an oligonucleotide comprising both single-stranded and double-stranded structures can be cleaved using an enzyme having 5' nuclease activity by various techniques known in the art. In one embodiment, the Q-PTO hybridized with the target nucleic acid may be cleaved by an enzyme having 5' nuclease activity, and specifically, by a DNA polymerase having 5' nuclease activity.

In one embodiment, extension of a primer hybridized to a first region of the target nucleic acid induces cleavage of the Q-PTO by a DNA polymerase having 5' nuclease activity. Specifically, the primer is hybridized with the target nucleic acid at a position spaced apart from the Q-PTO, and the DNA polymerase having 5' nuclease activity facilitates extension of the primer, wherein the DNA polymerase having 5' nuclease activity bound to the extension product of the primer cleaves the Q-PTO.

In another embodiment, the primer is hybridized in close proximity to the Q-PTO such that it induces cleavage of the Q-PTO by a DNA polymerase having 5' nuclease activity, and the DNA polymerase having 5' nuclease activity bound to the primer cleaves the Q-PTO without extension reaction.

Accordingly, cleavage of the Q-PTO may be accomplished in two different manners: (i) primer extension-dependent cleavage induction; and (ii) primer extension-independent cleavage induction.

Depending on the two cleaving manner, the primer may be positioned relative to the Q-PTO. The primer may be positioned at a distance from the Q-PTO such that it is sufficient to induce Q-PTO cleavage in an extension-dependent manner. In other words, the first region and the second region of the target nucleic acid may be spaced apart from each other. Alternatively, the primer may be positioned in close proximity to the Q-PTO such that it is sufficient to induce Q-PTO cleavage in an extension-independent manner. In other words, the first region and the second region of the target nucleic acid may be in close proximity to each other.

As used herein, the term "adjacent" with referring to positions or locations means that the primer is located adjacent to the 3'-targeting portion of the Q-PTO to form a nick. Also, the term means that the primer is located 1-30 nucleotides, 1-20 nucleotides or 1-15 nucleotides apart from the 3'-targeting portion of the Q-PTO.

As used herein, the term 'distant,' with referring to positions or locations, includes any position or location sufficient to allow an extension reaction to occur.

In one embodiment, the primer is positioned distant from the Q-PTO such that it is sufficient to induce cleavage of the Q-PTO in an extension-dependent manner.

In one embodiment, conventional technologies for primer-induced cleavage reactions may be applied to the present disclosure, so long as the primer hybridized with the first region of the target nucleic acid induces cleavage of the Q-PTO hybridized with the second region of the target nucleic acid, thereby releasing a fragment comprising the 5'-tagging portion of the Q-PTO or a part thereof. For example, U.S. Patent Nos. 5,210,015, 5,487,972, 5,691,142, 5,994,069, and 7,381,532, as well as U.S. Application Publication No. 2008-0241838, may be applied to the present disclosure.

The cleavage site of the Q-PTO varies depending on the type of primer, the hybridization position of the primer, and the cleavage conditions (see U.S. Patent Nos. 5,210,015, 5,487,972, 5,691,142, 5,994,069, and 7,381,532, or U.S. Application Publication No. 2008-0241838).

As described above, many conventional techniques can be used for the cleavage reaction of the Q-PTO to release a fragment comprising the 5'-tagging portion or a part thereof.

Briefly, three cleavage sites may exist in step (b). The first cleavage site is the junction site between the hybridized portion of the Q-PTO (3'-targeting portion) and the non-hybridized portion (5'-tagging portion). The second cleavage site is a position located several nucleotides apart in the 3'-direction from the 3'-end of the 5'-tagging portion of the Q-PTO, which may be at the 5'-end part of the 3'-targeting portion of the Q-PTO. The third cleavage site is a position located several nucleotides apart in the 5'-direction from the 3'-end of the 5'-tagging portion of the Q-PTO.

In one embodiment, the initial site for the cleavage of the Q-PTO by a DNA polymerase having 5' nuclease activity upon extension of the primer is a point where the double-stranded region between the Q-PTO and the target nucleic acid starts, or a site 1 to 3 nucleotides apart from this starting point.

In this regard, the phrase "a reporter-carrying fragment comprising the 5'-tagging portion of the Q-PTO or a part thereof" in the context of cleavage of the Q-PTO by a DNA polymerase having 5' nuclease activity as used herein is used to encompass (i) the 5'-tagging portion comprising a reporter molecule, (ii) the 5'-tagging portion comprising a reporter molecule and a 5'-end part of the 3'-targeting portion (e.g., the first nucleotide at the 5'-end of the 3'-targeting portion, the first to second nucleotides at the 5'-end of the 3'-targeting portion, the first to third nucleotides at the 5'-end of the 3'-targeting portion, the first to fourth nucleotides at the 5'-end of the 3'-targeting portion, or the first to fifth nucleotides at the 5'-end of the 3'-targeting portion), and (iii) a part of the 5'-tagging portion comprising a reporter molecule. The phrase "a fragment comprising the 5'-tagging portion of the Q-PTO or a part thereof" may be referred to as "a reporter-carrying fragment".

The term "part" used in conjunction with the Q-PTO or the CTO such as the part of the 5'-tagging portion of the Q-PTO, the 5'-end part of the 3'-targeting portion of the Q-PTO and the 5'-end part of the capturing portion of the CTO refers to a nucleotide sequence composed of 1-40, 1-30, 1-20, 1-15, 1-10 or 1-5 nucleotides, particularly 1, 2, 3 or 4 nucleotides.

As described above, the Q-PTO may be cleaved at a site spaced apart in the 3'-direction from the 3'-end of the 5'-tagging portion of the Q-PTO. The cleavage site may be located at the 5'-end part of the 3'-targeting portion of the Q-PTO. When the reporter-carrying fragment comprises the 5'-end part of the 3'-targeting portion of the Q-PTO, the portion of the CTO hybridized with the 5'-end part of the 3'-targeting portion may comprise a universal base, a degenerate sequence, or a combination thereof. For example, when the Q-PTO is cleaved at a site spaced apart by one nucleotide in the 3'-direction from the 3'-end of the 5'-tagging portion of the Q-PTO, it is advantageous that the 5'-end part of the capturing portion of the CTO comprises a universal base to allow hybridization with the nucleotide. When the Q-PTO is cleaved at a site spaced apart by two nucleotides in the 3'-direction from the 3'-end of the 5'-tagging portion of the Q-PTO, it is advantageous that the 5'-end of the capturing portion of the CTO comprises a degenerate sequence, and the nucleotide adjacent thereto in the 3'-direction comprises a universal base. As such, when cleavage of the Q-PTO occurs at various sites within the 5'-end part of the 3'-targeting portion, it is useful to use a universal base and a degenerate sequence in the CTO.

In one embodiment, the first quencher molecule of the Q-PTO is linked at a position such that it is not included in the fragment (i.e., the reporter-carrying fragment) released by the cleavage reaction of the Q-PTO in step (b). For example, when the cleavage site of the Q-PTO is at the junction between the hybridized portion (3'-targeting portion) and the non-hybridized portion (5'-tagging portion), the first quencher molecule is located within the 3'-targeting portion of the Q-PTO. In another embodiment, when the cleavage site of the Q-PTO is at a position spaced apart by several nucleotides in the 3'-direction from the 3'-end of the 5'-tagging portion of the Q-PTO (i.e., located at the 5'-end part of the 3'-targeting portion of the Quenching-PTO), the first quencher molecule may be located in the remaining portion of the 3'-targeting portion of the Q-PTO, excluding the 5'-end part of the 3'-targeting portion. In yet another embodiment, when the cleavage site of the Q-PTO is at a position spaced apart by several nucleotides in the 5'-direction from the 3'-end of the 5'-tagging portion of the Q-PTO, the first quencher molecule may be located within the 3'-end part of the 5'-tagging portion and the 3'-targeting portion of the Q-PTO.

In one embodiment, before the Q-PTO is cleaved by a DNA polymerase having 5' nuclease activity in step (b), the reporter molecule and the first quencher molecule of the Q-PTO are in close proximity to each other, allowing the first quencher molecule to intramolecularly quench the signal from the reporter molecule. Specifically, (i) the reporter molecule and the first quencher molecule of the Q-PTO may be three-dimensionally adjacent to each other due to the conformational structure of the Q-PTO (e.g., random coil or hairpin structure) (see FIG. 2), or (ii) the reporter molecule and the first quencher molecule may be positioned within the Q-PTO at a sufficiently close distance to allow interaction (e.g., the reporter molecule and the first quencher molecule are immediately adjacent within the Q-PTO or are positioned in close proximity with a slight spacing) (see FIG. 3), thereby enabling the first quencher molecule to intramolecularly quench the signal from the reporter molecule.

In one embodiment, on the Q-PTO, the first quencher molecule may be positioned 11 to 30 nucleotides apart from the reporter molecule. Specifically, the first quencher molecule may be positioned 15 to 30 nucleotides, 18 to 30 nucleotides, 20 to 30 nucleotides, 23 to 30 nucleotides, 25 to 30 nucleotides, 11 to 20 nucleotides, 15 to 20 nucleotides, 18 to 20 nucleotides, or 11 to 15 nucleotides apart from the reporter molecule. In this case, due to the conformational structure of the Q-PTO (e.g., random coil or hairpin structure), the reporter molecule and the first quencher molecule, which were spaced apart from each other, are positioned three-dimensionally adjacent to each other, whereby the first quencher molecule quenches the signal from the reporter molecule.

In another embodiment, on the Q-PTO, the first quencher molecule may be located immediately adjacent to or in close proximity to the reporter molecule. For example, they may be spaced apart by 1 to 10 nucleotides. Specifically, they may be spaced apart by 2 to 10 nucleotides, 3 to 10 nucleotides, 2 to 5 nucleotides, 3 to 5 nucleotides, 2 to 4 nucleotides, or 3 to 4 nucleotides. In this case, the first quencher molecule and the reporter molecule are sufficiently close to each other to allow interaction, thereby enabling the first quencher molecule to quench the signal from the reporter molecule.

In one embodiment, the reporter molecule and the first quencher molecule of the Q-PTO are located at positions such that they are separated from each other by the cleavage reaction in step (b). Specifically, the reporter molecule is included in the reporter-carrying fragment released by the cleavage reaction, and the first quencher molecule is included in the remaining portion of the Q-PTO excluding the released reporter-carrying fragment. As a result, the first quencher molecule and the reporter molecule are separated from each other, and the first quencher molecule unquenches the signal from the reporter molecule.

In the present disclosure, a suitable DNA polymerase having 5' nuclease activity is a thermostable DNA polymerase derived from various bacterial species, including *Thermus aquaticus* (Taq), *Thermus thermophilus* (Tth), *Thermus filiformis, Thermis flavus, Thermococcus literalis, Thermus antranikianii, Thermus caldophilus, Thermus chliarophilus, Thermus flavus, Thermus igniterrae, Thermus lacteus, Thermus oshimai, Thermus ruber, Thermus rubens, Thermus scotoductus, Thermus silvanus, Thermus species* Z05, *Thermus species sps 17, Thermus thermophilus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosipho africanus, Pyrococcus woesei, Pyrococcus horikoshii Pyrococcus abyssi, Pyrodictium occultum, Aquifex pyrophilus,* and *Aquifex aeolieus.* Most preferably, the thermostable DNA polymerase is Taq polymerase.

Alternatively, the present disclosure may use a DNA polymerase having 5' nuclease activity that is modified to have reduced polymerase activity.

In one embodiment, the conditions for cleavage of the Q-PTO include an extension reaction of the primer.

In one embodiment, a template-dependent polymerase is used for the extension of the primer, and the template-dependent polymerase is the same enzyme as the DNA polymerase having 5' nuclease activity.

Alternatively, a template-dependent polymerase may be used for the extension of the primer, and the template-dependent polymerase is an enzyme different from the DNA polymerase having 5' nuclease activity.

### Step (c): hybridization of the reporter-carrying fragment with the CTO

The reporter-carrying fragment released from the Q-PTO is hybridized with a Capturing and Templating Oligonucleotide (CTO) (see FIG. 1(c)).

The CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment, wherein the capturing portion of the CTO is hybridized with the reporter-carrying fragment.

The CTO has a second quencher molecule located at a position such that, upon hybridization of the capturing portion of the CTO with the reporter-carrying fragment, the second quencher molecule is in close proximity to the reporter molecule, thereby quenching a signal from the reporter molecule (see FIG. 1(c), FIG. 2, or FIG. 3).

In one embodiment, upon hybridization between the CTO and the reporter-carrying fragment, the second quencher molecule may be linked to a position on the CTO that is opposite to the position where the reporter molecule is linked on the reporter-carrying fragment. For example, when the reporter molecule is linked to the 3'-end of the reporter-carrying fragment, the second quencher molecule of the CTO may be linked to a nucleotide that hybridizes with the 3'-end nucleotide of the reporter-carrying fragment, and upon hybridization between the two, the second quencher molecule of the CTO intermolecularly quenches the reporter molecule of the reporter-carrying fragment.

In one embodiment, as long as the second quencher molecule of the CTO is capable of quenching a signal from the reporter molecule of the reporter-carrying fragment, the second quencher molecule may be located at a position spaced apart by several nucleotides from the site of the CTO opposite to the site of the reporter-carrying fragment to which the reporter molecule is linked. For example, the second quencher molecule may be linked to a position spaced apart by 1 to 5 nucleotides from the complementary nucleotide on the CTO that is opposite to the specific nucleotide of the reporter-carrying fragment to which the reporter molecule is linked.

In one embodiment, the second quencher molecule of the CTO is of the same type as the first quencher molecule of the Q-PTO. For example, when the first quencher molecule is a non-fluorescent black quencher molecule (e.g., BHQ), the second quencher molecule may also be a non-fluorescent black quencher molecule of the same type (e.g., BHQ). When the first quencher molecule is a fluorescent acceptor molecule (e.g., rhodamine dye), the second quencher molecule may also be a fluorescent acceptor molecule of the same type (e.g., rhodamine dye).

In another embodiment, the second quencher molecule of the CTO is of a different type from the first quencher molecule of the Q-PTO.

The CTO serves as a template for extension of the reporter-carrying fragment. The reporter-carrying fragment as a primer is hybridized with the CTO and is extended to generate an extended duplex.

The templating portion of the CTO may comprise any sequence, as long as it is non-complementary to the 5'-tagging portion and the 3'-targeting portion of the Q-PTO. In addition, the templating portion of the CTO may comprise any sequence, as long as it can serve as a template for extension of the reporter-carrying fragment released from the Q-PTO.

The capturing portion of the CTO comprises a hybridizing nucleotide sequence to the reporter-carrying fragment. Alternatively, the capturing portion of the CTO comprises a hybridizing nucleotide sequence to the 5'-tagging portion of the Q-PTO.

As described above, when the reporter-carrying fragment comprising the 5'-tagging portion of the Q-PTO is released, it is preferable that the capturing portion of the CTO is designed to comprise a hybridizing nucleotide sequence to the 5'-tagging portion. When a reporter-carrying fragment comprising the 5'-tagging portion and a 5'-end part of the 3'-targeting portion is released, it is preferable that the capturing portion of the CTO is designed to comprise a hybridizing nucleotide sequence to the 5'-tagging portion and the 5'-end part of the 3'-targeting portion. When a reporter-carrying fragment comprising a part of the 5'-tagging portion of the Q-PTO is released, it is preferable that the capturing portion of the CTO is designed to comprise a hybridizing nucleotide sequence to the part of the 5'-tagging portion.

In addition, the capturing portion of the CTO may be designed by predicting the cleavage site of the Q-PTO. For example, when the capturing portion of the CTO is designed to comprise a hybridizing nucleotide sequence to the 5'-tagging portion, a reporter-carrying fragment comprising a part of the 5'-tagging portion or a reporter-carrying fragment comprising the 5'-tagging portion may be hybridized with the capturing portion of the CTO and then extended.

In one embodiment, when a reporter-carrying fragment comprising the 5'-tagging portion and a 5'-end part of the 3'-targeting portion is released, a 5'-end part of the capturing portion of the CTO may be designed to comprise a nucleotide sequence complementary to the 5'-end part of the cleaved 3'-targeting portion. In particular, the nucleotide sequence of the 5'-end part of the capturing portion of the CTO that is complementary to the 5'-end part of the cleaved 3'-targeting portion may be selected based on the predicted cleavage site on the 3'-targeting portion of the Q-PTO. The nucleotide sequence of the 5'-end part of the capturing portion of the CTO that is complementary to the 5'-end part of the cleaved 3'-targeting portion may be 1 to 10 nucleotides, 1 to 5 nucleotides, or 1 to 3 nucleotides.

In one embodiment, the 3'-end of the CTO may comprise additional nucleotides that are not involved in hybridization with the reporter-carrying fragment. In addition, as long as the capturing portion of the CTO is stably hybridized with the reporter-carrying fragment, the capturing portion of the CTO may comprise a hybridizing nucleotide sequence to only a part of the reporter-carrying fragment (e.g., a part of the reporter-carrying fragment comprising the 3'-end portion of the reporter-carrying fragment).

The term "a capturing portion comprising a hybridizing nucleotide sequence to the 5'-tagging portion or a part thereof" as used herein is intended to encompass various designs and compositions of the capturing portion of the CTO as described above.

In one embodiment, the CTO may be designed to have a hairpin structure or not to have a hairpin structure.

The length of the CTO may vary widely. For example, the CTO may be 5-1000 nucleotides, 5-500 nucleotides, 5-300 nucleotides, 5-100 nucleotides, 5-80 nucleotides, 5-60 nucleotides, 5-40 nucleotides, 7-1000 nucleotides, 7-500 nucleotides, 7-300 nucleotides, 7-100 nucleotides, 7-80 nucleotides, 7-60 nucleotides, 7-40 nucleotides, 15-1000 nucleotides, 15-500 nucleotides, 15-300 nucleotides, 15-100 nucleotides, 15-80 nucleotides, 15-60 nucleotides, 15-40 nucleotides, 20-1000 nucleotides, 20-500 nucleotides, 20-300 nucleotides, 20-100 nucleotides, 20-80 nucleotides, 20-60 nucleotides, 20-40 nucleotides, 30-1000 nucleotides, 30-500 nucleotides, 30-300 nucleotides, 30-100 nucleotides, 30-80 nucleotides, 30-60 nucleotides, or 30-40 nucleotides in length.

The capturing portion of the CTO may have any length, as long as it is specifically hybridized with the reporter-carrying fragment. For example, the capturing portion of the CTO may be 5-100 nucleotides, 5-60 nucleotides, 5-40 nucleotides, 5-30 nucleotides, 5-20 nucleotides, 10-100 nucleotides, 10-60 nucleotides, 10-40 nucleotides, 10-30 nucleotides, 10-20 nucleotides, 15-100 nucleotides, 15-60 nucleotides, 15-40 nucleotides, 15-30 nucleotides, or 15-20 nucleotides in length.

The templating portion of the CTO may have any length, as long as it can function as a template for extension of the reporter-carrying fragment. For example, the templating portion of the CTO may be 1-900 nucleotides, 1-400 nucleotides, 1-300 nucleotides, 1-100 nucleotides, 1-80 nucleotides, 1-60 nucleotides, 1-40 nucleotides, 1-20 nucleotides, 2-900 nucleotides, 2-400 nucleotides, 2-300 nucleotides, 2-100 nucleotides, 2-80 nucleotides, 2-60 nucleotides, 2-40 nucleotides, 2-20 nucleotides, 5-900 nucleotides, 5-400 nucleotides, 5-300 nucleotides, 5-100 nucleotides, 5-80 nucleotides, 5-60 nucleotides, 5-40 nucleotides, 5-30 nucleotides, 10-900 nucleotides, 10-400 nucleotides, 10-300 nucleotides, 15-900 nucleotides, 15-100 nucleotides, 15-80 nucleotides, 15-60 nucleotides, 15-40 nucleotides, or 15-20 nucleotides in length.

In one embodiment, the 3'-end of the CTO may have a 3'-OH terminus. Alternatively, the 3'-end of the CTO may be blocked to prevent extension. The blocking may be achieved by conventional methods, and details thereof are described with reference to the above-described methods for blocking the Q-PTO.

The reporter-carrying fragment is hybridized with the CTO, providing a conformation suitable for extension of the reporter-carrying fragment. Although uncleaved Q-PTO may also hybridize with the capturing portion of the CTO through its 5'-tagging portion, its 3'-targeting portion is not hybridized with the CTO, thereby preventing the generation of an extended duplex.

The hybridization in step (c) may be described in detail with reference to the description provided in step (a).

### Step (d): extension of the reporter-carrying fragment and generation of the extended duplex

An extension reaction is performed using the resultant of step (c) and the DNA polymerase having 5' nuclease activity. The reporter-carrying fragment hybridized with the capturing portion of the CTO is extended to generate an extended strand comprising an extended sequence complementary to the templating portion of the CTO (see FIG. 1(d)). This forms an extended duplex between the extended strand and the CTO. In contrast, uncleaved Q-PTO hybridized with the capturing portion of the CTO is not extended, and thus, an extended duplex is not generated.

In step (d), the reporter-carrying fragment hybridized with the capturing portion of the CTO is extended by the DNA polymerase having 5' nuclease activity along the templating portion of the CTO as a template.

In step (d), the terms "extended sequence," "extended strand," and "extended duplex" used in connection with the extension reaction of the reporter-carrying fragment have the following meanings:
As used herein, the term "extended sequence" refers to a sequence newly generated by extension from the reporter-carrying fragment in step (d). In other words, the extended sequence refers to a portion of the extended strand as described below, excluding the reporter-carrying fragment (see FIG. 1(d)).
As used herein, the term "extended strand" refers to a sequence encompassing the reporter-carrying fragment and the extended sequence. In other words, the extended strand refers to a portion of the extended duplex as described below, excluding the CTO (see FIG. 1(d)).
As used herein, the term "extended duplex" refers to a duplex generated by an extension reaction in which the reporter-carrying fragment hybridized with the capturing portion of the CTO is extended by a DNA polymerase having 5' nuclease activity using the templating portion of the CTO as a template. In other words, the extended duplex refers to a duplex between the CTO and an extended strand comprising the reporter-carrying fragment and the extended sequence (see FIG. 1(d)).

The Tm value of the extended duplex is adjustable by (i) a sequence and/or length of the reporter-carrying fragment, (ii) a sequence and/or length of the CTO, or (iii) the sequence and/or length of the reporter-carrying fragment and the sequence and/or length of the CTO.

As used herein, the term "Tm" refers to the melting temperature at which half of a population of double-stranded nucleic acid molecules dissociates into single-stranded molecules. The Tm value is determined by the length and G/C content of nucleotides hybridized. The Tm value may be calculated by conventional methods such as the Wallace rule (R.B. Wallace, et al., Nucleic Acids Research, 6:3543-3547 (1979)) and the nearest-neighbor method (SantaLucia J. Jr., et al., Biochemistry, 35:3555-3562 (1996); Sugimoto N., et al., Nucleic Acids Res., 24:4501-4505 (1996)).

In one embodiment, the Tm value refers to an actual Tm value under the conditions in which the reaction is actually performed.

### Step (e): detection of the presence of the extended strand

Finally, the presence of the extended strand is detected. The presence of the extended strand indicates the presence of the target nucleic acid.

Step (e) may be achieved by detecting a signal indicating the presence of the extended strand (see FIG. 1(e)).

As used herein, the term "signal" refers to any signal that can indicate the presence or absence of the extended strand. For example, the signal includes a signal from a label (signal generation or disappearance), a change in the signal from a label (signal increase or decrease), a melting curve, a melting pattern, and a melting temperature (or Tm value).

In one embodiment, the presence of the extended strand in step (e) is detected by measuring a signal provided from the extended duplex. Specifically, the signal provided from the extended duplex is a signal generated from dissociation of the extended duplex.

When the extended duplex generated in step (d) dependent on the presence of the target nucleic acid remains in a double-stranded form, the second quencher molecule of the CTO quenches the signal from the reporter molecule of the extended strand. When the extended duplex dissociates into two single strands, the second quencher molecule unquenches the reporter molecule, thereby providing a signal indicating the presence of the extended strand. On the other hand, when the target nucleic acid is absent, the extended duplex is not generated, and the reporter molecule of the Q-PTO is quenched by the first quencher molecule.

The detection of the presence of the extended strand in step (e) may be performed by (i) measuring a signal at a predetermined temperature, or (ii) measuring a signal by melting analysis or a melting followed by hybridization analysis.

### (i) Measuring a signal at a predetermined temperature

The detection in step (e) is performed by measuring a signal indicating the presence of the extended strand at a predetermined temperature.

In one embodiment, the presence of the extended strand in step (e) is detected by measuring a signal at a temperature within a temperature range in which some or all of the extended duplexes are present in a dissociated form. Accordingly, the temperature range for measuring a signal indicating the presence of the extended strand can be adjusted by controlling the Tm value of the extended duplex.

As used herein, the term "some" when referring to the form of a duplex within a certain temperature range (or at a certain temperature) refers to a portion of the total amount of the duplex within the certain temperature range, for example, 5% or more, 10% or more, 20% or more, 30% or more, 40% or more, 50% or more, 60% or more, 70% or more, or 80% or more of the total amount.

As used herein, the term "all" when referring to the form of a duplex within a certain temperature range (or at a certain temperature) refers to most of the total amount of the duplex within the certain temperature range, for example, 85% or more, 90% or more, 95% or more, 96% or more, 97% or more, 98% or more, or 99% or more of the total amount, and has a meaning encompassing substantially all of the duplexes.

The extended duplex has a Tm value that is adjustable by (i) a sequence and/or length of the reporter-carrying fragment, (ii) a sequence and/or length of the CTO, or (iii) the sequence and/or length of the reporter-carrying fragment and the sequence and/or length of the CTO.

According to one embodiment, the presence of the extended strand may be detected at a temperature at which the extended duplex generated in step (d) is dissociated.

### (ii) measuring a signal by melting analysis or a melting followed by hybridization analysis.

According to one embodiment, the detection in step (e) is performed by measuring a signal indicating the presence of the extended strand by melting analysis or a melting followed by hybridization analysis.

According to one embodiment, the presence of the extended strand in step (e) is detected by melting or hybridizing the extended duplex within a certain temperature range and measuring a signal from the extended duplex. Specifically, the method further comprises a step of melting the extended duplex or hybridizing it after melting following step (d) to provide a detectable signal, and the presence of the extended strand is detected by detecting the signal.

According to one embodiment, the detection of the presence of the extended strand in step (e) is performed by melting analysis, in which the extended duplex is melted to provide a signal indicating the presence of the extended strand.

As used herein, the term "a melting followed by hybridization analysis" refers to a method in which the duplex is melted and then hybridized to provide a signal indicating the presence of the duplex.

According to one embodiment, the detection of the presence of the extended strand in step (e) is performed through a melting followed by hybridization analysis. Specifically, the detection of the presence of the extended strand in step (e) is carried out by melting the extended duplex and hybridizing the resulting product within a certain temperature range to provide a signal indicating the presence of the extended strand.

The melting curve or hybridization curve may be obtained by conventional technologies, for example, as described in U.S. Pat. Nos. 6,174,670 and 5,789,167, Drobyshev et al, Gene 188: 45(1997); Kochinsky and Mirzabekov Human Mutation 19:343(2002); Livehits et al J. Biomol. Structure Dynam. 11:783(1994); and Howell et al Nature Biotechnology 17:87(1999). For example, a melting curve or hybridization curve may consist of a graphic plot or display of the variation of the output signal with the parameter of hybridization stringency. Output signal may be plotted directly against the hybridization parameter. Typically, a melting curve or hybridization curve will have the output signal, for example fluorescence, which indicates the degree of duplex structure (i.e. the extent of hybridization), on the Y-axis and the hybridization parameter on the X axis.

A plot of the first derivative of fluorescence versus temperature, i.e., a plot of the rate of change in fluorescence vs. temperature (dF/dT vs. T or -dF/dT vs. T) provides a melting peak.

When the present disclosure utilizes melting analysis or a melting followed by hybridization analysis, it enables multiplex detection of at least two target nucleic acids. Specifically, when the present disclosure is performed for simultaneous detection of at least two target nucleic acids by melting curve analysis, and the extended duplexes corresponding to the at least two target nucleic acids have different Tm values, at least two target nucleic acids can be detected using a single label (e.g., FAM).

In one embodiment, the method is performed to detect at least two target nucleic acids, specifically, at least three, and more specifically, at least five target nucleic acids.

In one embodiment, the method is performed to detect at least two target nucleic acids (specifically, at least three, and more specifically, at least five target nucleic acids); the primer comprises at least two primers (specifically, at least three, and more specifically, at least five primers); the Q-PTO comprises at least two Q-PTOs (specifically, at least three, and more specifically, at least five Q-PTOs); and the CTO comprises at least one CTO (specifically, at least two, more specifically, at least three, and even more specifically, at least five CTOs). When at least two target nucleic acids are present, the method provides at least two signals corresponding to the at least two target nucleic acids.

The 5'-tagging portions of at least two Q-PTOs may have the same sequence. For example, when the present disclosure is performed for screening of target nucleic acids, the 5'-tagging portions of the Q-PTOs may have the same sequence.

Furthermore, a single type of CTO may be used for detection of multiple target nucleic acids. For example, when Q-PTOs having the same sequence in their 5'-tagging portions are used for screening of target nucleic acids, a single type of CTO may be used. As such, since the CTO used in the method of the present disclosure can be premanufactured regardless of the type of target nucleic acid, the use of the CTO as a universal oligonucleotide will enable simple and cost-effective target detection.

In one embodiment, the extended duplexes corresponding to at least two target nucleic acids have different Tm values.

In one embodiment, at least two signals corresponding to at least two target nucleic acids are provided from the same type of label.

In a specific embodiment, at least two signals corresponding to at least two target nucleic acids are provided from the same type of label, and the extended duplexes corresponding to the at least two target nucleic acids have different Tm values.

In one embodiment, under primer extension-dependent cleavage induction, when Q-PTOs having the same 5'-tagging portion are used to screen multiple target nucleic acid sequences, the 5'-end part of the 3'-targeting portion may generate different reporter-carrying fragments. In such cases, as described above, universal bases and degenerate sequences are usefully employed in the CTO. The strategy of using universal bases and degenerate sequences in the CTO allows for the use of a single type or a minimal number of types of CTOs for screening multiple target nucleic acid sequences.

According to one embodiment, the method of the present disclosure further comprises repeating all or a portion of the steps (a)-(e) with denaturation between repeating cycles. Such repetition amplifies the target nucleic acid and/or the signal indicating the presence of the target nucleic acid.

The term "denaturation" in the phrase "with denaturation between repeating cycles" refers to the separation of a double-stranded nucleic acid molecule into single-stranded nucleic acid molecules.

In one embodiment, the repeating step including denaturation may include at least a step of denaturing the extended duplex. Other components (e.g., primers and enzymes) may be used in sufficient amounts so as not to become limiting factors.

The denaturation may be carried out by conventional technologies, including, but not limited to, heating, alkali, formamide, urea and glycoxal treatment, enzymatic methods (e.g., helicase action), and binding proteins. For instance, the denaturation can be achieved by heating at a temperature ranging from 80°C to 105°C. General methods for accomplishing this treatment are provided by Joseph Sambrook, et al., Molecular Cloning, A Laboratory Manual, Cold Spring Harbor Laboratory Press, Cold Spring Harbor, N.Y. (2001).

The detection of step (e) may be performed in a real-time manner, an end-point manner, or a predetermined time interval manner. When the present disclosure further comprises repeating the steps (a)-(e), signal detection is performed for each cycle of the repetition at a predetermined temperature (i.e., real-time manner), at the end of the repetition at a predetermined temperature (i.e., end-point manner), or at each of predetermined time intervals during the repetition at a predetermined temperature. Specifically, the detection may be performed for each cycle of the repetition in a real-time manner to improve detection efficiency and quantification.

In the repetition, the method of the present disclosure is performed in the presence of an additional primer that pairs with the primer to amplify the target nucleic acid, and is particularly performed by a PCR method.

In one embodiment, the steps (a)-(e) are performed in a single reaction vessel, or a portion of the steps (a)-(e) is performed in a separate reaction vessel.

The present disclosure does not require that the target nucleic acid to be detected and/or amplified have any particular sequence or length, including all DNA (gDNA and cDNA) and RNA molecules.

When mRNA is used as a starting material, a reverse transcription step is essential prior to the annealing step, and details thereof are disclosed in the literature [Joseph Sambrook, et al., Molecular Cloning, A Laboratory Manual, Cold Spring Harbor Laboratory Press, Cold Spring Harbor, N.Y. (2001); and Noonan, K. F. et al., Nucleic Acids Res. 16:10366 (1988)]. For the reverse transcription reaction, a random hexamer or an oligonucleotide dT primer hybridizable to mRNA may be used.

The present disclosure is also useful in detection of a nucleotide variation. Particularly, the target nucleic acid comprises a nucleotide variation. The term "nucleotide variation" as used herein refers to any single or multiple nucleotide substitutions, deletions, or insertions in a DNA sequence at a particular location among contiguous DNA segments. Such contiguous DNA segments include a gene or any other portion of a chromosome. These nucleotide variations may be mutant or polymorphic allele variations. For example, the nucleotide variation detected in the present disclosure includes SNP (single nucleotide polymorphism), mutation, deletion, insertion, substitution, and translocation. Exemplified nucleotide variation includes numerous variations in a human genome (e.g., variations in the MTHFR (methylenetetrahydrofolate reductase) gene), variations involved in drug resistance of pathogens and tumorigenesis-causing variations. The term "nucleotide variation" as used herein includes any variation at a particular location in a nucleic acid sequence. In other words, the term "nucleotide variation" includes a wild type and any mutant type at a particular location in a nucleic acid sequence.

In the present disclosure for detecting a nucleotide variation of a target nucleic acid, when the primer or Q-PTO used has a sequence complementary to the nucleotide variation of the target nucleic acid, the target nucleic acid comprising the nucleotide variation is referred to herein as a "matching template." When the primer or Q-PTO used has a non-complementary sequence to the nucleotide variation of the target nucleic acid, the target nucleic acid comprising the nucleotide variation is referred to herein as a "mismatching template."

For detection of a nucleotide variation, the 3'-end of a primer may be designed to be positioned opposite the site of the nucleotide variation in the target nucleic acid. In a specific embodiment, the 3'-end of the primer has a sequence complementary to the nucleotide variation of the target nucleic acid. The 3'-end of the primer having a sequence complementary to the nucleotide variation of the target nucleic acid anneals to the matching template and is extended to induce cleavage of the Q-PTO. The resulting reporter-carrying fragment is hybridized with the CTO and is extended to generate an extended duplex, thereby providing a signal indicating the presence of the extended strand. In contrast, when the 3'-end of the primer mismatches the nucleotide variation in the mismatching template, even if the primer is hybridized with the mismatching template, the 3'-end of the primer is not extended under conditions in which annealing at the 3'-end is essential for extension, and thus no signal indicating the presence of the extended strand (i.e., no signal indicating the presence of the target nucleic acid) is generated.

Alternatively, Q-PTO cleavage dependent on hybridization of the Q-PTO having a sequence complementary to the nucleotide variation of the target nucleic acid may be used. For example, under controlled conditions, a Q-PTO having a sequence complementary to the nucleotide variation of the target nucleic acid is hybridized with the matching template and is then cleaved. The resulting reporter-carrying fragment is hybridized with the CTO and is extended to generate an extended duplex, thereby providing a signal indicating the presence of the extended strand. In contrast, under controlled conditions, the Q-PTO is not hybridized with the mismatching template having a non-complementary sequence at the nucleotide variation site, and is not cleaved. In particular, in such cases, the sequence complementary to the nucleotide variation is located in the middle of the 3'-targeting portion of the Q-PTO.

Alternatively, for detection of a nucleotide variation, it is preferable that a 5'-end part of the 3'-targeting portion of the Q-PTO is located at the nucleotide variation of the target nucleic acid, and the 5'-end part of the 3'-targeting portion of the Q-PTO has a sequence complementary to the nucleotide variation of the target nucleic acid.

In an embodiment for detecting a single nucleotide variation, the 5'-end part of the 3'-targeting portion of the Q-PTO has a sequence complementary to the single nucleotide variation of the target nucleic acid. As described above, cleavage of the Q-PTO hybridized with the matching template may be induced at a site immediately adjacent in the 3'-direction to the 5'-end part of the 3'-targeting portion of the Q-PTO, for example, under primer extension-dependent cleavage induction. The 3'-end of the reporter-carrying fragment has a nucleotide complementary to the single nucleotide variation. The reporter-carrying fragment is hybridized with the CTO having a capturing portion that includes a sequence corresponding to the nucleotide variation, and is extended to form an extended duplex, thereby providing a signal indicating the presence of the target nucleic acid (i.e., the presence of the extended strand).

When the same Q-PTO is hybridized with a mismatching template having the same sequence as the matching template except for a single nucleotide variation, cleavage of the Q-PTO may occur at a site spaced two nucleotides in the 3'-direction from the 5'-end part of the 3'-targeting portion of the Q-PTO. The 3'-end of the reporter-carrying fragment has, in addition to a nucleotide complementary to the single nucleotide variation, an additionally cleaved nucleotide. When the region of the CTO hybridized with the additionally cleaved nucleotide is designed to have a non-complementary sequence to the additionally cleaved nucleotide, the 3'-end of the reporter-carrying fragment is not hybridized with the CTO and is not extended under controlled conditions.

According to a specific embodiment, the cleavage site of a Q-PTO having a sequence complementary to the nucleotide variation at a 5'-end part of the 3'-targeting portion varies depending on hybridization with the matching template or the mismatching template. Accordingly, the reporter-carrying fragment released from either one of the hybridization events has a different sequence, particularly in its 3'-end part, and more particularly at its 3'-end.

According to a specific embodiment, selection of the nucleotide sequence of the CTO in consideration of the difference in the 3'-end part of the reporter-carrying fragment allows discrimination between the matching template and the mismatching template.

According to a specific embodiment, the target nucleic acid used in the present disclosure is a pre-amplified nucleic acid sequence. The use of a pre-amplified nucleic acid sequence significantly increases the sensitivity and specificity of target detection in the present disclosure.

### II. Composition for Detecting a Target Nucleic Acid

In another aspect, the present disclosure provides a composition for detecting a target nucleic acid in a sample, comprising:
(i) a primer;
   wherein the primer comprises a hybridizing nucleotide sequence to a first region of the target nucleic acid,
(ii) a Quenching-Probing and Tagging Oligonucleotide (Q-PTO);
   wherein the Q-PTO comprises in a 5' to 3' direction: (i) a 5'-tagging portion comprising a non-hybridizing nucleotide sequence to the target nucleic acid, and (ii) a hybridizing nucleotide sequence to a second region of the target nucleic acid,
   wherein the 5'-tagging portion of the Q-PTO is not hybridized with the target nucleic acid and the 3'-targeting portion of the Q-PTO is hybridized with the target nucleic acid,
   wherein the primer is extended to induce cleavage of the Q-PTO by a DNA polymerase having 5' nuclease activity such that the cleavage releases the reporter-carrying fragment;
   wherein the Q-PTO has a first quencher molecule in the 3'-targeting portion and a reporter molecule located at position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in the reporter-carrying fragment,
(iii) a Capturing and Templating Oligonucleotide (CTO);
   wherein the CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment,
   wherein the capturing portion of the CTO is hybridized with the reporter-carrying fragment,
   wherein the CTO has a second quencher molecule located at positions such that upon hybridization of the capturing portion of the CTO with the reporter-carrying fragment, the second quencher molecule is in close proximity to the reporter molecule, thereby causing the second quencher molecule to quench a signal from the reporter molecule,
   wherein the reporter-carrying fragment hybridized with the capturing portion of the CTO is extended to generate an extended strand comprising an extended sequence complementary to the templating portion of the CTO, thereby forming an extended duplex between the extended strand and the CTO.

Since the second aspect of the present disclosure, "composition for detecting a target nucleic acid" (hereinafter referred to as "Q-PTOCE composition"), is configured to implement the "method for detecting a target nucleic acid by Q-PTOCE assay" described in the first aspect above, common description between the two aspects is omitted herein to avoid undue complexity of the present specification.

In one embodiment, the reporter molecule and the first quencher molecule of the Q-PTO are in close proximity to each other, allowing the first quencher molecule to intramolecularly quench the signal from the reporter molecule.

In one embodiment, on the Q-PTO, the first quencher molecule may be positioned immediately adjacent to the reporter molecule.

In another embodiment, on the Q-PTO, the first quencher molecule may be located near the reporter molecule. For example, the first quencher molecule may be located 1 to 30 nucleotides apart from the reporter molecule.

In one embodiment, the reporter molecule and the first quencher molecule of the Q-PTO are located at positions such that they are separated from each other by the cleavage reaction of the Q-PTO. Specifically, the reporter molecule is included in a reporter-carrying fragment released by the cleavage reaction, and the first quencher molecule is included in a remaining portion of the Q-PTO other than the released reporter-carrying fragment. As a result, the first quencher molecule and the reporter molecule are spaced apart from each other, allowing the first quencher molecule to unquench the signal from the reporter molecule.

In one embodiment, the second quencher molecule of the CTO is of the same type as the first quencher molecule of the Q-PTO.

In one embodiment, the Tm value of the extended duplex is adjustable by (i) a sequence and/or length of the reporter-carrying fragment, (ii) a sequence and/or length of the CTO or (iii) the sequence and/or length of the reporter-carrying fragment and the sequence and/or length of the CTO.

In one embodiment, the Q-PTO and/or the CTO are blocked at its 3'-end to prohibit its extension.

In one embodiment, the Q-PTOCE composition further comprises a DNA polymerase having 5' nuclease activity.

In one embodiment, the Q-PTOCE composition further comprises an additional primer that pairs with the primer to amplify the target nucleic acid.

In one embodiment, the Q-PTOCE composition provides a signal dependent on the presence of the target nucleic acid. Specifically, the Q-PTOCE composition according to the present disclosure provides a change in signal as the target nucleic acid is amplified.

In one embodiment, the signal dependent on the presence of the target nucleic acid is a signal provided from the extended duplex.

In one embodiment, the reaction with the target nucleic acid may include an amplification reaction, for example, a signal amplification reaction and/or a nucleic acid amplification reaction.

In one embodiment, the Q-PTOCE composition has a signal-changing temperature range (SChTR), in which a signal changes depending on the presence of the target nucleic acid, and a signal-constant temperature range (SCoTR), in which the signal is constant even in the presence of the target nucleic acid. Specifically, the Q-PTOCE composition is an OverSC (Over-Signal-Change) composition having a characteristic that the signal-changing temperature range is higher than the signal-constant temperature range.

As used herein, the term "signal-constant temperature range (SCoTR)" refers to a temperature range in which a composition for detecting a target nucleic acid provides a constant signal despite the presence of the target nucleic acid. The signal-constant temperature range is a temperature range in which the composition for detecting a target nucleic acid provides a constant signal over reaction time, which may also be referred to herein as a temperature range in which the composition for detecting a target nucleic acid does not provide a significant signal, or a temperature range in which the composition for detecting a target nucleic acid does not provide a signal indicative of the presence of a target nucleic acid.

The term "constant signal" as used herein refers to no substantial change in signal during a reaction between a target nucleic acid and a composition for detecting the target nucleic acid (e.g., during a target nucleic acid amplification reaction). That is, the term refers to all or any signal pattern other than significant signal changes brought about by the amplification of the target nucleic acid present. In particular, the constant signal means no signal change. For example, if the signal during an amplification reaction does not exceed the background signal intensity or the intensity of a signal in the absence of the target nucleic acid, it may be expressed as "the signal is constant". In the present disclosure, the constant signal may be used interchangeably with the signal that does not change, or the signal that does not show change.

As used herein, the term "signal-changing temperature range (SChTR)" refers to a temperature range in which a composition for detecting a target nucleic acid provides a signal that changes dependent on the presence of a target nucleic acid. The signal-changing temperature range is a temperature range in which the composition for detecting a target nucleic acid provides a signal that changes over reaction time, which may also be referred to herein as a temperature range in which the composition for detecting a target nucleic acid provides a significant signal, or a temperature range in which the composition for detecting a target nucleic acid provides a signal indicative of the presence of the target nucleic acid.

In the present disclosure, a "signal change" means a significant signal change that is, a significant change in signal where the signal change indicates the presence of the target nucleic acid. For example, a significant signal change, that is, a signal change that indicates the presence of the target nucleic acid may refer to the generation or extinguishment of a signal that has a distinct intensity compared to the background signal intensity or the intensity of a signal in the absence of the target nucleic acid, or may refer to a substantial increase or decrease of the intensity of a signal indicating the presence of the target nucleic acid, as the target nucleic acid and/or signal is amplified.

International Patent Application Publication WO 2022-265463 discloses that various signal generation mechanisms known in the art for detecting a target nucleic acid have a signal-changing temperature range (SChTR) in which the signal changes depending on the presence of the target nucleic acid, and one or two signal-constant temperature ranges (SCoTRs) in which the signal is constant even in the presence of the target nucleic acid. In addition, the literature discloses that based on the number and order of the signal-changing temperature range and the signal-constant temperature range, various conventional signal generation mechanisms can be categorized into any one of the following: (i) an Under-Signal-Change (UnderSC) signal generation mechanism having a characteristic that the signal-changing temperature range is lower than the signal-constant temperature range, (ii) an Inter-Signal-Change (InterSC) signal generation mechanism having a characteristic that the signal-changing temperature range is higher than one of two signal-constant temperature ranges, and lower than the other of the two signal-constant temperature ranges, and (iii) an Over-Signal-Change (OverSC) signal generation mechanism having a characteristic that the signal-changing temperature range is higher than the signal-constant temperature range.

With respect to the terminology disclosed in the above-mentioned reference, the method for detecting a target nucleic acid according to the present disclosure is classified as an Over-Signal-Change (OverSC) signal generation mechanism among the three types of signal generation mechanisms described above, and the composition for detecting a target nucleic acid according to the present disclosure can be used as an OverSC composition.

In one embodiment, the Q-PTOCE composition is an Over-Signal-Change (OverSC) composition having a characteristic that the signal-changing temperature range is higher than the signal-constant temperature range. In this regard, a more detailed description is provided below with reference to the drawings.

FIGS. 2 and 3 show the predominant conformations of oligonucleotides depending on the presence of the target nucleic acid and temperature. In particular, in FIG. 2, the Q-PTO has a reporter molecule and a first quencher molecule located three-dimensionally in close proximity to each other due to its conformational structure (e.g., random coil). In FIG. 3, the Q-PTO has a reporter molecule and a first quencher molecule positioned sufficiently close to each other to allow interaction.

FIG. 2(a) and FIG. 3(a) show the conformations of the Q-PTO and the CTO within (i) a signal-constant temperature range and (ii) a signal-changing temperature range, when the target nucleic acid is absent or before the Q-PTOCE composition reacts with the target nucleic acid. When the target nucleic acid is absent or before the reaction with the target nucleic acid, cleavage of the Q-PTO does not occur and no extended duplex is generated. Accordingly, as shown in FIG. 2(a) and FIG. 3(a), within the signal-constant temperature range, the 5'-tagging portion of the Q-PTO and the 3'-capturing portion of the CTO are hybridized, and the reporter molecule of the Q-PTO is quenched by the first quencher molecule and/or the second quencher molecule. Within the signal-changing temperature range, the Q-PTO and the CTO dissociate from each other and exist as single strands, but the reporter molecule of the Q-PTO remains quenched by the first quencher molecule. That is, when the target nucleic acid is absent or before the reaction with the target nucleic acid, the reporter molecule of the Q-PTO is quenched by the first quencher molecule and/or the second quencher molecule across the entire temperature range.

Meanwhile, when the target nucleic acid is present, the Q-PTO is cleaved dependent on the presence of the target nucleic acid to generate an extended duplex. By such cleavage, the reporter molecule and the first quencher molecule of the Q-PTO are separated from each other, and the first quencher molecule unquenches the signal from the reporter molecule. FIG. 2(b) and FIG. 3(b) show the conformations of the extended duplex generated dependent on the presence of the target nucleic acid at different temperatures. As shown in FIG. 2(b) and FIG. 3(b), the extended duplex exists in a double-stranded form within the signal-constant temperature range, and the second quencher molecule of the CTO is in close proximity to the reporter molecule of the extended strand to quench the signal from the reporter molecule. Within the signal-changing temperature range, the extended duplex dissociates and the reporter molecule of the extended strand is separated from the second quencher molecule of the CTO. As a result, the reporter molecule is unquenched from both the first quencher molecule and the second quencher molecule, thereby providing a signal indicating the presence of the extended strand.

That is, within the signal-constant temperature range, the reporter molecule is quenched by the first quencher molecule and/or the second quencher molecule regardless of the presence of the target nucleic acid. Accordingly, within the signal-constant temperature range, the signal remains constant without any signal change even in the presence of the target nucleic acid. Meanwhile, within the signal-changing temperature range, when the target nucleic acid is absent, the first quencher molecule quenches the signal from the reporter molecule, whereas when the target nucleic acid is present, both the first quencher molecule and the second quencher molecule unquench the signal from the reporter molecule. As a result, within the signal-changing temperature range, the signal changes dependent on the presence of the target nucleic acid (see FIGS. 2 and 3).

In one embodiment, the extended duplex forms a double strand at temperatures within the signal-constant temperature range in the presence of the target nucleic acid.

In one embodiment, the extended duplex dissociates at temperatures within the signal-changing temperature range in the presence of the target nucleic acid.

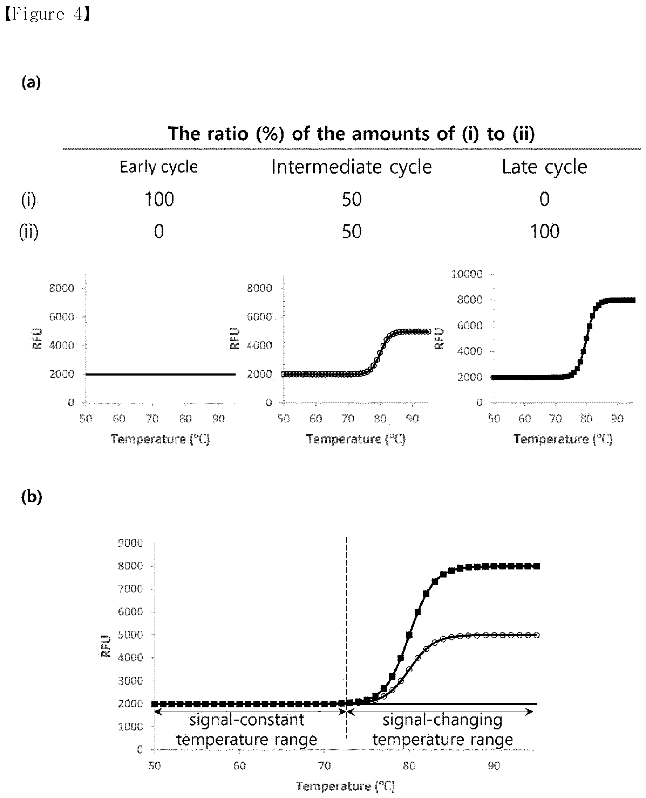

FIG. 4 shows the amount ratio and melt curve of the reaction product obtained from the reaction of the Q-PTOCE composition with the target nucleic acid. FIG. 4(a) shows (i) the amount ratio (or abundance ratio) of the Q-PTO and the CTO and (ii) the amount ratio (or abundance ratio) of the extended duplex, as well as their melt curves, in the early, intermediate, and late cycles when all or a portion of steps (a)-(d) of the Q-PTOCE assay are repeated (e.g., target nucleic acid amplification). FIG. 4(b) presents merged melt curves in each cycle in FIG. 4(a).

Specifically, in FIG. 4(a), the numbers in row (i) represent the total amount ratio of uncleaved Q-PTO and CTO existing in the form shown in FIG. 2(a) or FIG. 3(a), and the numbers in row (ii) represent the total amount ratio of the extended strand of the reporter-carrying fragment and the CTO (i.e., the extended duplex) existing in the form shown in FIG. 2(b) or FIG. 3(b). The amount ratios in rows (i) and (ii) change as the cycle increases, that is, as the target nucleic acid is amplified. In particular, when the melting curves for each of the early, intermediate, and late cycles are merged, a graph as shown in FIG. 4(b) can be obtained. Through this graph, it can be confirmed that the Q-PTOCE composition according to the present disclosure exhibits a signal-constant temperature range in which the signal remains constant even in the presence of the target nucleic acid, and a signal-changing temperature range in which the signal changes as the target nucleic acid is amplified. In addition, the signal-changing temperature range is higher than the signal-constant temperature range. That is, the Q-PTOCE composition according to the present disclosure can be used as the above-described OverSC composition.

In one embodiment, the signal-changing temperature range depends on the Tm value of the extended duplex.

The Q-PTOCE composition according to the present disclosure may optionally comprise reagents necessary for performing a target nucleic acid amplification reaction (e.g., PCR reaction), such as a buffer, a DNA polymerase cofactor, and deoxyribonucleotide-5-triphosphates. Optionally, the composition may further comprise various polynucleotide molecules, reverse transcriptases, various buffers and reagents, and antibodies that inhibit DNA polymerase activity. In addition, the composition may comprise reagents necessary for performing positive and negative control reactions. The optimal amounts of reagents used in specific reactions can be readily determined by those skilled in the art who are aware of the advantages of the present disclosure. The components of the composition may be present in individual containers, or two or more components may be present in a single container.

### III. Method for Detecting Multiple Target Nucleic Acids Using Q-PTOCE Assay

In another aspect, the present disclosure provides a method for detecting *n* target nucleic acids in a sample, comprising:
(a) detecting signals at *n* detection temperatures, while incubating with *n* compositions for detecting the *n* target nucleic acids, a sample suspected of containing at least one of the *n* target nucleic acids in a reaction vessel;
   wherein *n* is an integer of 2 or more,
   wherein the incubating comprises a plurality of reaction cycles and the detection of signals is carried out at at least one of the plurality of reaction cycles,
   wherein each of the *n* compositions for detecting the *n* target nucleic acids provides a signal change at a corresponding detection temperature among the *n* detection temperatures in the presence of a corresponding target nucleic acid, the signal change indicating the presence of a corresponding target nucleic acid,
   wherein a composition for detecting an *i*^{th} target nucleic acid among the *n* compositions for detecting the *n* target nucleic acids provides a signal change at an *i*^{th} detection temperature among the *n* detection temperatures and provides a constant signal at the other detection temperatures in the presence of the *i*^{th} target nucleic acid,
   wherein *i* represents an integer from 1 to *n,* and the *i*^{th} detection temperature is lower than a (*i*+1)^{th} detection temperature,
   wherein in the temperature range covering all of the *n* detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid,
   wherein the composition for detecting the *i*^{th} target nucleic acid is any one of:
      (i) an Under-Signal-Change (UnderSC) composition having a characteristic that the signal-changing temperature range is lower than the signal-constant temperature range,
      (ii) an Inter-Signal-Change (InterSC) composition having a characteristic that the signal-changing temperature range is higher than one of two signal-constant temperature ranges, and lower than the other of the two signal-constant temperature ranges, and
      (iii) an Over-Signal-Change (OverSC) composition having a characteristic that the signal-changing temperature range is higher than the signal-constant temperature range, and
   wherein the composition for detecting the *n*^{th} target nucleic acid among the *n* compositions for detecting the *n* target nucleic acids is a composition comprising a primer, a Q-PTO and a CTO, and;
(b) determining the presence of the *n* target nucleic acids from the signals detected in step (a), wherein the presence of the *i*^{th} target nucleic acid is determined by the signal change detected at the *i*^{th} detection temperature.

The third aspect of the present disclosure, "a method for detecting *n* target nucleic acids in a sample," utilizes the first and second aspects of the present disclosure, and thus, common description between the two aspects is omitted herein to avoid undue complexity of the present specification.

The method for detecting *n* target nucleic acids according to the present disclosure enables detection of multiple target nucleic acids in a single reaction vessel by combining the Q-PTOCE composition as an OverSC composition with various UnderSC and InterSC compositions to which signal generation mechanisms known in the art for detecting target nucleic acids are applied, using a single type of label and a single type of detector.

Hereinafter, the present disclosure is described in further detail below.

### Step (a): Incubating and Detection of Signals

First, a sample suspected of containing at least one of the *n* target nucleic acids is mixed with *n* compositions for detecting target nucleic acids in a single reaction vessel and incubated.

In one embodiment, the *n* target nucleic acids may include a nucleotide variation. For example, one of the *n* target nucleic acids may include one type of nucleotide variation, and another may include a different type of nucleotide variation.

The *n* target nucleic acids herein may be genes from *n* different organisms, *n* different genes from the same organism, or combinations thereof.

The *n* target nucleic acids herein may be genes from *n* different organisms, *n* different genes from the same organism, or combinations thereof.

As used herein, the incubation reaction refers to any reaction that induces a signal change depending on the presence of a corresponding target nucleic acid at a corresponding detection temperature, as each of the target nucleic acids reacts with a corresponding composition for detecting a target nucleic acid.

Herein, the incubating comprises a plurality of cycles.

In one embodiment, the incubating in step (a) may include an amplification reaction, and may include, for example, a signal amplification reaction and/or a nucleic acid amplification reaction.

In one embodiment, the amplification reaction comprises a plurality of cycles.

In one embodiment, the incubating in step (a) is carried out under conditions that allow target amplification and a signal change by a composition for detecting a target nucleic acid. Such conditions include a temperature, salt concentration and pH of a solution.

In one embodiment, the incubating in step (a) is carried out at a signal amplification process with no nucleic acid amplification.

In one embodiment, the signal may be amplified simultaneously with target amplification. Alternatively, the signal may be amplified without target amplification.

In one embodiment, the signal change takes place during a process involving signal amplification and target amplification.

In one embodiment, the amplification of the target nucleic acid may be carried out by a polymerase chain reaction (PCR). The PCR is widely used in the art to amplify target nucleic acids and includes cycles of denaturation of target nucleic acids, annealing (hybridization) between target nucleic acids and primers, and extension of primers (Mullis et al., U.S. Pat. Nos. 4,683,195, 4,683,202, and 4,800,159; Saiki et al., (1985) Science 230, 1350-1354).

In one embodiment, the amplification of target nucleic acids may be carried out by ligase chain reaction (LCR) (U.S. Pat. Nos. 4,683,195 and 4,683,202; PCR Protocols: A Guide to Methods and Applications (Innis et al., eds, 1990)), strand displacement amplification (SDA) (Walker, et al., Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), helicase dependent amplification (HAD) (M. Vincent, Y. Xu and H. Kong, EMBO Rep., 2004, 5, 795-800), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)), Q-Beta Replicase (Lizardi et al., BiolTechnology6:1197 (1988)), Loop-mediated isothermal amplification (LAMP) (Y. Mori, H. Kanda and T. Notomi, J. Infect. Chemother, 2013, 19, 404-411), or Recombinase Polymerase Amplification (RPA) (J. Li, J. Macdonald and F. von Stetten, Analyst, 2018, 144, 31-67).

Various DNA polymerase may be used in the amplification reaction and include the "Klenow" fragment of E. coli DNA polymerase I, thermostable DNA polymerase, bacteriophage T7 DNA polymerase, Vent^{®} polymerase, and Terminator^{™} polymerase. In particular, the polymerase is a thermostable DNA polymerase obtainable from various bacteria, which include *Thermus aquaticus* (Taq), *Thermus thermophilus* (Tth), *Thermus filiformis*, *Thermus flavus, Thermococcus litoralis,* and *Pyrococcus furiosus* (Pfu). Most of the above polymerases can be isolated as is from bacteria, or can be commercially purchased.

The above-described amplification method can amplify a target nucleic acid and/or signal through a repetition of a series of reactions with or without change in temperature. The unit of amplification involving the repetition of such series of reactions, is referred to as a "cycle". The cycle may be expressed as the number of repetitions or a duration, depending on the amplification method used.

In one embodiment, the series of reactions may be carried out sequentially. For example, for a PCR, after denaturation of target nucleic acids (that is, templates), annealing of primers and subsequently, extension of the primers may be carried out sequentially. In this case, the cycle may be expressed as the number of repetitions.

In one embodiment, the series of reactions may be carried out simultaneously. In this case, the cycle may be expressed as a duration. In particular, 1 cycle may be 5 seconds, 10 seconds, 30 seconds, 1 minute, 2 minutes, 3 minutes, 4 minutes, 5 minutes, 10 minutes, 20 minutes, 30 minutes, 1 hour, or 2 hours.

In one embodiment, the incubating may be carried out for a plurality of cycles that allows for the measurement of a signal change dependent on the presence of a target nucleic acid. For example, the plurality of cycles may include 2 to 100 cycles, 2 to 90 cycles, 2 to 80 cycles, 2 to 70 cycles, 2 to 60 cycles, 2 to 50 cycles, 2 to 40 cycles, 2 to 30 cycles, 2 to 20 cycles, 2 to 10 cycles, 5 to 100 cycles, 5 to 90 cycles, 5 to 80 cycles, 5 to 70 cycles, 5 to 60 cycles, 5 to 50 cycles, 5 to 40 cycles, 5 to 30 cycles, 5 to 20 cycles, 5 to 10 cycles, 10 to 100 cycles, 10 to 90 cycles, 10 to 80 cycles, 10 to 70 cycles, 10 to 60 cycles, 10 to 50 cycles, 10 to 40 cycles, 10 to 30 cycles, 10 to 20 cycles, 20 to 100 cycles, 20 to 90 cycles, 20 to 80 cycles, 20 to 70 cycles, 20 to 60 cycles, 20 to 50 cycles, 20 to 40 cycles, or 20 to 30 cycles, and particularly, may include 10 cycles, 15 cycles, 20 cycles, 25 cycles, 30 cycles, 35 cycles, 40 cycles, 45 cycles, or 50 cycles.

In one embodiment, the detection of signals may be carried out in each cycle, in a selected few cycles, or in the end-point of an incubation reaction including a plurality of cycles.

In one embodiment, the amplification reaction of a target nucleic acid may be a multiple target nucleic acid amplification reaction.

The term "multiple target nucleic acid amplification reaction" used herein refers to a reaction that amplifies two or more nucleic acids as targets in a single reaction vessel. The multiple target nucleic acid amplification reaction refers to a reaction that amplifies two or more nucleic acids together. For example, the multiple target nucleic acid amplification reaction may amplify, in a single reaction, 2 or more, 3 or more, 4 or more, 5 or more, 6 or more, 7 or more, 8 or more, 9 or more, 10 or more, 20 or more, 30 or more, 40 or more, or 50 or more of target nucleic acids together.

In one embodiment, the method according to the present disclosure may detect 2 to 50, 2 to 40, 2 to 30, 2 to 20, 2 to 15, 2 to 12, 2 to 10, 2 to 9, 2 to 8, 2 to 7, 2 to 6, 2 to 5, 3 to 50, 3 to 40, 3 to 30, 3 to 20, 3 to 15, 3 to 12, 3 to 10, 3 to 9, 3 to 8, 3 to 7, 3 to 6, 3 to 5, 4 to 50, 4 to 40, 4 to 30, 4 to 20, 4 to 15, 4 to 12, 4 to 10, 4 to 9, 4 to 8, 4 to 7, 4 to 6 or 4 to 5 target nucleic acids by using a single type of label in a single reaction vessel.

In one embodiment, the method according to the present disclosure, when using multiple types of labels, may detect a greater number of target nucleic acids than the number of target nucleic acids detectable using a single type of label according to the method of the present disclosure, and for example, may be able to detect more target nucleic acids by a fold increase of the number of label types used.

The method according to the present disclosure is used to determine whether at least one of *n* target nucleic acids is present in a sample. For example, when *n is* 2, the present disclosure may be used to determine whether at least one of a first target nucleic acid and a second target nucleic acid is present in the sample. In another example, when *n* is 3, the present disclosure may be used to determine whether at least one of a first target nucleic acid, a second target nucleic acid, and a third target nucleic acid is present in the sample.

In one embodiment, *n* is an integer of 2 or more. For example, *n* may be 2, 3, 4, 5, 6, 7, 8, 9, 10, 11, 12, 13, 14, 15, 20, 25, 30, 35, 40, 45, or 50, but is not limited thereto.

In the present disclosure, a combination of compositions for detecting *n* target nucleic acids, *i.e.*, the first to the *n*^{th} target nucleic acids, is used to detect each of *n* target nucleic acids, *i.e.*, the first to the *n*^{th} target nucleic acids, wherein *n* is an integer of 2 or more.

As used herein, the term "combination of compositions for detecting the first to the *n*^{th} target nucleic acids" refers to a compilation or mixture of compositions specific for each of the first to *n*^{th} target nucleic acids. Herein, a composition for detecting one target nucleic acid is specific for a corresponding target nucleic acid. The expression "a composition for detecting a target nucleic acid is specific for a corresponding target nucleic acid" means that the composition for detecting the target nucleic acid is involved in detecting the corresponding target nucleic acid, but is not involved in detecting other target nucleic acids. In other words, the expression means that the composition for detecting the target nucleic acid interacts with the corresponding target nucleic acid but does not interact with other target nucleic acids.

Herein, the composition for detecting the *n*^{th} target nucleic acid is specific for the *n*^{th} target nucleic acid. For example, the composition for detecting the first target nucleic acid is specific for the first target nucleic acid, the composition for detecting the second target nucleic acid is specific for the second target nucleic acid, and the composition for detecting the third target nucleic acid is specific to the third target nucleic acid.

The combination of compositions for detecting the first to the *n*^{th} target nucleic acids as used herein is employed in a single reaction. In other words, the compositions for detecting the first to the *n*^{th} target nucleic acids coexist in a single reaction solution or reaction vessel.

As used herein, the term "composition for detecting a target nucleic acid" refers to a composition comprising components used to detect a target nucleic acid.

According to the method of the present disclosure, each of the compositions for detecting the first to *n*^{th} target nucleic acids comprises a label that provides a signal depending on the presence of a corresponding target nucleic acid among the first to *n*^{th} target nucleic acids, and the signals provided from each of the compositions for detecting the first to *n*^{th} target nucleic acids are not distinguished from each other by a single detection channel.

The composition for detecting a target nucleic acid may comprises various oligonucleotides involved in amplifying and detecting the target nucleic acid.

The label herein may be linked to an oligonucleotide or may exist in free form. Alternatively, the label may be incorporated into the oligonucleotide during the incubating.

Although the label and oligonucleotide are described as key elements in the compositions for detecting the first to *n*^{th} target nucleic acids, it should be understood that various other components may be also included in the compositions.

Examples of components included in the composition for detecting the target nucleic acid include, but are not limited to, an oligonucleotide set used to amplify or detect the target nucleic acid, a label, a nucleic acid polymerase, a buffer, a polymerase cofactor, and a deoxyribonucleotide-5-triphosphate. Optionally, the composition for detecting the target nucleic acid may also include various polynucleotide molecules, reverse transcriptase, various buffers and reagents, and antibodies that inhibit nucleic acid polymerase activity. The composition for detecting the target nucleic acid may also include an oligonucleotide set or reagents necessary for performing a positive control reaction. Optimal amounts of reagents to be used in a given reaction can be readily determined by one of ordinary skill in the art having the benefit of the present disclosure. The above-described components of the composition for detecting the target nucleic acid may be present or stored in one or more containers before the reaction.

Each of the *n* compositions for detecting target nucleic acids used in the present disclosure is any one of (i) an UnderSC (Under-Signal-Change) composition, (ii) an InterSC (Inter-Signal-Change) composition, and (iii) an OverSC (Over-Signal-Change) composition, and the composition for detecting the *n*^{th} target nucleic acid among the *n* compositions for detecting target nucleic acids is a Q-PTOCE composition.

In one embodiment, when *n* is 2, the composition for detecting the first target nucleic acid is an UnderSC composition or an InterSC composition, and the composition for detecting the second target nucleic acid is a Q-PTOCE composition, which is an OverSC composition.

In one embodiment, when *n is* 3 or more, the composition for detecting the first target nucleic acid is an UnderSC composition or an InterSC composition, the composition for detecting the *n*^{th} target nucleic acid is a Q-PTOCE composition, which is an OverSC composition, and each of the compositions for detecting target nucleic acids other than the first and *n*^{th} target nucleic acids is an InterSC composition.

Specifically, when *n* is 2, the composition for detecting the first target nucleic acid and the composition for detecting the second target nucleic acid may be combined as shown in Table 1 below. When *n* is 3, the compositions for detecting the first to the third target nucleic acids may be combined as shown in Table 2. When *n* is 4, the compositions for detecting the first to the fourth target nucleic acids may be combined as shown in Table 3." In Tables 1 to 3, "UnderSC" and "InterSC" refer to UnderSC and InterSC compositions to which various UnderSC and InterSC signal generation mechanisms known in the art are respectively applied."

**[Table 1]**

| when *n* is 2 | | |
|---|---|---|
| N=2 | The first target | The second target |
| 1 | UnderSC | Q-PTOCE composition |
| 2 | InterSC | Q-PTOCE composition |

**[Table 2]**

| when *n* is 3 | | | |
|---|---|---|---|
| N=3 | The first target | The second target | The third target |
| 1 | UnderSC | InterSC | Q-PTOCE composition |
| 2 | InterSC | InterSC | Q-PTOCE composition |

**[Table 3]**

| when *n* is 4 | | | | |
|---|---|---|---|---|
| N=3 | The first target | The second target | The third target | The fourth target |
| 1 | UnderSC | InterSC | InterSC | Q-PTOCE composition |
| 2 | InterSC | InterSC | InterSC | Q-PTOCE composition |

Details of the UnderSC and InterSC compositions are found in International Patent Application Publication WO 2022-265463, which is incorporated herein by reference in its entirety.

In one embodiment, each of the *n* compositions for detecting the target nucleic acids provides a signal change at a corresponding detection temperature among the *n* detection temperatures, the signal change indicating the presence of a corresponding target nucleic acid.

For example, the composition for detecting an *i*^{th} target nucleic acid among the *n* target nucleic acids provides a signal change at an *i*^{th} detection temperature among the *n* detection temperatures and provides a constant signal at the other detection temperatures in the presence of the *i*^{th} target nucleic acid.

In one embodiment, *i* represents an integer from 1 to *n,* and the *i*^{th} detection temperature is lower than the (*i*+1)^{th} detection temperature. In one embodiment, when *i* is *n,* there is no *i*+1 detection temperature (*i.e., n+1* detection temperature). For example, when *n is* 3, *i* represents an integer from 1 to 3, and there are a first detection temperature, a second detection temperature, and a third detection temperature, wherein the first detection temperature is lower than the second detection temperature, and the second detection temperature is lower than the third detection temperature.

According to the present disclosure, within the temperature range covering all of the *n* detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid.

In one embodiment, the composition for detecting the *i*^{th} target nucleic acid is any one of: (i) an Under-Signal-Change (UnderSC) composition having a characteristic that the signal-changing temperature range is lower than the signal-constant temperature range, (ii) an Inter-Signal-Change (InterSC) composition having a characteristic that the signal-changing temperature range is higher than one of two signal-constant temperature ranges, and lower than the other of the two signal-constant temperature ranges, and (iii) an Over-Signal-Change (OverSC) composition having a characteristic that the signal-changing temperature range is higher than the signal-constant temperature range.

In one embodiment, in the presence of the *i*^{th} target nucleic acid, the composition for detecting the *i*^{th} target nucleic acid provides a signal change (*i.e.,* change in an *i*^{th} signal) upon amplification of the target nucleic acid at the *i*^{th} detection temperature, but does not provide any signal change (*i.e.,* the signal is constant) at the other detection temperatures despite amplification of the target nucleic acid. That is, the composition for detecting the *i*^{th} target nucleic acid have a signal-changing temperature range in which the signal changes as the *i*^{th} target nucleic acid is amplified, and signal-constant temperature range(s) in which the signal is constant despite amplification of the *i*^{th} target nucleic acid.

The term "*i*^{th} signal" as used herein refers to a signal provided at an *i*^{th} detection temperature by the composition for detecting an *i*^{th} target nucleic acid, which is interchangeably used with "signal at an *i*^{th} detection temperature". In one embodiment, when detecting *n* target nucleic acids, the *i*^{th} signal may mean a signal provided by the *n* compositions for detecting target nucleic acids including the composition for detecting the *i*^{th} target nucleic acid, at the *i*^{th} detection temperature.

In one embodiment, the composition for detecting the *i*^{th} target nucleic acid provides no signal change, *i.e.*, provides a constant signal at the *i*^{th} detection temperature during an incubation reaction (e.g., a target nucleic acid amplification reaction) in the absence of the *i*^{th} target nucleic acid.

In one embodiment, the signal-changing temperature range is a temperature range in which a difference in signal value (e.g., signal intensity) occurs depending on the presence or absence of the target nucleic acid.

In one embodiment, the signal-changing temperature range is a temperature range in which a change in signal value occurs depending on the level of amplification of the target nucleic acid (e.g., the amount of the amplified target nucleic acid).

In one embodiment, the signal-constant temperature range is a temperature range in which the signal value does not change regardless of the presence of the target nucleic acid. In other words, the signal-constant temperature range is a temperature range in which there is no difference between the signal value in the presence of the target nucleic acid and the signal value in the absence of the target nucleic acid.

In one embodiment, the *i*^{th} detection temperature may be selected from within the signal-changing temperature range of the composition for detecting the *i*^{th} target nucleic acid. In the present disclosure, the composition for detecting the *i*^{th} target nucleic acid is referred to as having the *i*^{th} detection temperature. In addition, the *i*^{th} target nucleic acid corresponding to the composition for detecting the *i*^{th} target nucleic acid may be referred to as a target nucleic acid having the *i*^{th} detection temperature.

According to an embodiment, one detection temperature, which is determined by the composition for detecting a corresponding target nucleic acid, is assigned to one target nucleic acid.

In certain embodiments, when *n* is 2, the composition for detecting the first target nucleic acid provides a signal change at the first detection temperature and provides a constant signal at the other detection temperature, that is, the second detection temperature in the presence of the first target nucleic acid; and the composition for detecting the second target nucleic acid provides a signal change at the second detection temperature and provides a constant signal at the first detection temperature in the presence of the second target nucleic acid.

In certain embodiments, when *n* is 3, the composition for detecting the first target nucleic acid provides a signal change at the first detection temperature and provides a constant signal at the second detection temperature and the third detection temperature in the presence of the first target nucleic acid; the composition for detecting the second target nucleic acid provides a signal change at the second detection temperature and provides a constant signal at the first detection temperature and the third detection temperature in the presence of the second target nucleic acid; and the composition for detecting the third target nucleic acid provides a signal change at the third detection temperature and provides a constant signal at the first detection temperature and the second detection temperature in the presence of the third target nucleic acid.

In one embodiment, the *i*^{th} detection temperature is selected within the signal-changing temperature range of the composition for detecting the *i*^{th} target nucleic acid, and the *i*^{th} detection temperature is not included in the signal-changing temperature ranges of the compositions for detecting the other target nucleic acids.

In one embodiment, the signal-changing temperature range of any one of the compositions for detecting target nucleic acids may overlap with the signal-changing temperature range of a composition for detecting a target nucleic acid having an adjacent detection temperature, while not overlapping with the signal-changing temperature range of a composition for detecting a target nucleic acid having a detection temperature not adjacent thereto. In this case, the detection temperature of the composition for detecting a target nucleic acid that has the signal-changing temperature range overlapping with the signal-changing temperature range of the composition for detecting another target nucleic acid is selected within the signal-changing temperature range that does not overlap with the signal-changing temperature range of the composition for detecting another target nucleic acid. By selecting the detection temperature as such, only the signal indicative of the presence of a single particular target nucleic acid can be provided at a single detection temperature.

In one embodiment, the signal-changing temperature range of any one of compositions for detecting target nucleic acids may overlap with the signal-changing temperature range of the composition for detecting a target nucleic acid having an adjacent detection temperature, but either of the two signal-changing temperature ranges is not completely included in the other signal-changing temperature range.

The term "adjacent detection temperature" is used herein to refer to consecutive detection temperatures among *n* detection temperatures, and for example, the adjacent detection temperature of the *i*^{th} detection temperature is the *(i*-1)^{th} detection temperature or the (*i*+1)^{th} detection temperature.

In one embodiment, the signal-changing temperature range of the composition for detecting the *i*^{th} target nucleic acid may partially overlap with the signal-changing temperature range of the composition for detecting a target nucleic acid having an adjacent detection temperature, while not overlapping with the signal-changing temperature range of the composition for detecting a target nucleic acid having a detection temperature not adjacent thereto.

In one embodiment, the composition for detecting the *i*^{th} target nucleic acid comprises a label that provides a signal dependent on the presence of the *i*^{th} target nucleic acid.

In one embodiment, the label herein may be linked to an oligonucleotide or may exist in free form. Alternatively, the label may be incorporated into the oligonucleotide during the incubating (e.g., a nucleic acid amplification). In other words, the composition for detecting the target nucleic acid may initially include a labeled oligonucleotide or may provide a labeled oligonucleotide by incorporating the label into a newly generated oligonucleotide (e.g., an extended strand) during an incubation reaction.

In one embodiment, the composition for detecting the *i*^{th} target nucleic acid includes an incorporating label that is incorporated into an oligonucleotide during the incubating and provides a signal depending on the presence of the *i*^{th} target nucleic acid.

In one embodiment, the composition for detecting the *i*^{th} target nucleic acid provides a labeled oligonucleotide that serves to provide a signal depending on the presence of the *i*^{th} target nucleic acid.

In one embodiment, the composition for detecting the *i*^{th} target nucleic acid initially includes a labeled oligonucleotide that serves to provide a signal depending on the presence of the *i*^{th} target nucleic acid. The Q-PTO and the CTO as described herein correspond to examples of the labeled oligonucleotide.

Alternatively, the composition for detecting the *i*^{th} target nucleic acid may include an oligonucleotide and a label that provides a signal depending on the presence of the *i*^{th} target nucleic acid, and the label is incorporated into the oligonucleotide during an incubation reaction (e.g., a nucleic acid amplification reaction), thereby providing a labeled oligonucleotide that serves to provide a signal depending on the presence of the *i*^{th} target nucleic acid.

As used herein, the term "labeled oligonucleotide" refers to an oligonucleotide involved in the generation of a signal being detected.

In one embodiment, the labeled oligonucleotide may comprise an oligonucleotide (e.g., a probe or a primer) that specifically hybridizes to a target nucleic acid; when the probe or primer hybridized with the target nucleic acid is cleaved to release a fragment, the labeled oligonucleotide may comprise a capture oligonucleotide that specifically hybridizes to the fragment; when the fragment hybridized to the capture oligonucleotide is extended to form an extended strand, the labeled oligonucleotide may comprise an oligonucleotide that specifically hybridizes to the extended strand, an oligonucleotide that is produced by incorporating a label during the fragment extension, an oligonucleotide that specifically hybridizes with the capture oligonucleotide, and a combination thereof.

According to one embodiment, the labeled oligonucleotide includes an oligonucleotide involved in actual signal generation. For example, hybridization or non-hybridization between the labeled oligonucleotide and another oligonucleotide (e.g., an oligonucleotide comprising a nucleotide sequence complementary to the labeled oligonucleotide or the target nucleic acid) determines signal generation.

In one embodiment, the labeled oligonucleotide may be a 'probe' known in the art. According to an embodiment, the 3'-end of the probe is "blocked" to prohibit its extension. The blocking may be achieved in accordance with conventional methods. For instance, the blocking may be performed by adding to the 3'-hydroxyl group of the last nucleotide a chemical moiety such as biotin, labels, phosphate groups, alkyl groups, non-nucleotide linkers, phosphorothioate or alkane-diol residues. Alternatively, the blocking may be carried out by removing the 3'-hydroxyl group of the last nucleotide or using a nucleotide with no 3'-hydroxyl group such as dideoxynucleotide.

In one embodiment, the labeled oligonucleotide may be composed of at least one oligonucleotide. According to an embodiment, when the labeled oligonucleotide is composed of a plurality of oligonucleotides, the labeled oligonucleotide may be labeled in various fashions. For example, all or portion of the plurality of oligonucleotides may have at least one label.

In one embodiment, the label may be a single label or interactive labels.

For example, the single label includes a fluorescent label, a luminescent label, a chemiluminescent label, an electrochemical label, and a metal label. In one embodiment, the single label provides different signals (for example, different signal intensities) depending on its presence on a double strand or a single strand. In one embodiment, the single label is a fluorescent label. Preferred types and binding sites of single fluorescent labels used in the present disclosure are disclosed in U.S. Pat. Nos. 7,537,886 and 7,348,141, the teachings of which are incorporated herein by reference in their entirety. For example, the single fluorescent label includes JOE, FAM, TAMRA, ROX, and fluorescein-based label. The single label may be linked to an oligonucleotide by various methods. For example, the label may be linked to a probe via a spacer containing carbon atoms (e.g., a 3-carbon spacer, a 6-carbon spacer, or a 12-carbon spacer).

In one embodiment, the interactive labels may be those comprising at least one reporter molecule and at least one quencher molecule. In particular, the interactive labels may be interactive dual labels comprising one reporter molecule and one quencher molecule. Or, the interactive labels may be those comprising one reporter molecule and two quencher molecules.

As a representative example of the interactive label system, the FRET (fluorescence resonance energy transfer) label system includes a fluorescent reporter molecule (donor molecule) and a quencher molecule (acceptor molecule). In FRET, the energy donor is fluorescent, but the energy acceptor may be fluorescent or non-fluorescent. In another form of interactive label systems, the energy donor is non-fluorescent, e.g., a chromophore, and the energy acceptor is fluorescent. In yet another form of interactive label systems, the energy donor is luminescent, e.g., bioluminescent, chemiluminescent, or electrochemiluminescent, and the acceptor is fluorescent. The interactive label system includes a label pair based on "contact-mediated quenching" (Salvatore et al., Nucleic Acids Research, 2002 (30) no.21 e122 and Johansson et al., J. AM. CHEM. SOC 2002 (124) pp 6950-6956). The interactive label system includes any label system in which signal change is induced by interaction between at least two molecules (e.g., dyes).

Reporter molecule and quencher molecule useful in the present disclosure may include any molecules known in the art, details of which are found in the section describing reporter molecule and quencher molecule in the first aspect of the specification.

In one embodiment, when the label is interactive labels, the interactive labels may include at least one reporter molecule and at least one quencher molecule, wherein the interactive labels may be all linked to one oligonucleotide or may be linked to each of a plurality of oligonucleotides.

In one embodiment, an incorporating label may be used in the process of incorporating a label during a primer extension to generate a signal (e.g., Plexor technology, Sherrill C B et al., Journal of the American Chemical Society, 126:4550-45569 (2004)). In addition, the incorporating label may be used in a signal generation by a duplex formed in a manner dependent on cleavage of a mediation oligonucleotide hybridized with a target nucleic acid.

In one embodiment, the incorporating label may be generally linked to a nucleotide. In addition, a nucleotide having a non-natural base may be used.

As used herein, the term "non-natural base" refers to derivatives of natural bases such as adenine (A), guanine (G), thymine (T), cytosine (C) and uracil (U), which are capable of forming hydrogen-bonding base pairs. The term "non-natural base" as used herein includes bases having base pairing patterns different from natural bases as mother compounds, as described, for example, in U.S. Pat. Nos. 5,432,272, 5,965,364, 6,001,983, and 6,037,120. The base pairing between non-natural bases includes two or three hydrogen bonds as with natural bases. The base pairing between non-natural bases is also formed in a specific manner. Specific examples of non-natural bases include the following bases in base pair combinations: iso-C/iso-G, iso-dC/iso-dG, Z/P, V/J, K/X, H/J, Pa/Ds, Pa/Q, Pn/Ds, Pn/Dss, Px/Ds, NaM/5SICS, 5FM/5SICS, and M/N (see U.S. Pat. Nos. 5,432,272; 5,965,364; 6,001,983; 6,037,120; 6,140,496; 6,627,456; 6,617,106; and 7,422,850; and Filip Wojciechowski et al., Chem. Soc. Rev., 2011, 40, 5669-5679).

Conventional methods for detecting multiple target nucleic acids have the disadvantage of requiring the use of different types of fluorescent labels for different target nucleic acids or, even when using a single type of fluorescent label, requiring additional analyses such as melting curve analysis. In contrast, the method according to the present disclosure enables real-time detection of multiple target nucleic acids without the need for additional analyses such as melting curve analysis, by using a composition for detecting target nucleic acids that provides a duplex, while utilizing a single type of label (e.g., a single fluorescent label).

In one embodiment, each of the *n* compositions for detecting target nucleic acids provides one or more duplexes.

As used herein, the term "duplex" refers to a double-stranded nucleic acid molecule formed by hybridizing two single-stranded nucleic acid molecules having a sequence partially or totally complementary to each other under hybridization conditions. The two single-stranded nucleic acid molecules forming the duplex may exist in associated form (i.e., a double-stranded molecule) or dissociated form (i.e., two single-stranded molecules) depending on the temperature (in particular, the detection temperature). In this respect, the term "duplex" when referring the expression "a composition for detecting a target nucleic acid provides a duplex" is used to encompass both a duplex in associated form and a duplex in dissociated form.

In one embodiment, the duplex may be also referred to as "hybrid".

The term "association" or "dissociation" has the same meaning as the term "hybridization" or "denaturation", respectively.

As mentioned above, the expression "a composition for detecting a target nucleic acid provides a duplex" as used herein may mean that the composition provides a duplex in associated form and/or a duplex in dissociated form. Likewise, the expression "a composition for detecting a target nucleic acid generates a duplex during incubating" as used herein may mean that the composition generates a duplex in associated form and/or a duplex in dissociated form during an incubation reaction.

In one embodiment, at least one of the duplexes provided by the composition for detecting a target nucleic acid is a duplex providing a signal. In particular, the duplex is a duplex providing a signal change. In other words, the composition for detecting an *i*^{th} target nucleic acid provides a duplex providing a signal, and particularly, the composition for detecting the *i*^{th} target nucleic acid provides a duplex providing a signal change depending on the presence of the *i*^{th} target nucleic acid.

The term "duplex providing a signal" as used herein refers to a duplex capable of providing a signal that can be distinguished depending on whether the duplex is in associated form or dissociated form. For example, this means that the duplex in associated form generates (or extinguishes) a signal, and the duplex in dissociated form extinguishes (or generates) a signal.

In one embodiment, the duplex providing the signal may include at least one label.

As used herein, the term "duplex providing a signal change" refers to a duplex providing a signal change indicative of the presence of a target nucleic acid as the amount of the duplex providing the signal change changes depending on the presence of the target nucleic acid. In particular, the extended duplex according to the present disclosure corresponds to an example of a duplex providing a signal change as described herein.

In one embodiment, the duplex providing the signal change includes a label. In particular, at least one label is linked to at least one of the two single-stranded nucleic acid molecules constituting the duplex. For example, the duplex providing the signal change includes a single label, and in this case, the single label is linked to any one of the two single-stranded nucleic acid molecules constituting the duplex. As another example, the duplex providing the signal change includes interactive labels, and in this case, the interactive labels are all linked to one of the two single-stranded nucleic acid molecules constituting the duplex providing the signal change, or one of the interactive labels is linked to one of the two single-stranded nucleic acid molecules and the other of the interactive label is linked to the other of the two single-stranded nucleic acid molecules.

In one embodiment, the composition for detecting the *i*^{th} target nucleic acid provides a duplex providing a signal change.

In one embodiment, the composition for detecting the *i*^{th} target nucleic acid provides a signal from the label when the duplex providing the signal change is in associated form. In other words, the composition for detecting the *i*^{th} target nucleic acid provides a signal depending on association of the two single-stranded nucleic acid molecules constituting the duplex.

In one embodiment, the composition for detecting the *i*^{th} target nucleic acid provides a signal from the label when the duplex providing the signal change is in dissociated form. In other words, the composition for detecting the *i*^{th} target nucleic acid provides a signal depending on dissociation of the two single-stranded nucleic acid molecules constituting the duplex.

In one embodiment, the association or dissociation of the duplex may depend on temperature.

In one embodiment, the duplex providing the signal change may be a duplex that has initially (originally) been included in the composition for detecting a target nucleic acid.

In one embodiment, when the duplex providing the signal change has been included in the composition for detecting the target nucleic acid, the duplex may be generated by hybridization between a labeled oligonucleotide and an oligonucleotide hybridizable with the labeled oligonucleotide. A double-stranded nucleic acid hybridization probe (U.S. Pat. No. 7,799,522), also known as Yin-Yang probe, is an exemplary duplex providing a signal change, which has initially been included in the composition for detecting the target nucleic acid.

In one embodiment, when the duplex providing the signal change has initially been included in the composition for detecting the target nucleic acid, the amount of the duplex providing the signal change varies, in particular, decreases, in a manner dependent on the presence of the target nucleic acid, thereby providing the signal change. For example, a double-stranded nucleic acid hybridization probe (i.e., the duplex providing the signal change) initially included in the composition for detecting the target nucleic acid forms a new duplex as the target nucleic acid is amplified by hybridization of one of the two single strands constituting the Yin-Yang probe with the amplified target nucleic acid. As a result, the amount of the double-stranded nucleic acid hybridization probe decreases, thereby providing a signal change depending on the presence of the target nucleic acid.

In one embodiment, the duplex providing the signal change may be a duplex newly provided by the composition for detecting a target nucleic acid during an incubation reaction.

In one embodiment, the duplex providing the signal change, which is generated during the incubation reaction, may be provided by hybridization between a labeled oligonucleotide and the target nucleic acid.

Signals by formation of a duplex between the labeled oligonucleotide and the target nucleic acid may be generated by various methods, including Scorpion method (Whitcombe et al., Nature Biotechnology 17:804-807 (1999)), Sunrise (or Amplifluor) method (Nazarenko et al., Nucleic Acids Research, 25(12):2516-2521 (1997), and U.S. Pat. No. 6,117,635), LUX method (U.S. Pat. No. 7,537,886), Plexor method (Sherrill CB, et al., Journal of the American Chemical Society, 126:4550-4556 (2004)), Molecular beacon method (Tyagi et al., Nature Biotechnology v.14 MARCH 1996), Hybeacon method (French DJ et al., Mol Cell Probes, 15(6):363-374 (2001)), adjacent hybridization probe method (Bernard P.S. et al., Anal Biochem., 273:221 (1999)), and LNA method (U.S. Pat. No. 6,977,295).

In one embodiment, the duplex providing the signal change generated during the incubating reaction may be a duplex formed by a cleavage reaction dependent on the presence of the target nucleic acid. The extended duplex according to the present disclosure corresponds to an example of a duplex formed by a cleavage reaction that is dependent on the presence of the target nucleic acid.

In one embodiment, the signal change occurs by a duplex generated in a manner dependent on the cleavage of a mediation oligonucleotide specifically hybridized with the target nucleic acid.

As used herein, the term "mediation oligonucleotide" refers to an oligonucleotide mediating the generation of a duplex not including a target nucleic acid.

In one embodiment, the cleavage of the mediation oligonucleotide alone does not generate a signal, but after hybridization and cleavage of the mediation oligonucleotide, a fragment (a cleavage product) produced by the cleavage is involved in a series of reactions for signal generation.

In one embodiment, the hybridization or cleavage of the mediation oligonucleotide alone does not generate a signal.

In one embodiment, the mediation oligonucleotide includes an oligonucleotide that hybridizes with a target nucleic acid and is cleaved to release a fragment, thereby mediating the generation of a duplex.

In one embodiment, the fragment mediates the generation of a duplex by extension of the fragment on a capture oligonucleotide.

According to an embodiment, the mediation oligonucleotide comprises (i) a targeting portion comprising a hybridizing nucleotide sequence complementary to the target nucleic acid, and (ii) a tagging portion comprising a non-complementary nucleotide sequence to the target nucleic acid.

In one embodiment, the composition for detecting a target nucleic acid may include a tagging oligonucleotide that hybridizes with the target nucleic acid, and the cleavage reaction dependent on the presence of the target nucleic acid may involve cleavage of the tagging oligonucleotide. Specifically, the tagging oligonucleotide corresponds to an example of the mediation oligonucleotide described above. More specifically, the Q-PTO of the present disclosure corresponds to an example of the tagging oligonucleotide.

According to an embodiment, cleavage of the mediation oligonucleotide releases a fragment, and the fragment is specifically hybridized with a capture oligonucleotide and extended on the capture oligonucleotide. When the capture oligonucleotide comprises a label, the capture oligonucleotide corresponds to an example of the labeled oligonucleotide described herein.

According to an embodiment, the mediation oligonucleotide hybridized with a target nucleic acid is cleaved and releases a fragment, the fragment is specifically hybridized with a capture oligonucleotide, and the fragment is extended to generate an extended strand, which induces the formation of an extended duplex between the extended strand and the capture oligonucleotide, thereby providing a signal indicative of the presence of the target nucleic acid.

According to an embodiment, a third oligonucleotide comprising a hybridizing nucleotide sequence complementary to the extended strand may be additionally used. In this case, the hybridization of the extended strand and the third oligonucleotide forms another type of duplex, thereby providing a signal indicating the presence of the target nucleic acid (e.g., PCE-SH). The another type of duplex is a duplex providing the signal change.

Signals by a duplex generated in a manner dependent on cleavage of the mediation oligonucleotide may be generated by various methods, including PTOCE (PTO cleavage and extension) method (WO 2012/096523), PCE-SH (PTO Cleavage and Extension-Dependent Signaling Oligonucleotide Hybridization) method (WO 2013/115442), and PCE-NH (PTO Cleavage and Extension-Dependent Non-Hybridization) method (WO 2014/104818).

Regarding the terms disclosed in the above references, the corresponding examples of the oligonucleotides are as follows: the mediation oligonucleotide corresponds to a PTO (Probing and Tagging Oligonucleotide), the capture oligonucleotide corresponds to a CTO (Capturing and Templating Oligonucleotide), and the third oligonucleotide corresponds to an SO (Signaling Oligonucleotide) or an HO (Hybridization Oligonucleotide). The SO, HO, CTO, extended strand, or a combination thereof may serves as a labeled oligonucleotide.

In one embodiment, the duplex providing the signal change may be a single-typed duplex or plural-typed duplexes. Specifically, when the duplex providing the signal change is a single-typed duplex, the number of the duplexes may be 1, and when the duplex providing the signal change is plural-typed duplexes, the number of the duplexes may be 2, 3, 4, 5, 6, 7, 8, 9, 10, 15, or 20, specifically, 2, 3, or 4, and more specifically, 2 or 3.

In one embodiment, the single-typed duplex, or any of the plural-typed duplexes includes a label.

In one embodiment, when the duplex providing the signal change is a single-typed duplex, the amount of the single-typed duplex changes depending on the presence of a target nucleic acid, thereby changing the signal.

In one embodiment, when the duplex is plural-typed duplexes, the amount ratios between the plural-typed duplexes changes depending on the presence of a target nucleic acid, thereby changing the signal.

In one embodiment, Tm values of the plural-typed duplexes are different from each other. For example, the Tm values of the duplexes are different from each other by at least 2°C, at least 3°C, at least 4°C, at least 5°C, at least 7°C, at least 8°C, at least 9°C, at least 10°C, at least 11°C, at least 12°C, at least 13°C, at least 14°C, at least 15°C, or at least 20°C.

In one embodiment, the amount of the duplex refers to the sum of the amount of the duplex in a dissociated state, in which the two nucleic acid strands constituting the duplex are separated (i.e., a dissociated form of the duplex), and the amount of the duplex in an associated state, in which the two nucleic acid strands are hybridized (i.e., an associated form of the duplex).

In one embodiment, at least two of the plural-typed duplexes include the same single-stranded nucleic acid molecule. In this case, the same single-stranded nucleic acid molecule is included in a first duplex initially included in the composition for detecting the target nucleic acid, and during an incubation reaction, a new second duplex including the same single-stranded nucleic acid molecule may be generated. In this case, the same single-stranded nucleic acid molecule included in the first duplex can be considered to be consumed as it participates in the generation of the second duplex during the incubation reaction, and thus, the amount of the first duplex including the same single-stranded nucleic acid molecule decreases, whereas the amount of the second duplex including the same single-stranded nucleic acid molecule increases.

In one embodiment, the signal-changing temperature range of the composition for detecting the *i*^{th} target nucleic acid may be determined on the basis of the length and/or sequence of the duplex providing a signal change.

In one embodiment, when the composition for detecting the *i*^{th} target nucleic acid provides a single-typed duplex, the composition for detecting the target nucleic acid may have one signal-changing temperature range and one signal-constant temperature range. The signal-changing temperature range and the signal-constant temperature range may be determined on the basis of the length and/or sequence of the single-typed duplex.

In one embodiment, when the composition for detecting the *i*^{th} target nucleic acid provides plural-typed duplexes, in particular, two different types of duplexes, the composition for detecting the target nucleic acid may have one signal-changing temperature range and two signal-constant temperature ranges. The signal-changing temperature range and the signal-constant temperature ranges may be determined on the basis of the lengths and/or sequences of the two different types of duplexes.

In one embodiment, any one of the *n* compositions for detecting the target nucleic acids may include an amplification oligonucleotide that serves to amplify a corresponding target nucleic acid. In one embodiment, the amplification oligonucleotide may be the same as the labeled oligonucleotide.

As used herein, the term "amplification oligonucleotide" refers to any oligonucleotide that serves to amplify target nucleic acids.

In one embodiment, the amplification oligonucleotide may be a 'primer' known in the art. As used herein, the term 'primer' refers to an oligonucleotide that is capable of acting as a point of initiation of synthesis when placed under conditions in which synthesis of primer extension product which is complementary to a target nucleic acid strand (template) is induced, *i.e.*, in the presence of nucleotides and an agent for polymerization, such as DNA polymerase, and at a suitable temperature and pH. The primer must be long enough to prime the synthesis of extension products in the presence of the agent for polymerization. An appropriate length of the primer is determined by multiple factors, including temperature, the field of application, and the source of primer.

The primer may include a forward primer (also referred to as an upstream primer or an upstream oligonucleotide), a reverse primer (also referred to as a downstream primer or a downstream oligonucleotide), or both. The amplification oligonucleotide may be an oligonucleotide having a structure known in the art, or may be synthesized by a method known in the art.

That the amplification oligonucleotide and the labeled oligonucleotide are the same means that a single oligonucleotide simultaneously acts as an amplification oligonucleotide that amplifies a target nucleic acid, and as a labeled oligonucleotide that generates a signal in the presence of the target nucleic acid. As one example, the labeled oligonucleotide may be hybridized with the target nucleic acid and extended, thereby generating a signal.

Note that the composition for detecting a target nucleic acid used in the present disclosure does not necessarily provide a signal at any temperature in the presence of the target nucleic acid.

In one embodiment, even when the signal generation mechanisms of the compositions for detecting target nucleic acids are the same, the compositions for detecting target nucleic acids including oligonucleotides of different sequences may be considered to be different from each other. The different compositions for detecting nucleic acids have different detection temperatures from each other.

In one embodiment, the detection temperatures according to the present disclosure may be pre-determined in light of the signal-changing temperature range of each of the *n* compositions for detecting target nucleic acids.

In one embodiment, the signal-changing temperature range of any one of the *n* compositions for detecting target nucleic acids may be determined on the basis of the length and/or sequence of the duplex. In other words, by adjusting the Tm value of the duplex, the signal-changing temperature range may be predetermined.

In one embodiment, when the signal change is generated by a labeled oligonucleotide (e.g., a molecular beacon) that specifically hybridizes to the target nucleic acid, the detection of signals may be successfully achieved at a predetermined detection temperature by adjusting the Tm value of the labeled oligonucleotide.

In one embodiment, when a scorpion primer is used as the labeled oligonucleotide, the detection of the signal is successfully achieved at a predetermined temperature by adjusting the Tm value of the region hybridizing with the extended strand.

In one embodiment, when the signal is provided by a duplex generated in the presence of the target nucleic acid, the detection of the signal is successfully achieved at a predetermined temperature by adjusting the Tm value of the duplex.

As described above, the detection temperature is determined in view of the signal-changing temperature range that varies depending on the duplex provided by the composition for detecting a target nucleic acid.

In one embodiment, the detection temperature of any one of the *n* compositions for the detection of *n* target nucleic acids may be predetermined within a signal-changing temperature range that does not overlap with the signal-changing temperature ranges of the other compositions.

In one embodiment, the detection temperatures assigned to the compositions for detecting the target nucleic acids are different from each other by at least 2°C, at least 3°C, at least 4°C, at least 5°C, at least 7°C, at least 8°C, at least 9°C, at least 10°C, at least 11°C, at least 12°C, at least 15°C, at least 20°C, or more.

In one embodiment, the *n* detection temperatures may be selected within a temperature range of 45°C to 97°C, 45°C to 96°C, 45°C to 95°C, 45°C to 94°C, 45°C to 93°C, 45°C to 92°C, 45°C to 91°C, 45°C to 90°C, 46°C to 97°C, 46°C to 96°C, 46°C to 95°C, 46°C to 94°C, 46°C to 93°C, 46°C to 92°C, 46°C to 91°C, 46°C to 90°C, 47°C to 97°C, 47°C to 96°C, 47°C to 95°C, 47°C to 94°C, 47°C to 93°C, 47°C to 92°C, 47°C to 91°C, 47°C to 90°C, 48°C to 97°C, 48°C to 96°C, 48°C to 95°C, 48°C to 94°C, 48°C to 93°C, 48°C to 92°C, 48°C to 91°C, 48°C to 90°C, 49°C to 97°C, 49°C to 96°C, 49°C to 95°C, 49°C to 94°C, 49°C to 93°C, 49°C to 92°C, 49°C to 91°C, 49°C to 90°C, 50°C to 97°C, 50°C to 96°C, 50°C to 95°C, 50°C to 94°C, 50°C to 93°C, 50°C to 92°C, 50°C to 91°C, or 50°C to 90°C.

For example, the highest detection temperature *(i.e.,* the *n*^{th} detection temperature) among the *n* detection temperatures may be selected within a temperature range of 70°C to 97°C, 70°C to 95°C, 70°C to 93°C, 70°C to 90°C, 73°C to 97°C, 73°C to 95°C, 73°C to 93°C, 73°C to 90°C, 75°C to 97°C, 75°C to 95°C, 75°C to 93°C, 75°C to 90°C, 78°C to 97°C, 78°C to 95°C, 78°C to 93°C, 78°C to 90°C, 80°C to 97°C, 80°C to 95°C, 80°C to 93°C, 80°C to 90°C, 83°C to 97°C, 83°C to 95°C, 83°C to 93°C, 83°C to 90°C, 85°C to 97°C, 85°C to 95°C, 85°C to 93°C, or 85°C to 90°C.

For example, the lowest detection temperature *(i.e.,* a first detection temperature) among the *n* detection temperatures may be selected within a temperature range of 45°C to 70°C, 45°C to 68°C, 45°C to 65°C, 45°C to 63°C, 45°C to 60°C, 45°C to 58°C, 45°C to 55°C, 48°C to 70°C, 48°C to 68°C, 48°C to 65°C, 48°C to 63°C, 48°C to 60°C, 48°C to 58°C, 48°C to 55°C, 50°C to 70°C, 50°C to 68°C, 50°C to 65°C, 50°C to 63°C, 50°C to 60°C, 50°C to 58°C, or 50°C to 55°C.

For example, intermediate detection temperatures (for example, from a second detection temperature to the (*n*-1)^{th} detection temperature) among the *n* detection temperatures may be selected within a temperature range of 55°C to 85°C, 55°C to 83°C, 55°C to 80°C, 55°C to 78°C, 55°C to 7.5°C, 55°C to 73°C, 55°C to 70°C, 55°C to 68°C, 55°C to 65°C, 55°C to 63°C, 55°C to 60°C, 58°C to 85°C, 58°C to 83°C, 58°C to 80°C, 58°C to 78°C, 58°C to 75°C, 58°C to 73°C, 58°C to 70°C, 58°C to 68°C, 58°C to 65°C, 58°C to 63°C, 58°C to 60°C, 60°C to 85°C, 60°C to 83°C, 60°C to 80°C, 60°C to 78°C, 60°C to 75°C, 60°C to 73°C, 60°C to 70°C, 60°C to 68°C, 60°C to 65°C, 60°C to 63°C, 63°C to 85°C, 63°C to 83°C, 63°C to 80°C, 63°C to 78°C, 63°C to 75°C, 63°C to 73°C, 63°C to 70°C, 63°C to 68°C, 63°C to 65°C, 65°C to 85°C, 65°C to 83°C, 65°C to 80°C, 65°C to 78°C, 65°C to 75°C, 65°C to 73°C, 65°C to 70°C, 65°C to 68°C, 68°C to 85°C, 68°C to 83°C, 68°C to 80°C, 68°C to 78°C, 68°C to 75°C, 68°C to 73°C, 68°C to 70°C, 70°C to 85°C, 70°C to 83°C, 70°C to 80°C, 70°C to 78°C, 70°C to 75°C, or 70°C to 73°C.

According to an embodiment, the *n* target nucleic acids are each assigned to *n* detection temperatures, *n* compositions for detecting the *n* target nucleic acids appropriate for the *n* detection temperatures are prepared, and then step (a) may be performed.

In one embodiment, when *n is* 3, the first detection temperature may be selected from within a temperature range of 50°C to 60°C, the second detection temperature may be selected from within a temperature range of 65°C to 75°C, and the third detection temperature may be selected from within a temperature range of 80°C to 95°C.

In step (a), signals are detected at the *n* detection temperatures during the incubating.

In one embodiment, the detection of signals may be carried out in each cycle, in selected cycles, or at the end-point of reaction.

In one embodiment, the detection of signals may be carried out in at least one cycle. For example, the signals may be detected at *n* detection temperatures in one selected cycle or at *n* detection temperatures in each of two selected cycles. For example, when *n is* 3 and signals are detected in cycle 1 and cycle 30, the signals (*i.e.,* a first signal, a second signal, and a third signal) are detected at a first detection temperature, a second detection temperature, and a third detection temperature in cycle 1, and the signals are detected at the first detection temperature, the second detection temperature, and the third detection temperature in cycle 30.

In one embodiment, the detection of the signals may be carried out in at least two cycles.

In one embodiment, the signal change may be measured using the signals detected in at least two cycles. For example, the amplification of nucleic acids may be carried out over 30 cycles, 40 cycles, 45 cycles, or 50 cycles of PCR, and in each cycle, signals may be measured at *n* detection temperatures. Then, the values of signals detected at each detection temperature in a plurality of cycles may be depicted as an amplification curve (a collection of data points of cycles and RFUs in cycles) at each detection temperature. As a specific example, when *n* is 3, in the presence of each target nucleic acid, an amplification curve at the first detection temperature, an amplification curve at the second detection temperature, and an amplification curve at the third detection temperature may be obtained, and then change in signal may be identified from each of the amplification curves.

The term "amplification curve" as used herein refers to a curve resulting from a signal-generation reaction, in particular an amplification reaction of a target analyte (in particular, target nucleic acid). The amplification curve includes a curve resulting from an amplification reaction in the presence of the target nucleic acid in the sample, or a curve or line resulting from an amplification reaction in the absence of the target nucleic acid in the sample.

In one embodiment, a signal change and/or a constant signal may be measured from an indicator of amplification of a target nucleic acid.

As used herein, the term "indicator of amplification" refers to any indicator which is closely related to the occurrence of amplification of a target nucleic acid and obtainable from the signal provided in step (a). The indicator may refer to a value which is generated dependently on the amplification of a target nucleic acid. The indicator may be an indicator having larger values as the target nucleic acid is amplified (*i.e.,* as the amount of the target nucleic acid increases), or may be an indicator having smaller values as the target nucleic acid is amplified. The indicator may be any indicator as long as it indicates amplification of the target nucleic acid.

In one embodiment, the indicator may include one obtained from an amplification curve. The indicator may include a signal value (e.g., RFU) in a particular cycle, a signal value in each cycle, a difference in signal values between particular cycles, or a difference between a reference signal value and a signal value in a particular cycle in an amplification curve. In one embodiment, examples of the indicator include, without limitation, Ct (cycle threshold) value, ΔRFU (e.g., difference in RFUs in two cycles, difference between a reference RFU and a RFU in a particular cycle, etc.) or RFU ratio (e.g., ratio of RFUs in two cycles or ratio of RFUs between a reference RFU and a RFU in a particular cycle, etc.).

According to an embodiment, the indicator of amplification is the Ct value or Cq value. The concept of the Ct value and Cq value are well known in the art.

According to an embodiment, the indicator of amplification is the ΔRFU or RFU ratio between RFU values obtained in an amplification reaction. For example, the indicator is the difference (subtraction) or ratio between RFUs in two cycles, or the difference (subtraction) or ratio between a RFU in a particular cycle and a reference RFU.

The method according to the present disclosure exploits the fact that the composition for detecting a target nucleic acid provides a signal change depending on the presence of the target nucleic acid, only at a corresponding detection temperature. According to an embodiment, the method according to the present disclosure can measure a signal change using signal values detected at detection temperatures in at least two cycles. In another embodiment, the method according to the present disclosure can measure a signal change by using a signal value detected at a detection temperature in one cycle (*i.e.,* a signal value detected in step (a)), and a reference signal value.

In one embodiment, when signals are detected in a plurality of cycles in step (a), the first cycle and the end cycle in which signals are detected may be selected to be separated from each other by at least 1 cycle to at least 20 cycles. In particular, the first cycle and the end cycle in which the signals are detected may be selected to be separated from each other by 1 cycle, 2 cycles, 3 cycles, 4 cycles, 5 cycles, 6 cycles, 7 cycles, 8 cycles, 9 cycles, 10 cycles, 11 cycles, 12 cycles, 13 cycles, 14 cycles, 15 cycles, 16 cycles, 17 cycles, 18 cycles, 19 cycles, 20 cycles, or more, and more specifically, by 5 cycles, 10 cycles, 15 cycles, 20 cycles, 30 cycles, or more.

In one embodiment, the detection of signals may be performed in at least one of intermediate cycles including an exponential phase region, or in at least one of late cycles including a plateau region. For example, when signals may be detected in two cycles, one cycle being any of early cycles including a baseline region and the other cycle being any of intermediate or late cycles, or one cycle being any of intermediate cycles and the other cycle being any of intermediate or late cycles.

In one embodiment, the early cycles include cycles from cycle 1 to any cycle close to the value obtained by dividing the end cycle by 3. For example, when the end cycle is 45, 45 divided by 3 equals to 15, and thus, the early cycles may be determined to be from cycle 1 to cycle 20, cycle 1 to cycle 15, cycle 1 to cycle 10, or cycle 1 to cycle 5. The intermediate cycles may be cycles close to a value obtained by dividing the end cycle by 2. For example, when the end cycle is cycle 45, 45 divided by 2 equals to 22.5, and thus, the intermediate cycles may be from cycle 16 to cycle 30, cycle 18 to cycle 30, cycle 20 to cycle 30, cycle 16 to cycle 27, cycle 18 to cycle 27, cycle 20 to cycle 27, cycle 16 to cycle 25, cycle 18 to cycle 25, or cycle 20 to cycle 25. The late cycles may be the end cycle of, or cycles close to the end cycle of an amplification reaction. For example, when the end cycle is cycle 45, the late cycles may be from cycle 31 to cycle 45, cycle 35 to cycle 45, cycle 38 to cycle 45, cycle 40 to cycle 45, or cycle 43 to cycle 45. The early cycles, intermediate cycles and late cycles may vary depending on the end cycle of the amplification reaction.

In one embodiment, the signal change may be measured using a signal value detected in at least one cycle and a "reference signal value". The reference signal value **may refer** to a value that can be used to confirm a signal change dependent on the presence of the target nucleic acid through a separate reaction.

In one embodiment, the reference signal value may be obtained from a reaction in the absence of a corresponding target nucleic acid at a corresponding detection temperature. For example, the reference signal value may be a "signal value at a detection temperature" in the absence of the target nucleic acid.

In one embodiment, there are *n* reference signal values for *n* detection temperatures.

In one embodiment, the "signal value at a detection temperature (e.g., an *i*^{th} detection temperature)" detected in the absence of the target nucleic acid (e.g., an *i*^{th} target nucleic acid) may be obtained through a separate negative control reaction.

In one embodiment, the reference signal value may be obtained by performing a negative control reaction at the same time as or separately from the method according to the present disclosure.

In one embodiment, the reference signal value may be obtained through a negative control reaction. According to certain embodiments, the reference signal value at the *i*^{th} detection temperature may be obtained by mixing a sample (e.g., distilled water) free of the *i*^{th} target nucleic acid with *n* compositions for detecting *n* target nucleic acids and detecting signals at the *i*^{th} detection temperature while amplifying nucleic acids. Here, the detection of signals may be performed in any cycle. Specifically, a signal value detected in any of the early cycles or late cycles of the negative control reaction may be used as the reference signal value. More specifically, a signal value detected in the same cycle as the cycle in which the signal is detected in step (a) may be used as the reference signal value.

In one embodiment, the reference signal value may be obtained through a positive control reaction. According to certain embodiments, the reference signal value may be obtained by mixing a sample containing an *i*^{th} target nucleic acid with the composition for detecting an *i*^{th} target nucleic acid and detecting a signal at an *i*^{th} detection temperature while amplifying the target nucleic acids.

When the reference signal value is obtained through the positive control reaction, the cycle in which a signal value is detected may be in a baseline region of the positive control reaction. The baseline region refers to a region in which a signal (e.g., a fluorescent signal) remains substantially constant over the early (initial) cycles of an amplification reaction (e.g., PCR). In this region, since the level of amplification products is not enough to be detectable, most of the fluorescent signals in this region are attributed to the fluorescent signal inherent to the reaction sample, and to the background signal including the fluorescent signals of the measurement system itself. In other words, a signal value detected in a cycle in the baseline region of the positive control reaction will be substantially identical to the reference signal value obtained from a reaction in the absence of the target nucleic acid (e.g., the negative control reaction).

In one embodiment, a signal change may be measured through a difference between the reference signal value and the signal value detected in step (a).

In one embodiment, the reference signal value may be a threshold value predetermined from a negative control reaction, by taking into consideration the background signal and sensitivity of the detector, or characteristics of the labels used. Using the threshold value, significance of a signal change may be determined. The threshold value may be determined by any threshold setting method known in the art. For example, the threshold value may be determined in view of the background signal, sensitivity, label characteristics, signal variation of a detector, or margin of errors, and the like.

In one embodiment, when the signal value detected in step (a) is equal to or greater than the threshold value as the reference signal value, it may be determined that the signal has changed.

In one embodiment, detection of signals at each of the *n* detection temperatures may be performed using a single type of detector.

In one embodiment, the single type of detector is one detector. In one embodiment, the signals from the labels in each of the labeled oligonucleotides included in the *n* compositions for detecting target nucleic acids are not differentiated from each other by a single type of detector with respect to each target nucleic acid.

As used herein, a single or one type of fluorescent label refers to a fluorescent label that has identical or substantially identical signal characteristics (e.g., optical characteristics, emission wavelength, and electric signals). For example, FAM and CAL Fluor 610 provide different types of signals.

As used herein, the single or one type of fluorescent label means that signals from the fluorescent label are not differentiated from each other, using a detection channel. Such a single or one type of fluorescent label is not based on the chemical structure of the fluorescent label, and even when two fluorescent labels having different chemical structures are not differentiated using a detection channel, they are considered as one type.

According to the present disclosure, signals generated from the *n* compositions for detecting target nucleic acids that include one type of fluorescent label in common are not differentiated by one detection channel.

The term "detection channel" as used herein refers to a means for detecting a signal from a single type of a fluorescent label. Thermocyclers available in the art, e.g., ABI 7500 (Applied Biosystems), QuantStudio (Applied Biosystems), CFX96 (Bio-Rad Laboratories), Cobas z 480 (Roche), LightCycler (Roche), etc. include a several channels (e.g., optical diodes) for detecting signals from a few different types of fluorescent labels, and these channels correspond to the detection channel as used herein.

The detection channel as used herein includes a means for detecting signals. For example, the detection channel may be an optical diode capable of detecting a fluorescent signal at a particular wavelength.

In one embodiment, the signals detected at the *n* detection temperatures are not differentiated from each other by the single type of detector.

### Step (b): Determining the Presence of Target Nucleic Acid

After detection of signals, the presence of *n* target nucleic acids is determined from the signals detected in step (a).

In one embodiment, the presence of the *i*^{th} target nucleic acid is determined by the signal change detected at the *i*^{th} detection temperature. For example, a signal change is measured from the signals detected at the *i*^{th} detection temperature, to determine the presence of the *i*^{th} target nucleic acid.

In one embodiment, when a change in signal at the *i*^{th} detection temperature is detected, it may be determined that the *i*^{th} target nucleic acid is present.

In one embodiment, when the signal is constant at the *i*^{th} detection temperature, it may be determined that the *i*^{th} target nucleic acid is absent.

In one embodiment, the signal change can be measured using signals detected in at least two cycles, or a signal detected in at least one cycle and "a reference signal value".

Determining the presence of the target nucleic acid from signals detected at each detection temperature may be carried out by the process described in step (a) for measuring a signal change, e.g., a method using an indicator of amplification, or any other method known in the art.

In certain embodiments, when *n* is 3 and detection of signals is carried out in cycle 10, cycle 20, and cycle 30, the presence of the first target nucleic acid may be determined from signals detected at a first detection temperature (a first signal in cycle 10, a first signal in cycle 20, and a first signal in cycle 30), the presence of the second target nucleic acid may be determined from signals detected at a second detection temperature (a second signal in cycle 10, a second signal in cycle 20, and a second signal in cycle 30), and the presence of the third target nucleic acid may be determined from signals detected at a third detection temperature (a third signal in cycle 10, a third signal in cycle 20, and a third signal in cycle 30).

In certain embodiments, when *n is* 4 and detection of signals is performed in cycle 30, the presence of the first target nucleic acid is determined from a signal detected at a first detection temperature (*i.e.,* a first signal in cycle 30) and a reference signal value, the presence of the second target nucleic acid is determined from a signal detected at a second detection temperature (*i.e.,* a second signal in cycle 30) and a reference signal value, and the presence of the third target nucleic acid is determined from a signal detected at a third detection temperature (*i.e.,* a third signal in cycle 30) and a reference signal value.

In one embodiment, the reference signal value may be obtained through a separate negative control reaction or positive control reaction.

In one embodiment, the method according to the present disclosure may be performed along with a negative control reaction. A signal value detected in the negative control reaction may be used as a reference signal value. For example, a signal detected at an *i*^{th} detection temperature in one cycle (e.g., the end cycle) of a reaction comprising the composition for detecting an *i*^{th} target nucleic acid, may be compared with a signal detected at the same detection temperature (*i.e.,* the *i*^{th} detection temperature) in the same cycle (e.g., the end cycle) of the negative control reaction to determine whether or not the signal has changed.

In certain embodiments, when *n* is 3 and a signal is detected in cycle 30, the presence of the first target nucleic acid may be determined from a signal detected at the first detection temperature (*i.e.,* a first signal in cycle 30) and a first reference signal value (e.g., a signal detected at the first detection temperature in cycle 30 of a negative control reaction), the presence of the second target nucleic acid may be determined from a signal detected at the second detection temperature (*i.e.,* a second signal in cycle 30) and a second reference signal value (e.g., a signal detected at the second detection temperature in cycle 30 of a negative control reaction), and the presence of the third target nucleic acid may be determined from a signal detected at the third detection temperature (*i.e., a* third signal in cycle 30) and a third reference signal value (e.g., a signal detected at the third detection temperature in cycle 30 of a negative control reaction).

In one embodiment, the method according to the present disclosure may be performed along with a positive control reaction. A signal value detected in the positive control reaction may be used as a reference signal value. For example, a first signal detected at an *i*^{th} detection temperature in one cycle, e.g., cycle 30, may be compared with a signal detected at the *i*^{th} detection temperature in a cycle, e.g., cycle 1 prior to cycle 30 of a positive control reaction to determine whether the signal has changed.

In one embodiment, when signal detection is performed in one cycle in step (a) and a signal change is measured using a reference signal value obtained through a positive control reaction, the signal detection in the positive control reaction to obtain the reference signal value may be performed in a cycle that is at least 30 cycles, at least 20 cycles, at least 10 cycles, or at least 5 cycles prior to the cycle in which the signal detection is performed in step (a).

In certain embodiments, when *n is* 3 and signal detection is performed in cycle 30, the presence of the first target nucleic acid may be determined from a signal detected at the first detection temperature (*i.e.,* a first signal in cycle 30) and a first reference signal value (e.g., a signal detected at the first detection temperature in cycle 1 of a positive control reaction for the first target nucleic acid), the presence of the second target nucleic acid may be determined from a signal detected at the second detection temperature (*i.e.,* a second signal in cycle 30) and a second reference signal value (e.g., a signal detected at the second detection temperature in cycle 1 of a positive control reaction for the second target nucleic acid), and the presence of the third target nucleic acid may be determined from a signal detected at the third detection temperature (*i.e.,* a third signal in cycle 30) and a third reference signal value (e.g., a signal detected at the third detection temperature in cycle 1 of a positive control reaction for the third target nucleic acid).

### IV. Kit for Detecting Target Nucleic Acid

In another aspect, the present disclosure provides a kit comprising n compositions for detecting n target nucleic acids in a sample:
wherein *n* is an integer of 2 or more,
wherein each of the *n* compositions for detecting the target nucleic acids provides a signal change at a corresponding detection temperature among the *n* detection temperatures in the presence of a corresponding target nucleic acid, the signal change indicating the presence of a corresponding target nucleic acid,
   wherein a composition for detecting an *i*^{th} target nucleic acid among the *n* compositions for detecting the target nucleic acids provides a signal change at an *i*^{th} detection temperature among the *n* detection temperatures and provides a constant signal at the other detection temperatures in the presence of the *i*^{th} target nucleic acid,
wherein *i* represents an integer from 1 to *n,* and the *i*^{th} detection temperature is lower than a (*i*+1)^{th} detection temperature,
wherein in the temperature range covering all of the *n* detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid,
wherein the composition for detecting the *i*^{th} target nucleic acid is any one of:
   (i) an Under-Signal-Change (UnderSC) composition having a characteristic that the signal-changing temperature range is lower than the signal-constant temperature range,
   (ii) an Inter-Signal-Change (InterSC) composition having a characteristic that the signal-changing temperature range is higher than one of two signal-constant temperature ranges, and lower than the other of the two signal-constant temperature ranges, and
   (iii) an Over-Signal-Change (OverSC) composition having a characteristic that the signal-changing temperature range is higher than the signal-constant temperature range, and
wherein the composition for detecting the *n*^{th} target nucleic acid among the *n* compositions for detecting the *n* target nucleic acids comprises a primer, a Quenching-Probing and Tagging Oligonucleotide (Q-PTO) and a Capturing and Templating Oligonucleotide (CTO),
   wherein the primer comprises a hybridizing nucleotide sequence to a first region of the target nucleic acid,
   wherein the Q-PTO comprises in a 5' to 3' direction: (i) a 5'-tagging portion comprising a non-hybridizing nucleotide sequence to the target nucleic acid, and (ii) a hybridizing nucleotide sequence to a second region of the target nucleic acid,
   wherein the 5'-tagging portion of the Q-PTO is not hybridized with the target nucleic acid and the 3'-targeting portion of the Q-PTO is hybridized with the target nucleic acid,
   wherein the primer is extended to induce cleavage of the Q-PTO by a DNA polymerase having 5' nuclease activity such that the cleavage releases the reporter-carrying fragment;
   wherein the Q-PTO has a first quencher molecule in the 3'-targeting portion and a reporter molecule located at position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in the reporter-carrying fragment,
   wherein the CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment,
   wherein the capturing portion of the CTO is hybridized with the reporter-carrying fragment,
   wherein the CTO has a second quencher molecule located at positions such that upon hybridization of the capturing portion of the CTO with the reporter-carrying fragment, the second quencher molecule is in close proximity to the reporter molecule, thereby causing the second quencher molecule to quench a signal from the reporter molecule; and
   wherein the reporter-carrying fragment hybridized with the capturing portion of the CTO is extended to generate an extended strand comprising an extended sequence complementary to the templating portion of the CTO, thereby forming an extended duplex between the extended strand and the CTO.

The fourth aspect of the present disclosure, which is a kit comprising *n* compositions for detecting *n* target nucleic acids in a sample, is designed to implement the third aspect of the present disclosure. Therefore, common description between the two aspects is omitted herein to avoid undue complexity of the present specification.

According to an embodiment, the kit may further comprise instructions describing the method of the present invention.

According to an embodiment, the instructions for describing or practicing the methods of the present invention may be recorded on a suitable storage medium. For example, the instructions may be printed on a substrate, such as paper and plastic. In other embodiments, the instructions may be present as an electronic storage data file present on a suitable computer readable storage medium such as CD-ROM and diskette. In yet other embodiments, the instructions may not actually be present in the kit, but means for obtaining the instructions from a remote source, e.g. via the internet, are provided. An example of this embodiment is a kit that includes a web address where the instructions can be viewed and/or from which the instructions can be downloaded.

Hereinafter, the present invention will be described in more detail by examples. The following embodiments are provided to describe the present invention in further details, and it will become apparent to one of ordinary skill in the art to which this invention belongs that the scope of the present invention as set forth in the appended claims is not limited to or by the following examples.

### Mode for Invention

### Example

### Example 1: Detection of a Single Target Nucleic Acid

It was examined whether a target nucleic acid can be detected in real time using the Q-PTOCE assay according to the present disclosure.

### <1-1. Preparation of Template and Oligonucleotides>

The genome DNA of *Ureaplasma parvum* (UP) (Accession No: ATCC 27815) was used as the template for the target nucleic acid. As oligonucleotides for detecting the UP target nucleic acid, a Q-PTO, a CTO, a forward primer, and a reverse primer were prepared as shown in Table 4. The Q-PTO comprises in a 5' to 3' direction: (i) a 5'-tagging portion comprising a non-hybridizing nucleotide sequence to the UP target nucleic acid and (ii) a 3'-targeting portion comprising a hybridizing nucleotide sequence to a second region of the UP target nucleic acid. The CTO comprises in a 3' to 5' direction: (i) a capturing portion comprising a hybridizing nucleotide sequence to the 5'-tagging portion (i.e., reporter-carrying fragment) of the Q-PTO and (ii) a templating portion comprising a non-hybridizing nucleotide sequence to the reporter-carrying fragment. The Q-PTO has a reporter molecule (Cal Fluor Red 610) linked to the 5'-end of the 5'-tagging portion, and a first quencher molecule (BHQ-2) linked to the 3'-targeting portion. The 3'-end of the Q-PTO was blocked with Spacer C3 to prevent extension by DNA polymerase. The CTO has a second quencher molecule (BHQ-2) linked to the 3'-end of the 3'-capturing portion.

**[Table 4]**

| Oligonucleotide Sequences | | | |
|---|---|---|---|
| Target nucleic acid | SEQ NO. | Oligo type | Sequence (5'-3') |
| UP | 1 | Forward Primer | ACTGATGTAGCTGTTGCTATTC |
| | 2 | Reverse Primer | AATTGTTGGGTTTGTAGAAGC |
| | 3 | Q-PTO | [CAL Fluor Red 610]GCCATTACTGACGAGGGCATGCTCCAGA[T(BHQ- 2)]ATTCTAGAAACTGT[Spacer C3] |
| | 4 | CTO | ACGTCGATCTATCAATGAATACTCGTCAGTAATGGC[BHQ- 2] |

### <1-2. Real-time Polymerase Chain Reaction>

Real-time polymerase chain reaction (PCR) was performed using the above oligonucleotides.

The target nucleic acid (Tube 1: 500 pg of UP genomic DNA; Tube 2: distilled water (negative control)) was mixed with: 5 pmole of forward primer (SEQ NO: 1), 5 pmole of reverse primer (SEQ NO: 2), 0.5 pmole of Q-PTO (SEQ NO: 3), and 2 pmole of CTO (SEQ NO: 4), and then combined with 5 µL of 4X Master mix (final, 0.8 mM of dNTPs, 50 mM of KCl, 3.5 mM of MgCl₂, 20U Taq DNA polymerase) (Nanohelix, Korea), to prepare a reaction mixture in the final volume of 20 µL.

The tubes containing the reaction mixture were placed in a real-time thermal cycler (CFX96 Real-time Cycler, Bio-Rad), and denatured at 95°C for 15 minutes, and then subjected to 40 cycles of 10 seconds at 95°C, 15 seconds at 60°C, 10 seconds at 72°C, and 5 seconds at 85°C. Detection of signals was performed at two temperatures in each cycle : (i) 60°C, at which the extended duplex generated dependent on the presence of the UP target nucleic acid remains in its double-stranded state, so that the second quencher molecule quenches the signal from the reporter molecule (i.e., the temperature within the signal-constant temperature range); and (ii) 85°C, at which the extended duplex generated dependent on the presence of the UP target nucleic acid dissociates into a single-stranded state, so that the second quencher molecule unquenches the signal from the reporter molecule (i.e., the temperature within the signal-changing temperature range).

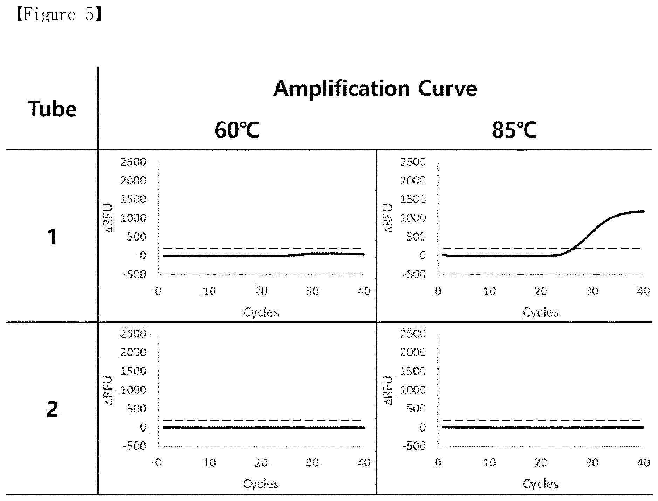

As a result, as shown in FIG. 5, in the case of Tube 1 containing the target nucleic acid, the amplification curve at 60°C, which is a temperature within the signal-constant temperature range, remained constant without a change in signal as the target nucleic acid was amplified, whereas the amplification curve at 85°C, which is a temperature within the signal-changing temperature range (i.e., the detection temperature), showed a change in signal as the target nucleic acid was amplified. These results indicate that the target nucleic acid can be detected in real time using the Q-PTOCE assay according to the present disclosure. In addition, the results indicate that the Q-PTOCE composition according to the present disclosure can be used as an OverSC composition for detecting a target nucleic acid, as described above.

Meanwhile, in the case of Tube 2 (negative control reaction) that does not contain the target nucleic acid, it was confirmed that the signal is constant at both 60°C and 85°C.

Subsequently, the Q-PTOCE assay according to the present disclosure can determine a signal indicating the presence of a target nucleic acid (i.e., a change in signal) by using a reference signal value obtained from the negative control reaction. The present inventors set the reference signal value to 200 based on the signal value of the negative control reaction (i.e., RFU: 0), and determined that a change in signal occurred when the signal value at 60°C or 85°C was equal to or greater than the reference signal value. As a result, as shown in Table 5, it was confirmed that a change in signal occurred only at the detection temperature of 85°C with a Ct value of 26.12, whereas the signal at 60°C remained constant without a change.

**[Table 5]**

| Ct value | | |
|---|---|---|
| Tube | Ct (Cycle threshold) | |
| | 60°C | 85°C |
| 1 | N/A | 26.12 |
| 2 | N/A | N/A |

| | | |
|---|---|---|
| Tube 1: 500 pg of UP genome DNA; Tube 2: Negative control; N/A: Not Applicable | | |

### Example 2: Detection of Multiple Target Nucleic Acids

According to the present disclosure, multiple target nucleic acids can be detected in a single reaction vessel using a single type of label by adjusting the signal-changing temperature ranges of compositions for detecting the multiple target nucleic acids.

Accordingly, it was examined whether multiple target nucleic acids can be detected in real time using a single type of label in a single reaction vessel by combining the Q-PTOCE composition according to the present disclosure, which is an OverSC composition for detecting a target nucleic acid, with another composition for detecting a target nucleic acid employing a different signal generation mechanism (e.g., an UnderSC or InterSC composition).

To this end, the PTOCE assay (WO 2012/096523) using a CTO with interactive dual labels, employing UnderSC signal generation mechanism, was used to detect the first target nucleic acid among the two target nucleic acids. The Q-PTOCE assay according to the present disclosure, employing OverSC signal generation mechanism, was used to detect the second target nucleic acid.

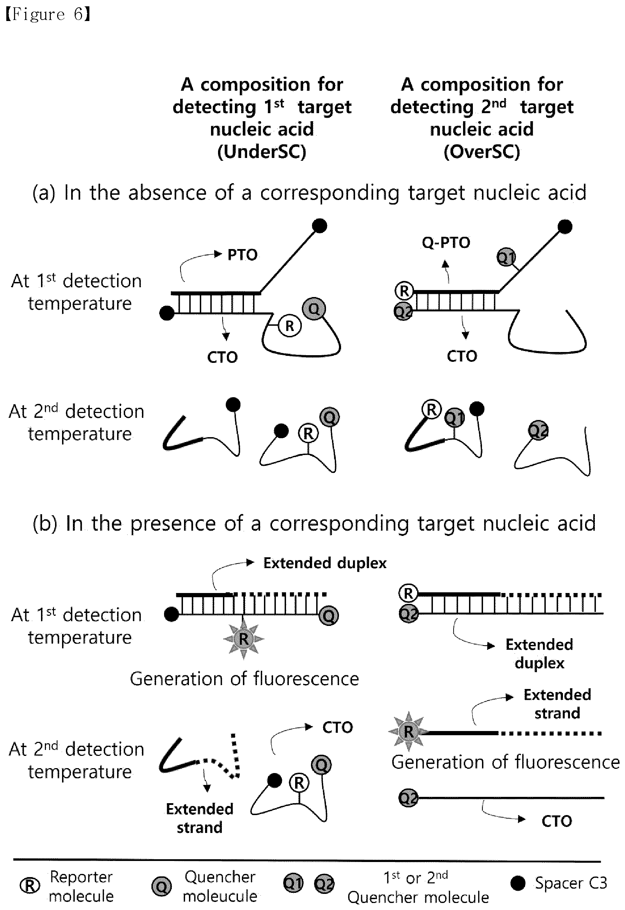

FIG. 6 schematically shows the principle of signal generation depending on the presence of target nucleic acids in the composition for detecting the CT target nucleic acid and the composition for detecting the UP target nucleic acid used in this Example. The PTOCE assay used for detecting the CT target nucleic acid employs the UnderSC signal generation mechanism, in which the signal-changing temperature range is lower than the signal-constant temperature range. The Q-PTOCE assay according to the present disclosure, used for detecting the UP target nucleic acid, employs the OverSC signal generation mechanism, as confirmed in Example 1. Therefore, by adjusting the signal-changing temperature ranges of the PTOCE assay and the Q-PTOCE assay, it can be adjusted such that only the signal corresponding to each target nucleic acid is provided at its respective detection temperature. To this end, oligonucleotides and detection temperatures were set as described below.

### <2-1. Preparation of Target Nucleic Acids and Oligonucleotides>

The genome DNA of *Chlamydia trachomatis* (CT) (Accession No: ATCC VR-1500) was used as the template for the first target nucleic acid, and the genome DNA of *Ureaplasma parvum* (UP) (Accession No: ATCC 27815) was used as the template for the second target nucleic acid.

The first detection temperature for detecting a change in signal indicating the presence of the first target nucleic acid, CT, was set to 60°C, and the second detection temperature for detecting a change in signal indicating the presence of the second target nucleic acid, UP, was set to 85°C. Then, oligonucleotides of the first composition for detecting the first target nucleic acid (CT) and the second composition for detecting the second target nucleic acid (UP) were prepared as described below.

First, as oligonucleotides for detecting the first target nucleic acid, CT, a PTO, a CTO, a forward primer, and a reverse primer were prepared as shown in Table 6. The PTO comprises in a 5' to 3' direction: (i) a 5'-tagging portion comprising a non-hybridizing nucleotide sequence to the CT target nucleic acid and (ii) a 3'-targeting portion comprising a hybridizing nucleotide sequence to the CT target nucleic acid. The CTO comprises in a 3' to 5' direction: (i) a capturing portion comprising a hybridizing nucleotide sequence to the 5'-tagging portion of the PTO and (ii) a templating portion comprising a non-hybridizing nucleotide sequence to the 5'-tagging portion of the PTO. The CTO has a quencher molecule (BHQ-2) linked to its 5'-end and a reporter molecule (Cal Fluor Red 610) linked to its 3'-targeting portion. The 3'-ends of the PTO and the CTO were blocked with Spacer C3 to prevent extension by DNA polymerase.

The oligonucleotides for detecting the second target nucleic acid, UP, were the same as those shown in Table 4 of Example 1-1.

**[Table 6]**

| Oligonucleotide Sequences | | | |
|---|---|---|---|
| Target nucleic acid | SEQ NO. | Oligo type | Sequence (5'-3') |
| CT | 5 | Forward Primer | GAGTTTT AAAA TGGGAAA TTCTGGT |
| | 6 | Reverse Primer | CCAATTGTAATAGAAGCATTGGTTG |
| | 7 | PTO | GATTACGCGACCGCATCAGAAGCTGTCATTTTGGCTGCG[Spa cer C3] |
| | 8 | CTO | [BHQ-2]TTTTTTTTTTTTTTTT[T(Cal Fluor Red 610)]TTTATGTGCGGTCGCGTAATC[Spacer C3] |
| UP | 1 | Forward Primer | ACTGATGTAGCTGTTGCTATTC |
| | 2 | Reverse Primer | AATTGTTGGGTTTGTAGAAGC |
| | 3 | Q-PTO | [CAL Fluor Red 610]GCCATTACTGACGAGGGCATGCTCCAGA[T(BHQ- 2)]ATTCTAGAAACTGT[Spacer C3] |
| | 4 | CTO | ACGTCGATCTATCAATGAATACTCGTCAGTAATGGC[BHQ-2] |

As shown in FIG. 6(a), the composition for detecting the CT target nucleic acid (i.e., the composition for detecting the first target nucleic acid) and the composition for detecting the UP target nucleic acid (i.e., the composition for detecting the second target nucleic acid) do not generate extended duplexes when the corresponding target nucleic acids are absent. In contrast, the composition for detecting the CT target nucleic acid and the composition for detecting the UP target nucleic acid each generate extended duplexes when the corresponding target nucleic acids are present (see FIG. 6(b)). The sequences and lengths of the oligonucleotides were designed as shown in Table 6 such that the extended duplexes exist in a double-stranded form at a first detection temperature and in a dissociated form at a second detection temperature. Specifically, as shown in FIG. 6(b), the extended duplex generated dependent on the presence of the CT target nucleic acid maintains a double-stranded form at the first detection temperature, allowing the quencher molecule of the CTO to unquench the signal from the reporter molecule of the CTO, and dissociates into two single strands at the second detection temperature, allowing the quencher molecule of the CTO to quench the signal from the reporter molecule of the CTO. Meanwhile, the extended duplex generated dependent on the presence of the UP target nucleic acid maintains a double-stranded form at the first detection temperature, allowing the second quencher molecule of the CTO to quench the signal from the reporter molecule of the extended strand, and dissociates into two single strands at the second detection temperature, allowing the second quencher molecule of the CTO to unquench the signal from the reporter molecule of the extended strand.

### <2-2. Multiplex Real-Time Polymerase Chain Reaction>

Multiplex PCR was performed in a single reaction vessel using the above oligonucleotides.

The target nucleic acids (Tube 1: 500 pg of CT genomic DNA; Tube 2: 500 pg of UP genomic DNA; Tube 3: 500 pg of CT genomic DNA and 500 pg of UP genomic DNA; Tube 4: distilled water (negative control)) were added to a single tube, and mixed with 5 pmole of forward primer (SEQ ID NO: 5), 5 pmole of reverse primer (SEQ ID NO: 6), 3 pmole of PTO (SEQ ID NO: 7), and 1 pmole of CT-CTO (SEQ ID NO: 8) for CT nucleic acid amplification, and 5 pmole of forward primer (SEQ ID NO: 1), 5 pmole of reverse primer (SEQ ID NO: 2), 0.5 pmole of Quenching-PTO (SEQ ID NO: 3), and 2 pmole of UP-CTO (SEQ ID NO: 4) for UP nucleic acid amplification. The mixture was then combined with 5 µL of 4X Master Mix (final, 0.8 mM dNTPs, 50 mM KCl, 3.5 mM MgCl₂, and 20 U of Taq DNA polymerase) (Nanohelix, Korea) to prepare a final 20 µL reaction mixture.

The PTOCE assay and the Q-PTOCE assay are identical to each other in that they generate extended duplexes dependent on the presence of the target nucleic acid. However, in the PTOCE assay, the signal changes depending on the presence of the CT target nucleic acid at the first detection temperature, while the signal remains constant at the second detection temperature even when the CT target nucleic acid is present (i.e., the reporter molecule remains quenched by the quencher molecule regardless of the presence of the CT target nucleic acid). In contrast, in the Q-PTOCE assay, the signal changes depending on the presence of the UP target nucleic acid at the second detection temperature, while the signal remains constant at the first detection temperature even when the UP target nucleic acid is present (i.e., the reporter molecule remains quenched by the first and/or second quencher molecule(s) regardless of the presence of the UP target nucleic acid) (see FIG. 6).

The tube containing the reaction mixture was placed in a real-time thermal cycler (CFX96 Real-time Cycler, Bio-Rad), denatured at 95°C for 15 minutes, and then subjected to 40 cycles of 10 seconds at 95°C, 15 seconds at 60°C, 10 seconds at 72°C, and 5 seconds at 85°C. Signal detection was performed at the first detection temperature of 60°C (CT target nucleic acid detection temperature) and the second detection temperature of 85°C (UP target nucleic acid detection temperature) in each cycle.

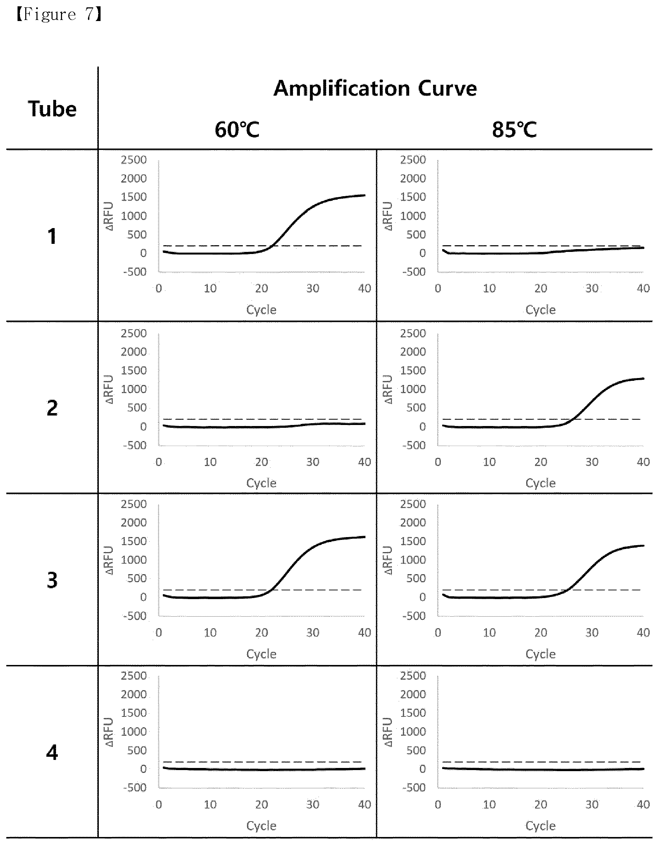

As a result, as shown in FIG. 7, in Tube 1, which contains only the CT target nucleic acid, the amplification curve at 85°C showed that the signal remained constant even though the CT target nucleic acid was amplified, whereas the amplification curve at 60°C, the detection temperature for the CT target nucleic acid, showed that the signal changed as the CT target nucleic acid was amplified. In Tube 2, which contains only the UP target nucleic acid, the amplification curve at 60°C showed that the signal remained constant even though the UP target nucleic acid was amplified, whereas the amplification curve at 85°C, the detection temperature for the UP target nucleic acid, showed that the signal changed as the UP target nucleic acid was amplified. In Tube 3, which contains both CT and UP target nucleic acids, the amplification curve at 60°C, the detection temperature for the CT target nucleic acid, and the amplification curve at 85°C, the detection temperature for the UP target nucleic acid, each showed that the signal changed as the respective target nucleic acids were amplified. In contrast, Tube 4, the negative control reaction tube, showed that the signal remained constant in both amplification curves at 60°C and 85°C.

These results demonstrate that multiple target nucleic acids can be detected in real time in a single reaction vessel using a single type of label by combining the Q-PTOCE composition according to the present disclosure, which is an OverSC composition for detecting a target nucleic acid, with another composition for detecting a target nucleic acid employing a different signal generation mechanism (i.e., an UnderSC composition for detecting a target nucleic acid and/or an InterSC composition for detecting a target nucleic acid).

Subsequently, a signal indicating the presence of a target nucleic acid (i.e., a change in signal) was determined using a reference signal value obtained from the negative control reaction. The present inventors set the reference signal value to 200 based on the signal value of the negative control reaction (i.e., RFU: 0), and determined that a change in signal occurred when the signal value at 60°C or 85°C was equal to or greater than the reference signal value.

As a result, as shown in Table 7, it was confirmed that in Tube 1, a change in signal occurred only at 60°C, the detection temperature for the CT target nucleic acid (i.e., the first detection temperature), with a Ct value of 21.67, and that in Tube 2, a change in signal occurred only at 85°C, the detection temperature for the UP target nucleic acid (i.e., the second detection temperature), with a Ct value of 25.91. In Tube 3, a change in signal occurred at both the first detection temperature of 60°C and the second detection temperature of 85°C with Ct values of 21.52 and 25.04, respectively. In contrast, at the other temperatures, the signal remained constant without a change.

These results demonstrate that, as described above, multiple different target nucleic acids can be independently detected at their respective detection temperatures.

**[Table 7]**

| Ct value | | |
|---|---|---|
| Tube | Ct (Cycle threshold) | |
| | First temperature (60°C) | Second temperature (85°C) |
| 1 | 21.67 | N/A |
| 2 | N/A | 25.91 |
| 3 | 21.52 | 25.04 |
| 4 | N/A | N/A |

| | | |
|---|---|---|
| Tube 1: 500 pg of CT genomic DNA; Tube 2: 500 pg of UP genomic DNA; Tube 3: 500 pg of CT genomic DNA and 500 pg of UP genomic DNA; Tube 4: Negative control; N/A: Not Applicable | | |

In summary, the Q-PTOCE assay according to the present disclosure is capable of detecting not only a single target nucleic acid, but also multiple target nucleic acids in real time in a single reaction vessel using a single type of label and a single detector, when used in combination with other signal generation mechanisms (e.g., UnderSC and/or InterSC signal generation mechanisms).

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A method for detecting a target nucleic acid in a sample by Quenching-PTO Cleavage and Extension (Q-PTOCE) assay, comprising:
(a) hybridizing a primer and a Quenching-Probing and Tagging Oligonucleotide (Q-PTO) with the target nucleic acid;
wherein the primer comprises a hybridizing nucleotide sequence to a first region of the target nucleic acid,
wherein the Q-PTO comprises in a 5' to 3' direction: (i) a 5'-tagging portion comprising a non-hybridizing nucleotide sequence to the target nucleic acid, and (ii) a hybridizing nucleotide sequence to a second region of the target nucleic acid,
wherein the 5'-tagging portion of the Q-PTO is not hybridized with the target nucleic acid and the 3'-targeting portion of the Q-PTO is hybridized with the target nucleic acid,
wherein the Q-PTO has a first quencher molecule in the 3'-targeting portion and a reporter molecule located at a position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in a reporter-carrying fragment,
wherein the primer is located upstream of the Q-PTO;
(b) contacting the resultant of the step (a) to a DNA polymerase having 5' nuclease activity under conditions for cleavage of the Q-PTO to provide the reporter-carrying fragment;
wherein the primer is extended to induce cleavage of the Q-PTO by the DNA polymerase having 5' nuclease activity such that the cleavage releases the reporter-carrying fragment;
(c) hybridizing the reporter-carrying fragment with a Capturing and Templating Oligonucleotide (CTO);
wherein the CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment,
wherein the capturing portion of the CTO is hybridized with the reporter-carrying fragment,
wherein the CTO has a second quencher molecule located at a position such that upon hybridization of the capturing portion of the CTO with the reporter-carrying fragment, the second quencher molecule is in close proximity to the reporter molecule, thereby causing the second quencher molecule to quench a signal from the reporter molecule;
(d) performing an extension reaction using the resultant of the step (c) and the DNA polymerase having 5' nuclease activity;
wherein the reporter-carrying fragment hybridized with the capturing portion of the CTO is extended to generate an extended strand comprising an extended sequence complementary to the templating portion of the CTO, thereby forming an extended duplex between the extended strand and the CTO; and
(e) detecting the presence of the extended strand;
whereby the presence of the extended strand indicates the presence of the target nucleic acid.

2. The method of claim 1, wherein the first quencher molecule of the Q-PTO is located immediately adjacent to or 1 to 30 nucleotides apart from the reporter molecule of the Q-PTO.

3. The method of claim 1, wherein the first quencher molecule of the Q-PTO and the second quencher molecule of the CTO are the same type of quencher molecule.

4. The method of claim 1, wherein the Tm value of the extended duplex is adjustable by (i) a sequence and/or length of the reporter-carrying fragment, (ii) a sequence and/or length of the CTO or (iii) the sequence and/or length of the reporter-carrying fragment and the sequence and/or length of the CTO.

5. The method of claim 1, wherein the presence of the extended strand in the step (e) is detected by measuring a signal provided from the extended duplex.

6. The method of claim 5, wherein the signal provided from the extended duplex is a signal generated from dissociation of the extended duplex.

7. The method of claim 1, wherein the presence of the extended strand in the step (e) is detected by measuring a signal at a temperature at which the extended duplex dissociates.

8. The method of claim 1, wherein the Q-PTO and/or the CTO are blocked at its 3'-end to prohibit its extension.

9. The method of claim 1, wherein the method further comprises repeating all or a portion of the steps (a)-(e) with denaturation.

10. The method of claim 1, wherein the target nucleic acid comprises a nucleotide variation.

11. The method of claim 1, wherein the method is performed in the presence of an additional primer that pairs with the primer to amplify the target nucleic acid.

12. A composition for detecting a target nucleic acid in a sample, comprising:
(i) a primer;
wherein the primer comprises a hybridizing nucleotide sequence to a first region of the target nucleic acid,
(ii) a Quenching-Probing and Tagging Oligonucleotide (Q-PTO);
wherein the Q-PTO comprises in a 5' to 3' direction: (i) a 5'-tagging portion comprising a non-hybridizing nucleotide sequence to the target nucleic acid, and (ii) a hybridizing nucleotide sequence to a second region of the target nucleic acid,
wherein the 5'-tagging portion of the Q-PTO is not hybridized with the target nucleic acid and the 3'-targeting portion of the Q-PTO is hybridized with the target nucleic acid,
wherein the primer is extended to induce cleavage of the Q-PTO by a DNA polymerase having 5' nuclease activity such that the cleavage releases the reporter-carrying fragment;
wherein the Q-PTO has a first quencher molecule in the 3'-targeting portion and a reporter molecule located at position such that (i) prior to cleavage of the Q-PTO, the reporter molecule is in close proximity to the first quencher molecule, thereby causing the reporter molecule to be quenched by the first quencher molecule, and (ii) upon cleavage of the Q-PTO, the reporter molecule is included in the reporter-carrying fragment,
(iii) a Capturing and Templating Oligonucleotide (CTO);
wherein the CTO comprises in a 3' to 5' direction: (i) a capturing portion comprising a hybridizing nucleotide sequence to the reporter-carrying fragment and (ii) a templating portion comprising a non-hybridizing nucleotide sequence to the reporter-carrying fragment,
wherein the capturing portion of the CTO is hybridized with the reporter-carrying fragment,
wherein the CTO has a second quencher molecule located at positions such that upon hybridization of the capturing portion of the CTO with the reporter-carrying fragment, the second quencher molecule is in close proximity to the reporter molecule, thereby causing the second quencher molecule to quench a signal from the reporter molecule,
wherein the reporter-carrying fragment hybridized with the capturing portion of the CTO is extended to generate an extended strand comprising an extended sequence complementary to the templating portion of the CTO, thereby forming an extended duplex between the extended strand and the CTO.

13. The composition of claim 12, wherein the first quencher molecule of the Q-PTO is located immediately adjacent to or 1 to 30 nucleotides apart from the reporter molecule of in the Q-PTO.

14. The composition of claim 12, wherein the first quencher molecule of the Q-PTO and the second quencher molecule of the CTO are the same type of quencher molecule.

15. The composition of claim 12, wherein the Tm value of the extended duplex is adjustable by (i) a sequence and/or length of the fragment, (ii) a sequence and/or length of the CTO or (iii) the sequence and/or length of the fragment and the sequence and/or length of the CTO.

16. The composition of claim 12, wherein the Q-PTO, the CTO or the Q-PTO and the CTO are blocked at its 3'-end to prohibit its extension.

17. The composition of claim 12, which further comprises a DNA polymerase having 5' nuclease activity.

18. The composition of claim 12, which further comprises an additional primer that pairs with the primer to amplify the target nucleic acid.

19. The composition of claim 12, which provides a signal dependent on the presence of the target nucleic acid.

20. The composition of claim 19, wherein the signal dependent on the presence of the target nucleic acid is a signal provided from the extended duplex.

21. The composition of claim 12, which has a signal-changing temperature range in which the signal changes depending on the presence of the target nucleic acid, and a signal-constant temperature range in which the signal is constant even in the presence of the target nucleic acid.

22. The composition of claim 21, wherein the signal-changing temperature range is higher than the signal-constant temperature range.

23. The composition of claim 21, wherein the extended duplex forms a double strand at temperatures within the signal-constant temperature range in the presence of the target nucleic acid.

24. The composition of claim 21, wherein the extended duplex dissociates at temperatures within the signal-changing temperature range in the presence of the target nucleic acid.

25. The composition of claim 21, wherein the signal-changing temperature range depends on the Tm value of the extended duplex.

26. A method for detecting n target nucleic acids in a sample, comprising:
(a) detecting signals at *n* detection temperatures, while incubating with *n* compositions for detecting the n target nucleic acids, a sample suspected of containing at least one of the n target nucleic acids in a reaction vessel;
wherein n is an integer of 2 or more,
wherein the incubating comprises a plurality of reaction cycles and the detection of signals is carried out at at least one of the plurality of reaction cycles,
wherein each of the n compositions for detecting the *n* target nucleic acids provides a signal change at a corresponding detection temperature among the n detection temperatures in the presence of a corresponding target nucleic acid, the signal change indicating the presence of a corresponding target nucleic acid,
wherein a composition for detecting an *i*^{th} target nucleic acid among the *n* compositions for detecting the *n* target nucleic acids provides a signal change at an *i*^{*t*h} detection temperature among the n detection temperatures and provides a constant signal at the other detection temperatures in the presence of the *i*^{th} target nucleic acid,
wherein *i* represents an integer from 1 to *n,* and the *i*^{th} detection temperature is lower than a (*i*+1)^{th} detection temperature,
**wherein** in the temperature range covering all of the n detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid,
wherein the composition for detecting the *i*^{th} target nucleic acid is any one of:
(i) an Under-Signal-Change (UnderSC) composition having a characteristic that the signal-changing temperature range is lower than the signal-constant temperature range,
(ii) an Inter-Signal-Change (InterSC) composition having a characteristic that the signal-changing temperature range is higher than one of two signal-constant temperature ranges, and lower than the other of the two signal-constant temperature ranges, and
(iii) an Over-Signal-Change (OverSC) composition having a characteristic that the signal-changing temperature range is higher than the signal-constant temperature range, and
wherein the composition for detecting the *n*^{th} target nucleic acid among the *n* compositions for detecting the *n* target nucleic acids is a composition according to any one of claims 12 to 25, and;
(b) determining the presence of the *n* target nucleic acids from the signals detected in step (a), wherein the presence of the *i*^{th} target nucleic acid is determined by the signal change detected at the *i*^{th} detection temperature.

27. The method of claim 26, wherein the *i*^{th} detection temperature is selected within the signal-changing temperature range of the composition for detecting the *i*^{th} target nucleic acid, wherein the *i*^{th} detection temperature is not comprised in the signal-changing temperature ranges of the compositions for detecting the other target nucleic acids.

28. The method of claim 26, wherein the signal-changing temperature range of the **composition** for detecting the *i*^{th} target nucleic acid overlaps partially with the signal-changing temperature range of a composition for detecting a target nucleic acid having an adjacent detection temperature, and does not overlap with the signal-changing temperature range of a composition for detecting a target nucleic acid having a detection temperature that is not adjacent thereto.

29. The method of claim 26, wherein, when *n is* 2, the composition for detecting the first target nucleic acid is an UnderSC or InterSC composition.

30. The method of claim 26, wherein, when *n is* 3 or more, the composition for detecting the first target nucleic acid is an UnderSC or InterSC composition, and each of compositions for detecting target nucleic acids other than the first target nucleic acid and the *n*^{th} target nucleic acid is an InterSC composition.

31. The method of claim 26, wherein the composition for detecting the *i*^{th} target nucleic acid comprises a label that provides a signal dependent on the presence of the *i*^{th} target nucleic acid.

32. The method of claim 31, wherein the label is linked to an oligonucleotide or is incorporated into an oligonucleotide during the incubating.

33. The method of claim 26, wherein the composition for detecting the *i*^{th} target nucleic acid provides a duplex providing a signal change.

34. The method of claim 33, wherein the duplex providing the signal change has initially been included in the composition for detecting the *i*^{th} target nucleic acid.

35. The method of claim 34, wherein the duplex providing the signal change is generated by hybridization between a label-linked oligonucleotide and an oligonucleotide hybridizable with the label-linked oligonucleotide.

36. The method of claim 33, wherein the duplex providing the signal change is generated in incubating.

37. The method of claim 36, wherein the duplex providing the signal change is generated by hybridization between a label-linked oligonucleotide and the corresponding target nucleic acid.

38. The method of claim 36, wherein the duplex providing the signal change is generated by a cleavage reaction dependent on the presence of the corresponding target nucleic acid.

39. The method of claim 33, wherein the duplex providing the signal change comprises a label.

40. The method of claim 26, wherein the composition for detecting the *i*^{th} target nucleic **acid provides** a duplex providing a signal change, and the signal-changing temperature range of the composition for detecting the *i*^{th} target nucleic acid varies depending on the length and/or sequence of the duplex.

41. The method of claim 26, wherein the detection of signals is carried out at at least two of the plurality of reaction cycles.

42. The method of claim 41, wherein the signal change is measured using the signals detected at the at least two of the plurality of reaction cycles.

43. The method of claim 26, wherein the signal change at the *i*^{th} detection temperature is measured using a signal detected at the at least one of the plurality of reaction cycles and a reference signal value.

44. The method of claim 43, wherein the reference signal value is obtained from a reaction in the absence of the *i*^{th} target nucleic acid.

45. The method of claim 26, wherein the detection of a signal at each of the n detection temperatures is carried out using a single type of detector.

46. The method of claim 45, wherein the signals detected at the n detection temperatures are not differentiated from each other by the single type of detector.

47. The method of claim 26, wherein the incubating comprises a nucleic acid amplification reaction.
